(19) Europäisches Patentamt
European Patent Office
Office européen des brevets

(11) Numéro de publication : **0 380 392 B1**

(12) # FASCICULE DE BREVET EUROPEEN

(45) Date de publication du fascicule du brevet : **20.04.94 Bulletin 94/16**

(21) Numéro de dépôt : **90400161.7**

(22) Date de dépôt : **22.01.90**

(51) Int. Cl.$^5$ : **C07D 311/96,** C07D 311/58, C07D 407/06, C07D 335/04, C07C 69/732, C07C 59/54, C07C 59/56, A61K 31/215, A01K 31/19

(54) **Dérivés d'acides benzocycloalcényl dihydroxy alcanoiques, procédés de préparation et médicaments les contenant.**

(30) Priorité : **24.01.89 FR 8900790**

(43) Date de publication de la demande : **01.08.90 Bulletin 90/31**

(45) Mention de la délivrance du brevet : **20.04.94 Bulletin 94/16**

(84) Etats contractants désignés : **AT BE CH DE DK ES FR GB GR IT LI LU NL SE**

(56) Documents cités :
**EP-A- 0 020 018**
**EP-A- 0 246 114**
**WO-A-86/03488**
**PATENT ABSTRACTS OF JAPAN, tome 12, no. 33 (C-472)(2880), 30 janvier 1988**
**CHEMICAL ABSTRACTS, tome 83, no. 8, 25 aout 1975, Columbus, OH (US); no. 68643f**
**CHEMICAL ABSTRACTS, tome 76, no. 23, 05 juin 1972, Columbus, OH (US); no. 140440n**

(73) Titulaire : **LIPHA, LYONNAISE INDUSTRIELLE PHARMACEUTIQUE**
**34, rue Saint Romain Boîte Postale 8481**
**F-69359 Lyon Cedex 08 (FR)**

(72) Inventeur : **Festal, Didier**
**Pins 5- Charrière Blanche**
**F-69130 Ecully (FR)**
Inventeur : **Nioche, Jean-Yves**
**16, allée des Cerisiers**
**F-69760 Limonest (FR)**
Inventeur : **Descours, Denis**
**59, Avenue Galline**
**F-69100 Villeurbanne (FR)**
Inventeur : **Bellemin, Robert**
**49, rue Saint Maximin**
**F-69003 Lyon (FR)**
Inventeur : **Decerprit, Jacques**
**83, Chemin de Sermenaz**
**F-01700 Neyron (FR)**

(74) Mandataire : **Moncheny, Michel et al**
**c/o Cabinet Lavoix 2 Place d'Estienne d'Orves**
**F-75441 Paris Cedex 09 (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux dérivés d'acides benzocycloalcényl dihydroxy alcanoïques, les procédés de préparation de ces composés et les compositions pharmaceutiques les contenant.

On sait que certains dérivés de l'acide dihydroxy-3,5-méthyl-3-pentanoïque, connu sous le nom d'acide "mévalonique" sont inhibiteurs de l'enzyme hydroxy-3- méthyl-3-glutaryl coenzyme A réductase, responsable de la biosynthèse du cholestérol, (cf. SINGER et Coll., Proc. Sc. Exper. Biol. Med., **102**, 275, 1959).

Plus récemment, un composé dérivé de l'acide mévalonique appelé "Lovastatine", anciennement "Mévinoline", a été proposé comme principe actif de compositions médicamenteuses utilisables dans le traitement des hypercholestérolémies, (cf. Brevet US 4231938 au nom de la Société MERCK).

On a trouvé à présent, de façon fortuite, que de nouveaux dérivés d'acides benzocycloalcényl dihydroxy alcanoïques sont doués de propriétés hypocholestérolémiantes, antithrombotiques et antifongiques.

L'invention concerne plus particulièrement les composés de formule **1**, numérotée pour permettre une meilleure compréhension de l'invention et à titre d'exemple uniquement comme indiqué,

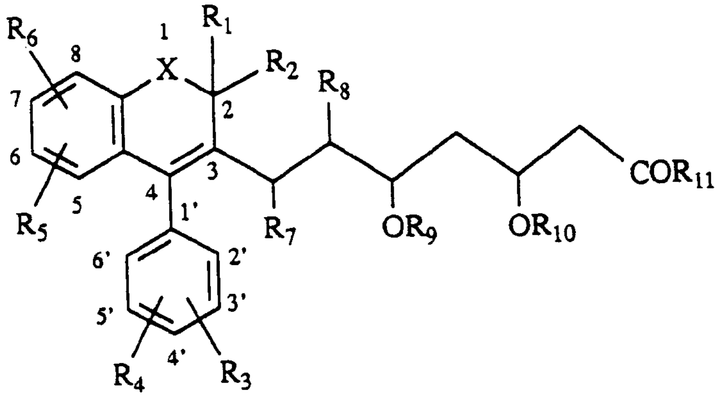

**1**

dans laquelle,

X représente un chaînon méthylène $-CH_2-$, un atome d'oxygène ou un atome de soufre.

$R_1$ et $R_2$ qui peuvent être identiques ou différents, représentent des atomes d'hydrogène ou des radicaux alkyle de 1 à 3 atomes de carbone. $R_1$ et $R_2$ peuvent aussi former ensemble une chaîne alkylène : $-(CH_2)_n-$, le cas échéant substituée symétriquement par un ou deux radicaux alkyle de 1 à 3 atomes de carbone et dont le nombre n de chaînons, peut prendre les valeurs 4 ou 5.

$R_3$ et $R_4$ qui peuvent être identiques ou différents, représentent des atomes d'hydrogène, des atomes d'halogène (fluor, chlore, ou brome), des radicaux : trifluorométhyle, N,N-dialkylamino de 1 à 3 atomes de carbone, alkyle de 1 à 4 atomes de carbone, alkoxy de 1 à 5 atomes de carbone, alkylthio de 1 à 3 atomes de carbone, ou phényle éventuellement substitué par deux substituants au plus qui peuvent être identiques ou différents et représenter des radicaux : alkyle de 1 à 3 atomes de carbone, alkoxy de 1 à 3 atomes de carbone, ou des atomes d'halogène (fluor ou chlore), à condition que lorsque l'un des substituants $R_3$ ou $R_4$ représente les radicaux : trifluorométhyle, N,N-dialkylamino, phényle ou phényle substitué, il se trouve sur les sommets 3', 4' ou 5' (méta ou para) selon la formule **1** et l'autre substituant représente un atome d'hydrogène.

$R_5$ et $R_6$ qui peuvent être identiques ou différents, représentent des atomes d'hydrogène, des atomes d'halogène (fluor, chlore ou brome), des radicaux : trifluorométhyle, alkyle de 1 à 3 atomes de carbone, alkoxy de 1 à 3 atomes de carbone, ou un radical phényle, le cas échéant substitué par deux substituants au plus qui peuvent être identiques ou différents et représenter des radicaux : alkyle de 1 à 3 atomes de carbone, alkoxy de 1 à 3 atomes de carbone, ou des halogènes (chlore ou fluor) à condition que lorsque l'un des substituants $R_5$ ou $R_6$ représente les radicaux : trifluorométhyle, phényle ou phényle substitué, il se trouve sur les sommets 6 ou 7 selon la formule **1** et l'autre substituant désigne un atome d'hydrogène.

Les substituants $R_3$ et $R_4$ respectivement $R_5$ et $R_6$ peuvent également former ensemble, à condition d'être sur deux sommets contigus, les diradicaux : diéthylène, alkylène, ou alkylènedioxy, respectivement de formules : $-CH=CH-CH=CH-$, $-(CH_2)_m-$, $-O(CH_2)_pO-$, dans lesquelles m peut prendre la valeur 3 ou 4 et p la valeur 1 ou 2, et à condition que lorsque $R_3$ et $R_4$ respectivement $R_5$ et forment un diradical alkylènedioxy, celui-ci se trouve relié aux sommets 3' et 4' ou 4' et 5, respectivement aux sommets 6 et 7, selon la formule 1.

Les substituants $R_7$ et $R_8$ représentent chacun un atome d'hydrogène ou forment ensemble et avec la liaison C-C existante, une double liaison de géométrie trans (E).

Les substituants $R_9$ et $R_{10}$ représentent chacun un atome d'hydrogène ou forment ensemble un reste dialkylméthylène, de 1 à 3 atomes de carbone.

$R_{11}$ peut représenter un radical hydroxyle, auquel cas les composés de formule **1** sont des acides carboxyliques, sous forme libre.

Les composés de formule **1** peuvent aussi être obtenus sous forme d'esters et d'amides qui font également partie de l'invention.

Sont particulièrement préférés les esters et amides, physiologiquement acceptables, de formule **1**, dans laquelle le substituant $R_{11}$ représente des radicaux alkoxy de 1 à 4 atomes de carbone, benzyloxy, alkylamino ou N,N-dialkylamino de 1 à 3 atomes de carbone, imino de 4 à 6 atomes de carbone, cycloalkylamino de 3 à 6 atomes de carbone ou les radicaux amino ou benzylamino.

Les composés de formule **1** sous forme de sels, c'est à dire dans la formule desquels, le substituant $R_{11}$ représente un reste de formule $-O^-M^+$ dans laquelle $M^+$ désigne un cation pharmaceutiquement acceptable, font également partie de l'invention ; parmi ceux-ci sont plus particulièrement préférés les sels de sodium, potassium, magnésium, et ammonium.

Les composés de formule **1** peuvent exister sous forme de δ-lactones, lorsque le substituant $R_{11}$ forme avec le substituant $R_9$ une simple liaison ; les composés de formule **1** sous forme δ-lactone font également partie de l'invention.

Suivant une forme préférée de l'invention, celle-ci a pour objet les composés de formule **1** dans laquelle les substituants $R_1$ et $R_2$ sont identiques ou forment une chaîne alkylène : $-(CH_2)_n-$, non substituée dont le nombre n de chaînons est égal à 4 ou 5.

Une signification particulière des substituants $R_1$ et $R_2$, lorsqu'ils désignent des radicaux alkyle de 1 à 3 atomes de carbone est par exemple méthyle.

Une valeur particulière de n lorsque les substituants $R_1$ et $R_2$ forment une chaîne alkylène, $-(CH_2)_n-$, est par exemple 4.

Des significations particulières pour l'un ou l'autre des substituants $R_3$ et $R_4$ sont à titre d'exemple uniquement,

- lorsqu'il représente un atome d'halogène : fluor ou chlore ;
- lorsqu'il représente un radical alkyle de 1 à 4 atomes de carbone : méthyle ou éthyle ;
- lorsqu'il représente un radical alkoxy de 1 à 5 atomes de carbone : méthoxy ou éthoxy ;
- lorsqu'il représente un radical alkylthio de 1 à 3 atomes de carbone : méthylthio ;
- lorsqu'il représente un radical phényle substitué : phényle substitué en para par un atome de fluor, par un radical méthyle ou méthoxy.

Une valeur particulière de m lorsque les substituants $R_3$ et $R_4$ forment un diradical : $-(CH_2)_m-$ est à titre d'exemple 4.

Une valeur particulière de p lorsque les substituants $R_3$ et $R_4$ forment un diradical alkylènedioxy : $-O(CH_2)_pO-$ est par exemple 1.

Des agencements particulièrement appropriés des radicaux $R_3$ et $R_4$ sont, par exemple réalisés lorsque $R_3$ et $R_4$ désignent des radicaux alkyle de 1 à 4 atomes de carbone et/ou des atomes d'halogène, ou un atome d'halogène et un radical alkoxy de 1 à 5 atomes de carbone ; lorsque l'un des substituants $R_3$ ou $R_4$ est un atome d'halogène, ou un radical alkyle de 1 à 4 atomes de carbone, ou un radical alkoxy de 1 à 5 atomes de carbone, ou un radical alkylthio de 1 à 3 atomes de carbone et l'autre représente un atome d'hydrogène.

Des agencements appropriés spécifiques des radicaux $R_3$ et $R_4$ sont, par exemple réalisés, lorsqu'ils représentent des radicaux méthyle en position 3' et 5', un atome de fluor en position 4' et un radical méthyle en position 3', des atomes de fluor en position 3' et 4', un atome de fluor en position 3' ou 4' et un atome d'hydrogène, un radical méthyle ou éthyle en position 4' et un atome d'hydrogène, un radical méthoxy en position 3' ou 4' et un atome d'hydrogène, un atome de chlore en position 3' ou 4' et un atome d'hydrogène, ou des atomes d'hydrogène.

Une signification particulière de l'un quelconque des substituants $R_5$ et $R_6$ est, à titre d'exemples,

- lorsqu'il représente un atome d'halogène : fluor ou chlore ;
- lorsqu'il représente un radical alkyle de 1 à 3 atomes de carbone : méthyle ;
- lorsqu'il représente un radical alkoxy de 1 à 3 atomes de carbone : méthoxy ;
- lorsqu'il représente un radical phényle substitué : un radical phényle substitué en para par un atome de fluor ou de chlore ou par un radical méthoxy.

Une valeur particulière de m lorsque les substituants $R_5$ et $R_6$ forment ensemble une chaîne alkylène $-(CH_2)_m-$ est par exemple la valeur 4.

Une valeur particulière de p lorsque les substituants $R_5$ et $R_6$ forment ensemble une chaîne alkylènedioxy $O(CH_2)_pO-$ est la valeur 1.

Des agencements particulièrement appropriés des radicaux $R_5$ et $R_6$ sont réalisés, par exemple, lorsqu'ils représentent des atomes d'halogène, (fluor, chlore, brome) des radicaux alkyle de 1 à 3 atomes de carbone ou des radicaux alkoxy de 1 à 3 atomes de carbone ou lorsque l'un des substituants $R_5$ ou $R_5$ représente un atome d'halogène qui peut être le fluor, le chlore ou le brome, ou représente un radical alkyle de 1 à 3 atomes de carbone ou un radical alkoxy de 1 à 3 atomes de carbone et l'autre substituant représente un atome d'hydrogène.

Des agencements appropriés spécifiques des radicaux $R_5$ et $R_5$ sont, par exemple, réalisés lorsqu'ils représentent des radicaux méthyle en positions 5 et 7, un atome de chlore en position 6 et un atome d'hydrogène, un radical méthoxy en position 6 et un atome d'hydrogène, un atome de fluor en position 6 ou 7 et un atome d'hydrogène, un radical méthyle en position 6 ou 7 et un atome d'hydrogène, un radical fluoro-4 phényle en position 6 ou 7 et un atome d'hydrogène, ou deux atomes d'hydrogène.

Une signification particulière des substituants et $R_9$ et $R_{10}$ lorsqu'ils forment un reste dialkylméthylène est par exemple diméthylméthylène.

Des significations particulières du substituant $R_{11}$ sont, à titre d'exemple uniquement,

- lorsqu'il représente un radical alkoxy de 1 à 4 atomes de carbone : méthoxy ou éthoxy ;
- lorsqu'il représente un radical alkylamino de 1 à 3 atomes de carbone : méthylamino, éthylamino, ou isopropylamino ;
- lorsqu'il représente un radical N,N-dialkylamino de 1 à 3 atomes de carbone : diéthylamino ;
- lorsqu'il représente un radical imino de 4 à 6 atomes de carbone : pyrrolidino.

Les composés de formule **1** dans laquelle les substituants $R_7$ et $R_8$ forment ensemble une liaison sont préférés aux composés de formule **1** correspondants dont les substituants $R_7$ et $R_5$ représentent chacun un atome d'hydrogène.

Toutes choses étant égales par ailleurs, les composés de formule **1**, dans laquelle les substituants $R_9$ et $R_{10}$ désignent chacun un atome d'hydrogène sont préférés aux composés dont les substituants $R_9$ et $R_{10}$ forment un reste dialkylméthylène.

Toutes choses étant égales par ailleurs, les composés de formule **1** non lactoniques sont préférés aux composés δ-lactoniques.

Parmi les composés de formule **1** non lactoniques, les esters, les amides et les sels sont généralement préférés aux acides libres.

Toutes choses étant égales par ailleurs, les stéréoisomères érythro sont préférés au stéréoisomères thréo (les termes érythro et thréo faisant référence à l'orientation relative des groupes $OR_9$ et $OR_{10}$).

Parmi les composés δ-lactoniques de formule **1**, les stéréoisomères trans sont préférés aux stéréoisomères cis (les termes cis et trans se rapportant aux positions relatives axiales ou équatoriales des deux substituants du cycle δ-lactone).

Des groupes spécifiques de composés de l'invention qui sont particulièrement préférés, comprennent les composés de formule **1** dans laquelle :

A) X désigne les atomes d'oxygène ou de soufre ou un chaînon méthylène, $R_1$ et $R_2$ désignent chacun un radical méthyle ou forment ensemble une chaîne tétraméthylène $-(CH_2)_4-$, seul l'un des radicaux $R_3$ ou $R_4$ respectivement $R_5$ ou $R_5$ désigne un atome d'hydrogène, $R_7$ et $R_8$ forment ensemble une liaison et $R_9$ et $R_{10}$ représentent chacun un atome d'hydrogène.

B) X, $R_1$, $R_2$, $R_7$, $R_8$, $R_9$ et $R_{10}$ ont les significations données immédiatement ci-dessus en A), l'un des substituants $R_3$ et $R_4$ désigne un atome d'hydrogène et l'autre un atome de fluor, et seul l'un des substituants $R_5$ ou $R_5$ désigne un atome d'hydrogène.

C) X, $R_1$, $R_2$, $R_7$, $R_5$, $R_9$ et $R_{10}$ ont les significations qui viennent d'être définies ci-dessus en A), l'un des substituants $R_3$ et $R_4$ est un atome d'hydrogène et l'autre désigne un atome de fluor ou de chlore, et chacun des deux substituants $R_5$ et $R_6$ désigne un atome d'hydrogène.

D) X, $R_1$, $R_2$, $R_7$, $R_5$, $R_9$ et $R_{10}$ ont les significations particulières définies en A), les deux substituants $R_3$ et $R_4$ sont des atomes d'hydrogène, et seul l'un des radicaux $R_5$ et $R_5$ désigne un atome d'hydrogène.

E) X, $R_3$, $R_4$, $R_5$, $R_5$, $R_7$, $R_5$, $R_9$, $R_{10}$ ont les significations définies ci-dessus en C) et $R_1$ et $R_2$ forment ensemble une chaîne tétraméthylène $-(CH_2)_4-$.

F) X, $R_1$, $R_2$, $R_7$, $R_8$, $R_9$, $R_{10}$ ont les significations indiquées immédiatement ci-dessus en E) et chacun des substituants $R_3$, $R_4$, $R_5$ et $R_5$ désigne un atome d'hydrogène.

Chaque composé de formule **1** renferme au moins deux centres d'asymétrie qui sont les deux atomes de carbone porteurs des restes $OR_9$ et $OR_{10}$, lorsque les substituants $R_9$ et $R_{10}$ représentent chacun un atome d'hydrogène ou forment ensemble un reste dialkylméthylène ou bien qui sont le carbone hydroxylé et le carbone tertiaire situé en position alpha de l'atome d'oxygène intracyclique lorsque les substituants $R_9$ et $R_{11}$ forment ensemble une liaison.

Il en résulte que chaque composé, acide libre, ester, amide, sel, ou δ-lactone, répondant à la formule **1**

peut exister sous forme d'au moins quatre stéréoisomères deux à deux diastéréoisomères, désignés en utilisant les notations configurationelles usuelles R et S par RR, SS, RS et SR ou sous forme des mélanges racémates diastéréoisomères, RR-SS et RS-SR.

Tous ces stéréoisomères font également partie de l'invention.

Les composés de formule **1** peuvent renfermer plus de deux centres d'asymétrie, notamment lorsque les substituants $R_1$ et $R_2$ sont différents, ce qui génère des stéréoisomères supplémentaires qui font aussi partie de l'invention.

Il entre dans la compétence normale du spécialiste d'isoler ou de synthétiser une forme optiquement active d'un composé de formule **1**, par exemple par dédoublement d'un racémate ou par synthèse au départ d'un composé optiquement actif et de déterminer les propriétés biologiques des isomères ainsi isolés suivant les essais décrits ci-après.

Par ester et amide physiologiquement acceptable, on entend un ester ou un amide d'un composé selon l'invention, qui lorsqu'il est hydrolysé dans les conditions physiologiques, génère dans de telles conditions un alcool ou une amine physiologiquement acceptable, c'est à dire non toxique aux doses désirées.

Par le terme "alkyle" on entend un enchaînement hydrocarbure saturé, linéaire ou ramifié, dérivé de l'alcane correspondant par suppression d'un atome d'hydrogène.

Par le terme "alkoxy" on entend un radical alkyle tel que défini ci-dessus, lié à la molécule parent par un atome d'oxygène.

Par le terme "alkylamino" on entend un atome d'azote substitué par un atome d'hydrogène et par un radical alkyle tel que défini ci-dessus, la valence libre étant utilisée pour établir la liaison avec la molécule parent.

Par le terme "N,N-dialkylamino" on entend un radical alkylamino tel que défini ci-dessus dont l'atome d'hydrogène est remplacé par un radical alkyle, tel que défini précédemment.

Par le terme "imino" on entend un radical dialkylamino, tel que défini ci-dessus dont les deux radicaux alkyle forment ensemble une chaîne alkylène.

L'expression "dialkyl... de 1 à x atomes de carbone" signifie que chacun des deux radicaux alkyle composant le reste dialkyl peut contenir indépendamment de 1 à x atomes de carbone.

Comme composés spécifiques de l'invention on peut citer, à titre d'exemples uniquement, les composés suivants dont les formules développées sont données sur les dessins annexés :

Composé n° 1 : (+,-)-6E-Erythro-((fluoro-4-phényl)-4-spiro(2H-benzopyranne-1-2,1'-cyclopentyl)-3)-7-dihydroxy-3,5-heptène-6-oate d'éthyle.

Composé n° 2 : (+,-)-6E-Erythro-(dihydro-1,2-diméthyl-2,2-phényl-4-naphtyl-3)-7-dihydroxy-3,5-heptène-6-oatede méthyle.

Composé n° 3 : (+,-)-6E-Erythro-((chloro-4-phényl)-4-diméthyl-2,2-2H-benzothiapyranne-3-yl)-7-dihydroxy-3,5-heptène-6-oate de méthyle.

Composé n° 4 : (+,-)-Erythro-((fluoro-4-phényl)-4-spiro (2H-benzopyranne-1-2,1'-cyclopentyl)-3)-7-dihydroxy-3,5-heptanoate d'éthyle.

Composé n° 5 : (+,-)-6E-Erythro-(fluoro-4-phényl)-4-spiro (2H-benzopyranne-1-2,1'-cyclopentyl)-3)-7-dihydroxy-3,5-heptène-6-oate de sodium.

Composé n° 6 : (+,-)-6E-Erythro-((fluoro-4-phényl)-4-spiro (2H-benzopyranne-1-2,1'- cyclopentyl)-3)-7-dihydroxy-3,5-heptène-6-oate de benzyle.

Composé n° 7 : (+,-)-6E-Erythro-((fluoro-4-phényl)-4-spiro (2H-benzopyranne-1-2,1'-cyclopentyl)-3)-7-dihydroxy-3,5-N-méthylheptène-6-amide.

Composé n° 8 : (+,-)-Trans-(1E-((fluoro-4-phényl)-4-spiro (2H-benzopyranne-1-2,1'-cyclopentyl)-3)-2-éthényl)-6-hydroxy-4-tétrahydro-3,4,5,6-pyrannone-2.

Composé n° 9 : (+,-)-Trans-hydroxy-4-tétrahydro-3,4,5,6-(((fluoro-4-phényl)-4-spiro(2H-benzopyranne-1-2,1'-cyclopentyl)-3) -2-éthyl)-6-pyrannone-2.

Composé n° 10 : (+,-)-Erythro-((fluoro-4-phényl)-4-spiro(2H-benzopyranne-1-2,1'-cyclopentyl)-3)-7-dihydroxy-3,5-heptanoatede sodium.

Composé n° 11 : (+,-)-((1E-((fluoro-4-phényl)-4-spiro(2H-benzopyranne-1-2,1'-cyclopentyl)-3)-2-éthényl)-6 diméthyl-2,2-dioxane-1,3-yl-4)acétate d'éthyle.

Composé n° 12 : (+,-)-6E-Erythro-dihydroxy-3,5-(phényl-4-spiro (2H-benzopyranne-1-2,1'-cyclopentyl)-3)-7-heptène-6-oate d'éthyle.

Composé n° 13 : (+,-)-6E-Erythro-((éthyl-4-phényl)-4-spiro (2H-benzopyranne-1-2,1'-cyclopentyl)-3)-7-dihydroxy-3,5-heptène-6-oate d'éthyle.

Composé n° 14 : (+,-)-6E-Erythro-dihydroxy-3,5-(méthyl-6-phényl-4-spiro(2H-benzopyranne-1-2,1'-cyclopentyl)-3)-7-heptène-6-oate d'éthyle.

Composé n° 15 : (+,-)-6E-Erythro-(fluoro-7-phényl-4-spiro (2H-benzopyranne-1-2,1'-cyclopentyl)-3)-7-dihydroxy-3,5-heptène-6-oate de méthyle.

Composé n° 16 : (+,-)-6E-Erythro-dihydroxy-3,5-((méthyl-4-phényl)-4-spiro(2H-benzopyranne-1-2,1'-cyclopentyl)-3)-7-heptène-6-oate d'éthyle.

Composé n° 17 : (+,-)-6E-Erythro-((fluoro-3-phényl)-4-spiro (2H-benzopyranne-1-2,1'-cyclopentyl)-3)-7-dihydroxy-3,5-heptène-6-oate d'éthyle.

Composé n° 18 : (+,-)-6E-Erythro-dihydroxy-3,5((méthoxy-4-phényl)-4-spiro(2H-benzopyranne-1-2,1'-cyclopentyl)-3)-7-heptène-6-oate d'éthyle.

Composé n° 19 : (+,-)-6E-Erythro-((chloro-4-phényl)-4-spiro (2H-benzopyranne-1-2,1'-cyclopentyl)-3)-7-dihydroxy-3,5-heptène-6-oate d'éthyle.

Composé n° 20 : (+,-)-6E-Erythro-dihydroxy-3,5-((naphtyl-1)-4-spiro(2H-benzopyranne-1-2,1'-cyclopentyl)-3)-7-heptène-6-oate d'éthyle.

Composé n° 21 : (+,-)-6E-Erythro-dihydroxy-3,5-((diméthyl-3,5-phényl)-4-spiro(2H-benzopyranne-1-2,1'-cyclopentyl)-3)-7-heptène-6-oate d'éthyle.

Composé n° 22 : (+,-)-6E-Erythro-((fluoro-4-phényl)-4-diméthyl-5,7-spiro(2H-benzopyranne-1-2,1'-cyclopentyl)-3)-7-dihydroxy-3,5-heptène-6-oate de méthyle.

Composé n° 23 : (+,-)-6E-Erythro-((éthoxy-4-phényl)-4-spiro (2H-benzopyranne-1-2,1'-cyclopentyl)-3)-7-dihydroxy-3,5-heptène-6-oate de méthyle.

Composé n° 24 : (+,-)-6E-Erythro-dihydroxy-3,5-((isopropyloxy-4-phényl)-4-spiro(2H-benzopyranne-1-2,1'-cyclopentyl)-3)-7-heptène-6-oate de méthyle.

Composé n° 25 : (+,-)-6E-Erythro-((fluoro-4-phényl)-4-méthoxy-6-spiro(2H-benzopyranne-1-2,1'-cyclopentyl)-3)-7-dihydroxy-3,5-heptène-6-oate de méthyle.

Composé n° 26 : (+,-)-6E-Erythro-((trifluorométhyl-4-phényl)-4-spiro(2H-benzopyranne-1-2,1'-cyclopentyl)-3)-7-dihydroxy-3,5-heptène-6-oate de méthyle.

Composé n° 27 : (+,-)-6E-Erythro-dihydroxy-3,5-((n.pentyloxy-4-phényl)-4-spiro(2H-benzopyranne-1-2,1'-cyclopentyl)-3)-7-heptène-6-oate de méthyle.

Composé n° 28 : (+,-)-6E-Erythro-((fluoro-4-phényl)-4-spiro (2H-naphto(b)pyranne-1-2,1'-cyclopentyl)-3)-7-dihydroxy-3,5-heptène-6-oate de méthyle.

Composé n° 29 : (+,-)-6E-Erythro-dihydroxy-3,5-((méthylthio-4-phényl)-4-spiro(2H-benzopyranne-1-2,1'-cyclopentyl)-3)-7-heptène-6-oate de méthyle.

Composé n° 30 : (+,-)-6E-Erythro-((tertiobutyl-4-phényl)-4-spiro (2H-benzopyranne-1-2,1'-cyclopentyl-3)-7-dihydroxy-3,5-heptène-6-oate d'éthyle.

Composé n° 31 : (+,-)-6E-Erythro-(fluoro-4-phényl)-4-spiro (2H-benzopyranne-1-2,1'-cyclohexyl)-3)-7-dihydroxy-3,5-heptène-6-oate d'éthyle.

Composé n° 32 : (+,-)-6E-Erythro-((fluoro-4-phényl)-4-isopropyloxy-7-spiro(2H-benzopyranne-1-2,1'-cyclopentyl)-3)-7-dihydroxy-3,5-heptène-6-oate de méthyle.

Composé n° 33 : (+,-)-6E-Erythro-((fluoro-4-phényl)-4-spiro (2H-benzopyranne-1-2,1'-cyclopentyl)-3)-7-dihydroxy-3,5-heptène-6-amide.

Composé n° 34 : (+,-)-6E-Erythro-((fluoro-4-phényl)-4-spiro (2H-benzopyranne-1-2,1'-cyclopentyl)-3)-7-dihydroxy-3,5-N-isopropyl-heptène-6 amide.

Composé n° 35 : (+,-)-6E-Erythro-((biphényl-1,1'-yl-4)-4-spiro (2H-benzopyranne-1-2,1'-cyclopentyl)-3)-7-dihydroxy-3,5-N,N-tétraméthylène-heptène-6-amide.

Composé n° 36 : (+,-)-6E-Erythro-((fluoro-4-phényl)-4-spiro (2H-benzopyranne-1-2,1'-cyclopentyl)-3)-7-dihydroxy-3,5-N-cyclohexyl-heptène-6-amide.

Composé n° 37 : (+,-)-6E-Erythro-N-benzyl-((fluoro-4-phényl)-4-spiro(2H-benzopyranne-1-2,1'-cyclopentyl)-3)-7-dihydroxy-3,5-heptène-6-amide.

Composé n° 38 : (+,-)-6E-Erythro-((éthyl-4-phényl)-4-spiro (2H-benzopyranne-1-2,1'-cyclopentyl)-3)-7-dihydroxy-3,5-heptène-6-oate de sodium.

Composé n° 39 : (+,-)-6E-Erythro-dihydroxy-3,5-(méthyl-6-phényl-4-spiro(2H-benzopyranne-1-2,1'-cyclopentyl)-3)-7-heptène-6-oate de sodium.

Composé n° 40 : (+,-)-6E-Erythro-dihydroxy-3,5-((isopropyl-4-phényl)-4-spiro(2H-benzopyranne-1-2,1'-cyclopentyl)-3)-7-heptène-6-oate de sodium.

Composé n° 41 : (+,-)-6E-Erythro-dihydroxy-3,5-(phényl-4-spiro (2H-benzopyranne-1-2,1'-cyclopentyl)-3)-7-heptène-6-oate de sodium.

Composé n° 42 : (+,-)-6E-Erythro-((chloro-4-phényl)-4-spiro (2H-benzopyranne-1-2,1'-cyclopentyl)-3)-7-dihydroxy-3,5-heptène-6-oate de sodium.

Composé n° 43 : (+,-)-6E-Erythro-dihydroxy-3,5-(phényl-4-2H-benzopyranne-1-yl-3)-7-heptène-6-oate d'éthyle.

Composé n° 44 : (+,-)-6E-Erythro-((fluoro-4-phényl)-4-diméthyl-2,2-2H-benzopyranne-1-yl-3)-7-dihydroxy-3,5-heptène-6-oate de méthyle.

Composé n° 45 : (+,-)-6E-Erythro-((fluoro-4-phényl)-4-isopropyl-2-2H-benzopyranne-1-yl-3)-7-dihydroxy-3,5-heptène-6-oate d'éthyle.

Composé n° 46 : (+,-)-Trans-hydroxy-4-tétrahydro-3,4,5,6-(1E-phényl-4-spiro(2H-benzopyranne-1-2,1'-cyclopentyl)-3)-2-éthényl)-6 -2H-pyrannone-2.

Composé n° 47 : (+,-)-Trans-(1E((fluoro-4-phényl)-4-méthoxy-6-spiro(2H-benzopyranne-1-2,1'-cyclopentyl)-3)-2-éthényl)-6-hydroxy-4-tétrahydro-3,4,5,6-2H-pyrannone-2.

Composé n° 48 : (+,-)-Trans-(1E(dihydro-1,2-diméthyl-2,2-phényl-4-naphtyl-3)-2-éthényl)-6-hydroxy-4-tétrahydro-3,4,5,6-2H-pyrannone-2.

Composé n° 49 : (+,-)-Trans-hydroxy-4-tétrahydro-3,4,5,6-(1E(2H-diméthyl-2,2 phényl-4-benzothiapyrannyl-3)-2-éthényl)-6-2H-pyrannone-2.

Composé n° 50 : (+,-)-6E-Erythro-dihydroxy-3,5-(phényl-4-spiro(dihydro-1,2-naphtalène-2,1'-cyclopentyl)-3)-7-heptène-6-oate de méthyle.

Composé n° 51 : (+,-)-6E-Erythro-((fluoro-4-phényl)-4-dihydro-1,2-diméthyl-2,2-naphtyl-3)-7-dihydroxy-3,5-heptène-6-oate de méthyle.

Composé n° 52 : (+,-)-6E-Erythro-(dihydro-1,2-diméthyl-2,2-phényl-4-naphtyl-3)-7-dihydroxy-3,5-heptène-6-oate de méthyle.

Composé n° 53 : (+,-)-6E-Erythro-((fluoro-4-phényl)-4-spiro(dihydro-1,2-naphtalène-2,1'-cyclopentyl-3)-7-dihydroxy-3,5-heptène-6-oate de méthyle.

Composé n° 54 : (+,-)-6E-Erythro-((chloro-4-phényl)-4-spiro(dihydro-1,2-naphtalène-2,1'-cyclopentyl)-3)-7-dihydroxy-3,5-heptène-6-oate de méthyle.

Composé n° 55 : (+,-)-6E-Erythro-((chloro-4-phényl)-4-dihydro-1,2-diméthyl-2,2-naphtyl-3)-7-dihydroxy-3,5-heptène -6-oate de méthyle.

Composé n° 56 : (+,-)-6E-Erythro-((fluoro-3-phényl)-4-spiro(dihydro-1,2-naphtalène-2,1'-cyclopentyl-3)-7-dihydroxy-3,5-heptène-6-oate de méthyle.

Composé n° 57 : (+,-)-6E-Erythro-((fluoro-4-phényl)-4-dihydro-1,2-naphtyl-3)-7-dihydroxy-3,5-heptène-6-oate de sodium.

Composé n° 58 : (+,-)-6E-Erythro-(dihydro-1,2-isopropyl-2-phényl-4-naphtyl-3)-7-dihydroxy-3,5-heptène-6-oate de méthyle.

Composé n° 59 : (+,-)-6E-Erythro-(dihydro-1,2-méthyl -2-phényl-4-naphtyl-3)-7-dihydroxy-3,5-heptène-6-oate de méthyle.

Composé n° 60 : (+,-)-6E-Erythro-dihydroxy-3,5-(méthyl-8-(méthyl-4-phényl)-4-spiro(dihydro-1,2-naphtalène-2,1'-cyclopentyl)-3)-7-heptène-6-oate de méthyle.

Composé n° 61 : (+,-)-6E-Erythro-(chloro-6-(chloro-4-phényl)-4-spiro(dihydro-1,2-naphtalène-2,1'-cyclopentyl)-3) -7-dihydroxy-3,5-heptène-6-oate de méthyle.

Composé n° 62 : (+,-)-6E-Erythro-dihydroxy-3,5-(méthyl -8-(méthoxy-4-phényl)-4-spiro(dihydro-1,2-naphtalène-2,1'-cyclopentyl)-3)-7-heptène-6-oate de méthyle.

Composé n° 63 : (+,-)-6E-Erythro-(chloro-6-(fluoro-4-

Composé n° 63 : (+,-)-6E-Erythro-(chloro-6-(fluoro-4-phényl)-4-spiro(dihydro-1,2-naphtalène-2,1'-cyclopentyl)-3) -7-dihydroxy-3,5-heptène-6-oate de méthyle.

Composé n° 64 : (+,-)-6E-Erythro-(dihydro-1,2-diméthyl -2,2-méthoxy-7-naphtyl-3)-7-dihydroxy-3,5-heptène-6-oate de méthyle.

Composé n° 65 : (+,-)-6E-Erythro-dihydroxy-3,5-(phényl -4-spiro(2H-benzothiapyranne-1-2,1'-cyclopentyl)-3)-7-heptène-6-oate de sodium.

Composé n° 66 : (+,-)-6E-Erythro-dihydroxy-3,5-((méthyl-4-phényl)-4-spiro(2H-benzothiapyranne-1-2,1'-cyclopentyl)-3) -7-heptène-6-oate de méthyle.

Composé n° 67 : (+,-)-6E-Erythro-dihydroxy-3,5-(phényl -4-diméthyl-2,2-2H-benzothiapyrannyl-3)-7-heptène-6-oate de méthyle.

Composé n° 68 : (+,-)-6E-Erythro-dihydroxy-3,5-(phényl -4-diméthyl-2,2-2H-benzothiapyrannyl-3)-7-heptène-6-oate de sodium.

L'invention concerne aussi les procédés de préparation des composés selon l'invention caractérisés en ce qu'ils comportent au moins,

a) la réduction d'un céto-ester de formule générale **4**, dans laquelle X, $R_1$, $R_2$, $R_3$, $R_4$, $R_5$, $R_6$, $R_7$, $R_8$ et $R_{11}$ ont les significations générales ou particulières déjà définies,

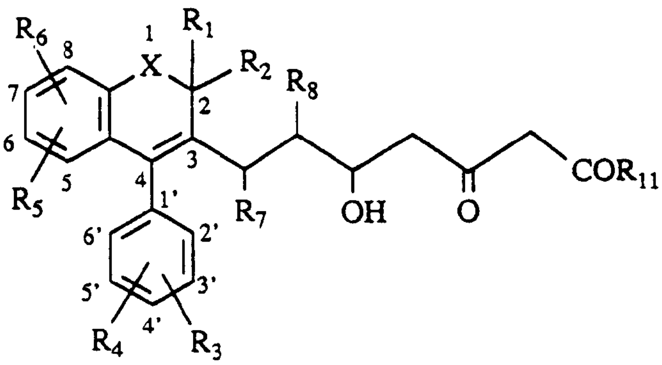

**4**

et le cas échéant,

b) la transestérification de composés de formule **1** ou l'alcoolyse de composés de formule **1** sous forme δ-lactone ou bien l'alkylation de composés de formule **1** sous forme de sels,

c) l'hydrolyse de composés de formule **1** sous forme ester ou δ-lactone,

d) lorsque $R_7$ et $R_8$ respectivement $R_9$ et $R_{11}$ forment une liaison simple : le traitement des composés correspondants de formule **1** sous forme de sels par une chloroamine tertiaire,

e) lorsque $R_7$, $R_8$ et $R_{10}$ désignent un atome d'hydrogène et $R_9$ et $R_{11}$ forment ensemble une liaison : la réduction catalytique des composés δ-lactoniques de formule **1** dans laquelle $R_7$ et $R_8$ forment une liaison ou la lactonisation des composés acides correspondants de formule **1** dans laquelle $R_7$, $R_8$ et $R_{10}$ désignent un atome d'hydrogène,

f) l'aminolyse des composés de formule **1** sous forme ester ou δ-lactone,

g) lorsque $R_9$ et $R_{10}$ forment ensemble un reste dialkyméthylène : la cyclisation des composés de formule **1** correspondants, dans laquelle $R_9$ et $R_{10}$ désignent chacun un atome d'hydrogène, par un alkoxyalcène.

Les composés de formule **1**, dans laquelle, X, $R_1$, $R_2$, $R_3$, $R_4$, $R_5$, $R_6$, $R_7$ et $R_8$ ont les significations générales ou particulières définies précédemment et dans laquelle les substituants $R_9$ et $R_{10}$ désignent chacun un atome d'hydrogène peuvent être préparés, sous forme ester, par la séquence réactionnelle illustrée par le schéma I suivant,

## SCHEMA I

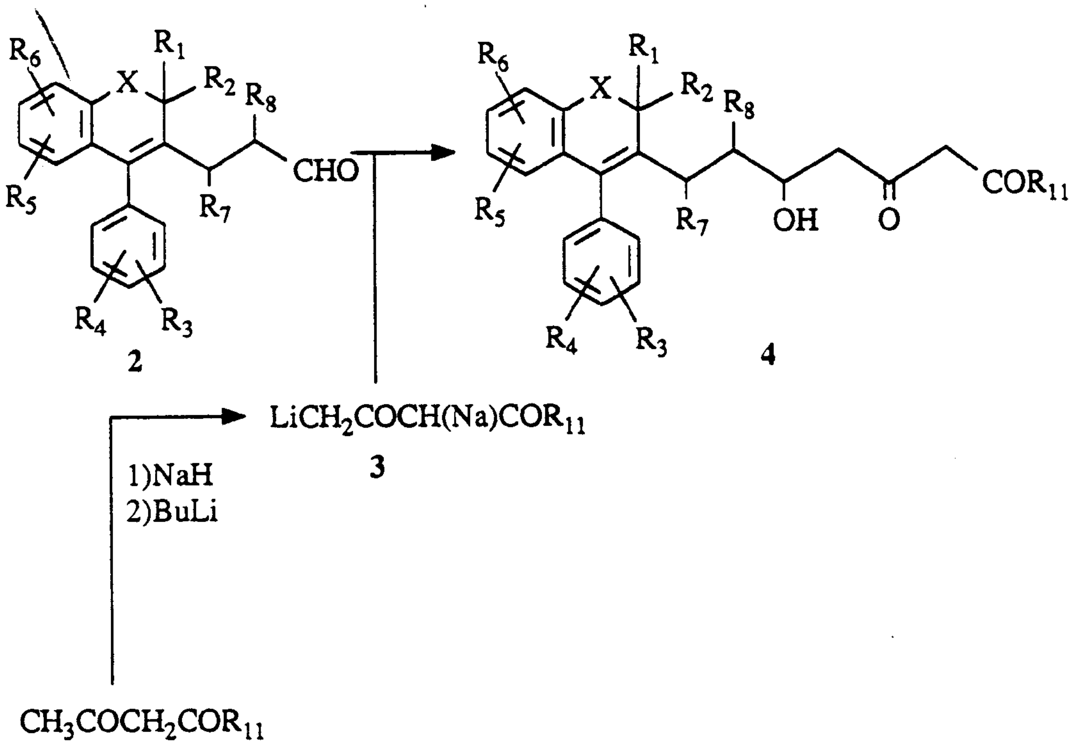


2, 3, 4

**SCHEMA I (suite)**

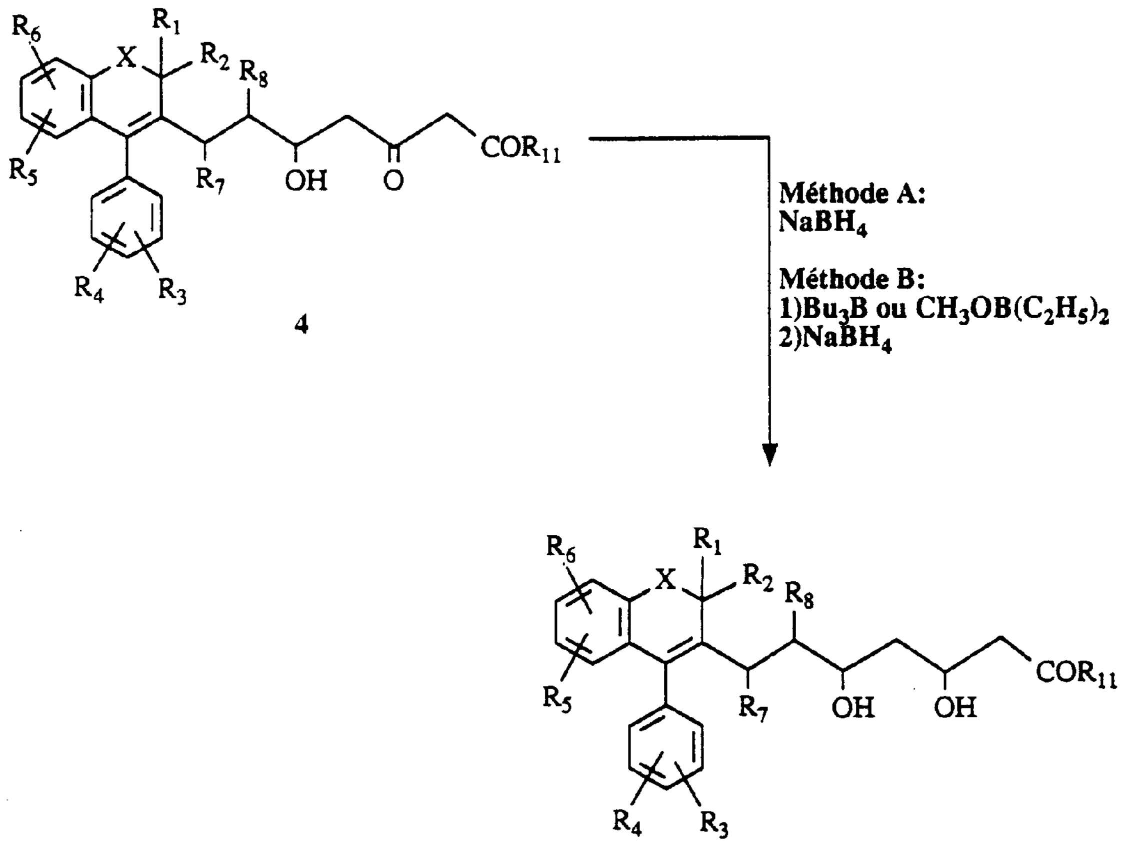


4

1

Comme indiqué sur le schéma I, les composés aldéhydiques de formule **2** dans laquelle les substituants $R_1$, $R_2$, $R_3$, $R_4$, $R_5$, $R_6$, $R_7$ et $R_8$ ont les significations particulières ou générales déjà définies, sont soumis à une condensation aldolique avec l'acétoacétate approprié sous forme de disel mixte de sodium et de lithium de formule **3** dans laquelle $R_{11}$ a les significations générales ou particulières déjà définies, au sein d'un solvant polaire, comme le THF (cf. KRAUS et coll., J. Org. Chem., **48**, 2111, 1983), pour donner les hydroxy-5-oxo-3-heptène-6-oate-7-substitués de formule **4**.

Les esters **4**, en solution dans un solvant inerte, comme le THF ou l'éther, traités par un borohydrure alcalin de préférence le borohydrure de sodium (méthode A) conduisent aux composés **1** sous forme d'un mélange d'isomères thréo et érythro, lesquels peuvent être séparés par les méthodes physicochimiques usuelles telles que la chromatographie par exemple.

Une variante (méthode B) préférée à la méthode A en ce qu'elle fournit les composés selon l'invention sous forme érythro, consiste, préalablement à la réduction avec un borohydrure alcalin, à traiter les esters **4** en solution dans un solvant approprié tel que le THF, par un agent complexant de préférence un trialkylborane comme le tributylborane (cf. NARASAKA, Chem. Letters, 1415-1418, 1980) ou un alkoxy dialkylborane comme le méthoxydiéthylborane (cf. CHEN et coll., Tetrahed. Lett. **28** (2), 155-158, 1987).

Les composés de formule **2**, dans laquelle les substituants $R_7$ et $R_8$ forment ensemble une liaison, peuvent être préparés selon la séquence réactionnelle illustrée parle schéma II suivant,

## SCHEMA II

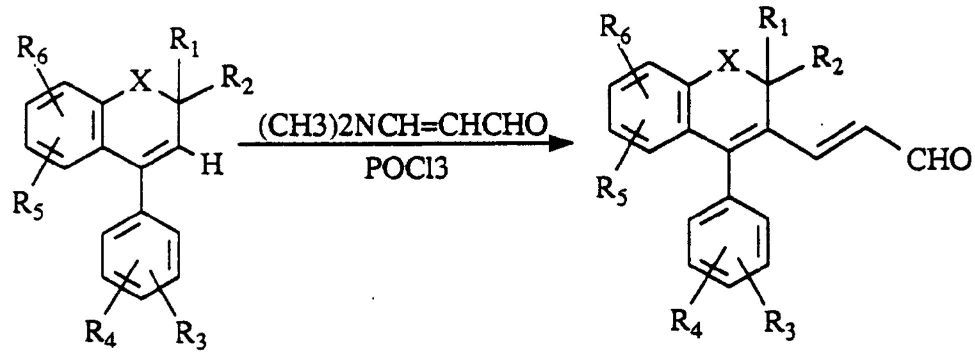


5 2

Comme indiqué sur le schéma II, les composés de formule **5**, dans laquelle X, $R_1$, $R_2$, $R_3$, $R_4$, $R_5$ et $R_6$ ont les significations générales ou particulières déjà définies sont soumis à une réaction de Vilsmeier avec la N,N-diméthylamino-3-acroléïne (cf. ULLRICH et BREITMAIER, Synthesis **8**, 641-645, 1983), dans un solvant inerte comme l'acétonitrile et à une température comprise entre 20°C et la température de reflux, pour donner les composés **2** de géométrie trans comme l'indique la spectroscopie RMN.

Un autre procédé de préparation des aldéhydes de formule **2**, dans laquelle, $R_7$ et $R_8$ forment ensemble une liaison est illustré par le schéma III suivant,

## SCHEMA III

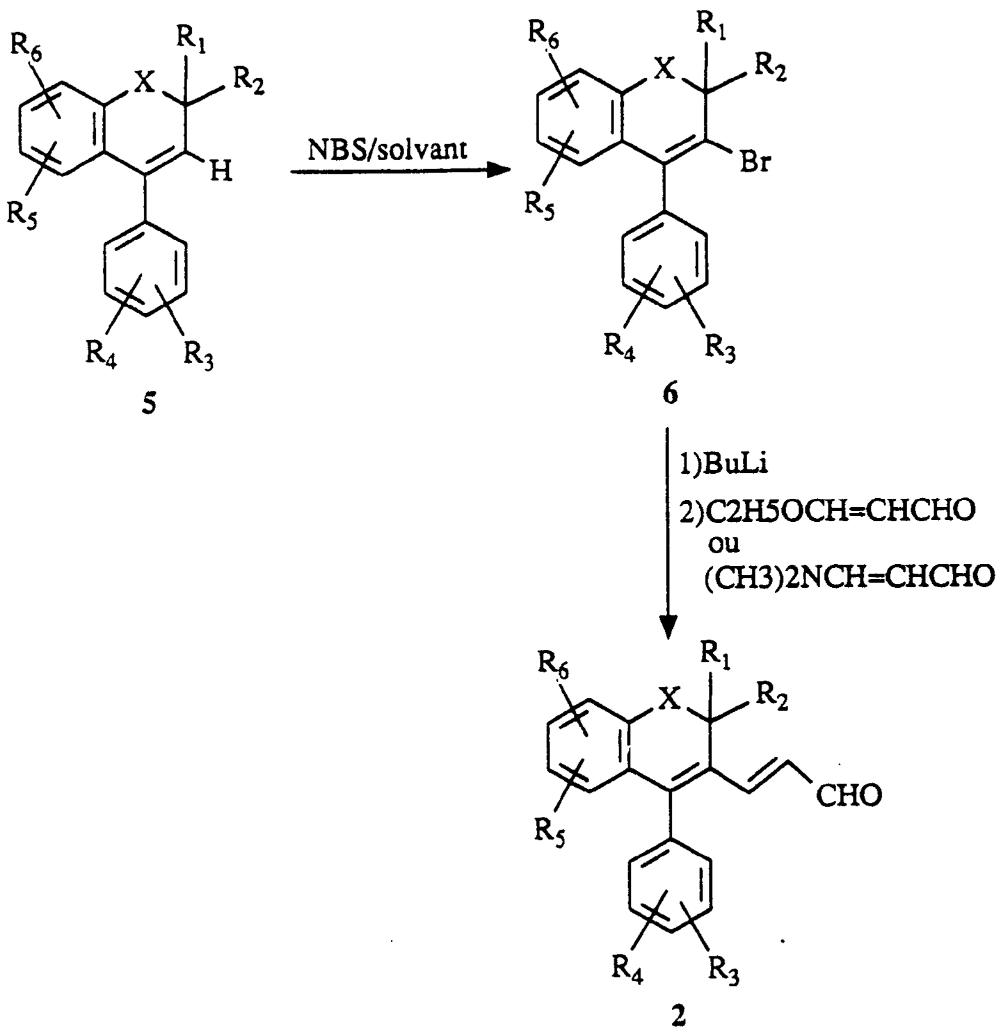


5

6

2

Comme indiqué sur le schéma III, les composés de formule **5** sont soumis à une réaction de bromation par le N-bromosuccinimide (NBS) au sein du N,N-diméthylformamide (DMF) ou d'un solvant chloré comme le tétrachlorure de carbone ou le chlorure de méthylène, pour produire les composés 3-bromés **6** correspondants, lesquels sont successivement traités par le butyllithium dans un solvant approprié, de préférence un éther comme par exemple le diéthyléther puis par l'éthoxy-3-acroléïne ou la N,N-diméthylamino-3-acroléïne pour former les aldéhydes **2** sous forme trans comme l'indique la spectroscopie RMN.

Une alternative pour préparer les intermédiaires aldéhydiques de formule **2** dans laquelle $R_7$ et $R_8$ forment ensemble une liaison, est illustrée par le schéma IV suivant,

## SCHEMA IV

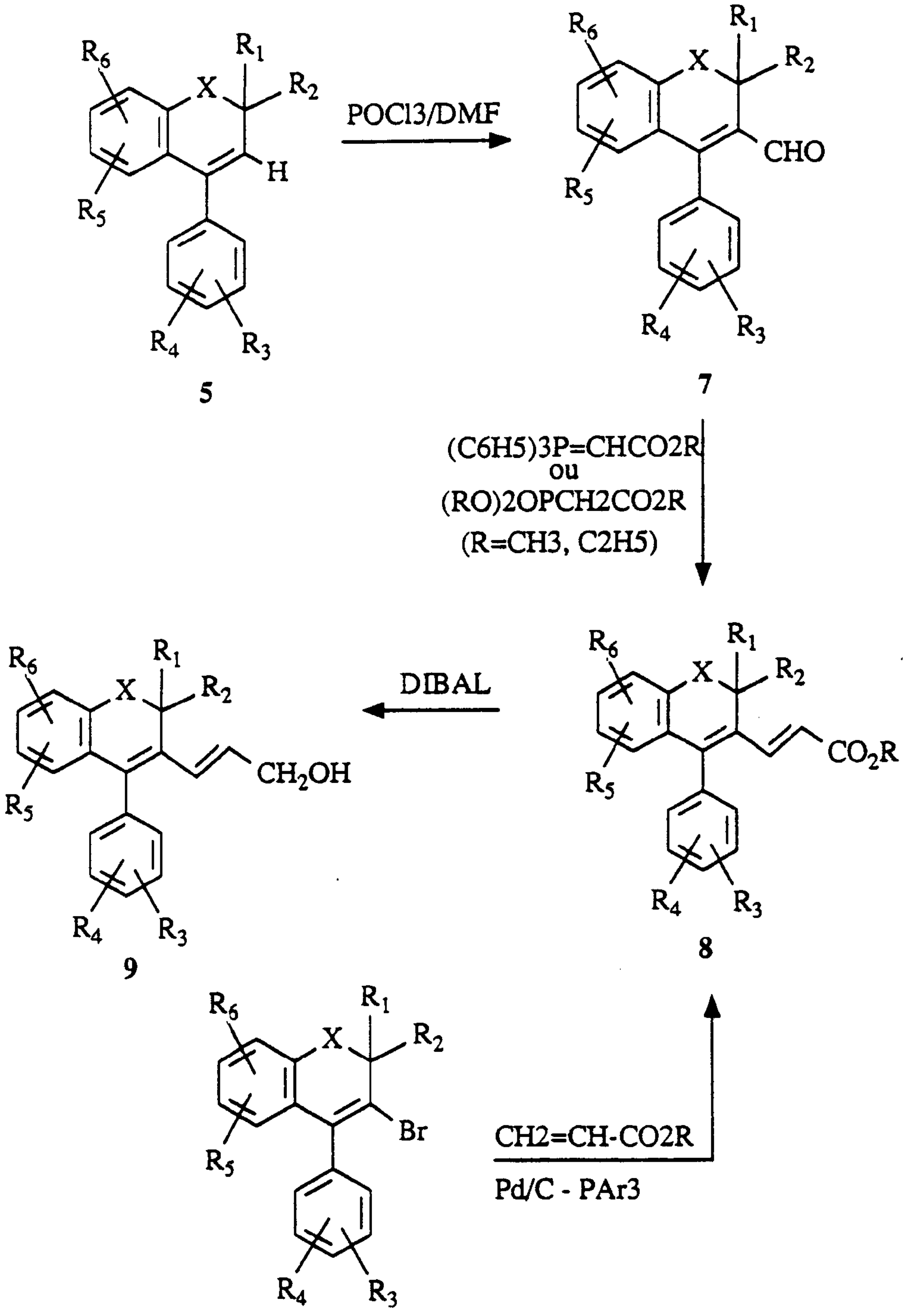


5

7

9

8

6

## SCHEMA IV (suite)

9

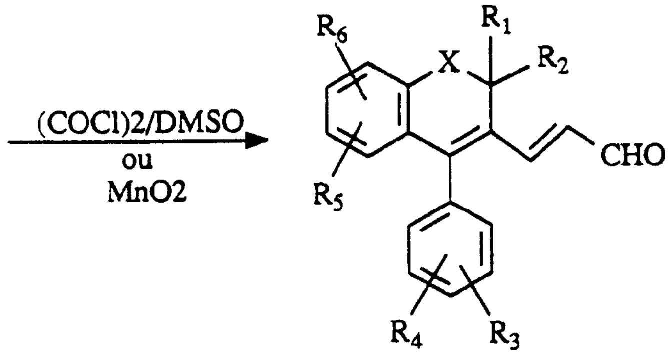


2

Comme indiqué sur le schéma IV, les composés de formule **5** sont soumis à une réaction de Vilsmeier avec le N,N-diméthylformamide (cf. JACKSON et Coll. J. Am. Chem. Soc. **103**, 533, 1981) au sein d'un solvant inerte pour former les aldéhydes 7 correspondants, lesquels sont soumis à une réaction de WITTIG avec l'éthoxy- ou le méthoxy-carbonyl méthylène triphényl phosphorane, ou encore avec un phosphonoacétate de méthyle ou d'éthyle (cf. Organic Reactions, **14**, 273, 1965) pour donner les esters propénoïque 3-substitués **8** dont la géométrie comme l'indique la spectroscopie de RMN est trans.

Une alternative pour préparer les composés intermédiaires de formule **8** qui est particulièrement préférée lorsque X désigne un atome de soufre, consiste à faire réagir un composé bromé de formule **6** avec un acrylate d'alkyle, de préférence l'acrylate de méthyle ou d'éthyle en présence d'un agent basique tel que la triéthylamine ou le bicarbonate de sodium, de palladium dispersé sur charbon ou d'un dérivé du palladium comme le dichlorure de palladium ou son acétate et d'un ligand, de préférence une triarylphosphine comme par exemple la triorthotolyl phosphine au sein d'un solvant adapté, comme le N,N-diméthylformamide.

Les esters **8** sont réduits, par l'hydrure de diisobutylaluminium (DIBAL) au sein d'un solvant, généralement l'éther diéthylique ou le THF, en les propénol 3-substitués **9** trans correspondants lesquels sont ensuite soumis à une oxydation ménagée par le chlorure d'oxalyle dans le DMSO (cf. SWERN et Coll., J. Org. Chem., **43**, 2480, 1978), ou par le dioxyde de manganèse dans un solvant approprié comme par exemple le THF, pour donner les aldéhydes de formule **2** de géométrie trans.

Les aldéhydes de formule **2**, dans laquelle, les substituants $R_7$ et $R_8$ représentent chacun un atome d'hydrogène peuvent être préparés selon la séquence réactionnelle illustrée par le schéma V suivant,

SCHEMA V

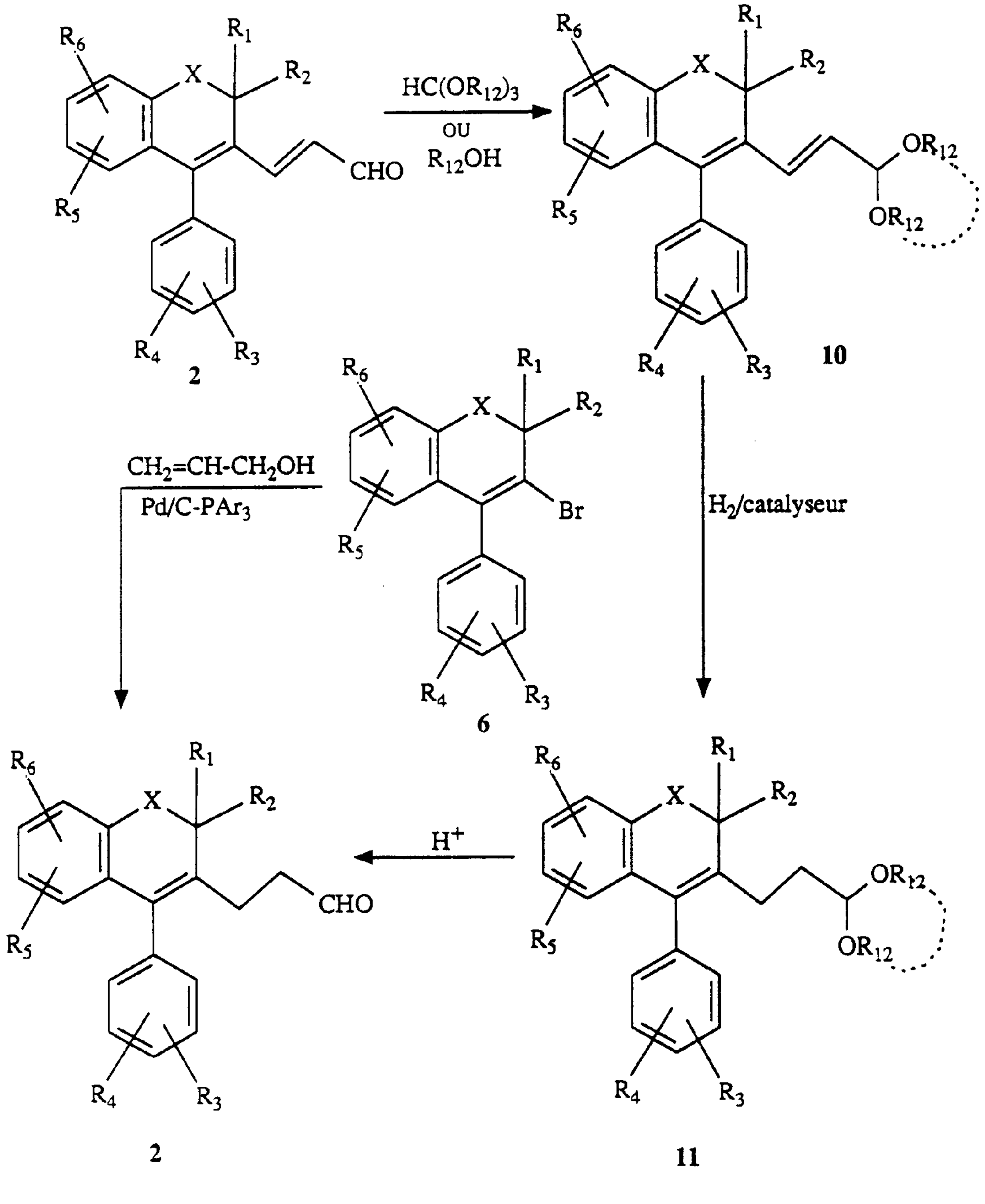


Selon la séquence réactionnelle du schéma V, les aldéhydes de formule **2** où $R_7$ et $R_8$ forment une double liaison sont soumis à une réaction d'acétalisation par exemple par un orthoformiate de formule $HC(OR_{12})_3$, dans laquelle, $R_{12}$ représente un radical alkyle de 1 à 4 atomes de carbone, de préférence méthyle ou éthyle en présence d'une résine acide ou encore à une réaction acidocatalysée avec un alcool de formule $R_{12}OH$ dans laquelle $R_{12}$ a les significations ci-dessus ou peut représenter un radical de formule $-(CH_2)_qOH$, dans laquelle q = 2, ou 3, pour former les composés **10**, le radical méthylène $-R_{12}....R_{12}-$ forme alors avec les atomes d'oxygène auxquels il est rattaché un cycle à 5 ou 6 chaînons.

Les composés **10** sont ensuite hydrogénés, de préférence sous faible pression et en présence d'un catalyseur métallique, comme par exemple le palladium dispersé sur charbon, dans un solvant approprié comme

par exemple le THF ou un alcool comme le méthanol, en les acétals des propanaldéhydes 3-substitués **11** correspondants, lesquels peuvent être ensuite désacétalisés en les propanaldéhydes **2** par les méthodes usuelles de désacétalisation, qui comprennent par exemple le traitement de l'acétal par une résine acide dans un mélange acétone-eau ou plus généralement par un catalyseur acide, dans un solvant ou un mélange de solvants appropriés.

Une alternative pour préparer les composés aldéhydiques de formule **2** dans laquelle les substituants $R_7$ et $R_8$ représentent chacun un atome d'hydrogène, consiste à faire réagir les composés bromés de formule **6** avec l'alcool allylique en présence d'un agent basique tel que la triéthylamine ou le bicarbonate de sodium, de palladium dispersé sur charbon ou d'un dérivé du palladium tel que le dichlorure de palladium ou son acétate et d'un ligand, de préférence une triarylphosphine comme par exemple la triorthotolyl phosphine au sein d'un solvant adapté comme le N, N-diméthylformamide.

Les composés de formule **5** peuvent être préparés selon la séquence illustrée par le schéma VI suivant,

## SCHEMA VI

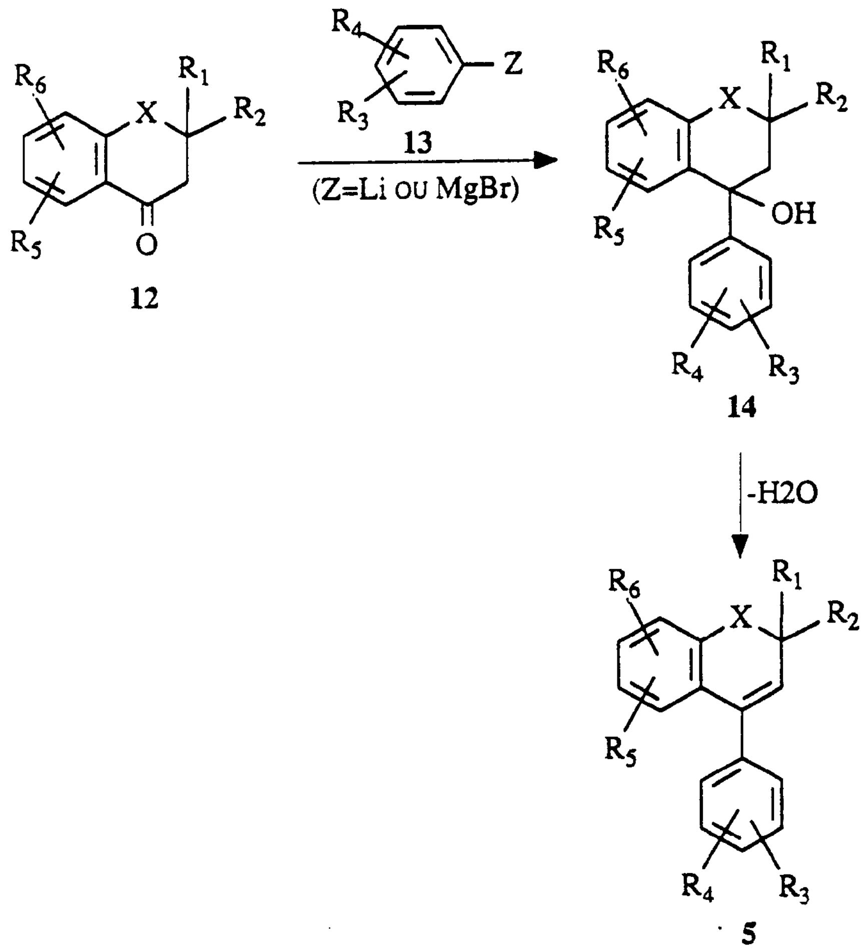


12

14

5

selon lequel, les composés cétoniques de formule **12**, dans laquelle X, $R_1$, $R_2$, $R_5$ et $R_8$ ont les significations particulières ou générales déjà définies, sont traités par le lithien ou le magnésien approprié de formule **13**, dans laquelle $R_3$ et $R_4$ ont les significations générales ou particulières définies précédemment, pour donner par addition 1-2 les alcools **14** correspondants, lesquels sont déshydratés en les composés **5** désirés par l'action d'un agent déshydratant comme le sulfate acide de potassium ou bien l'acide paratoluènesulfonique.

Les composés de formule **12**, dans laquelle X représente les atomes d'oxygène et de soufre et $R_1$, $R_2$, $R_8$ et $R_8$ ont les significations générales ou particulières définies précédemment, sont préparés selon le procédé de KABBE et coll. Synthesis, **12**, 886, 1978.

Les composés de formule **12**, dans laquelle X représente un chaînon méthylène et $R_1$, $R_2$, $R_8$ et $R_6$ ont les significations générales ou particulières énoncées ci-dessus sont préparés selon la séquence de réactions

illustrée par le schéma VII suivant,

## SCHEMA VII

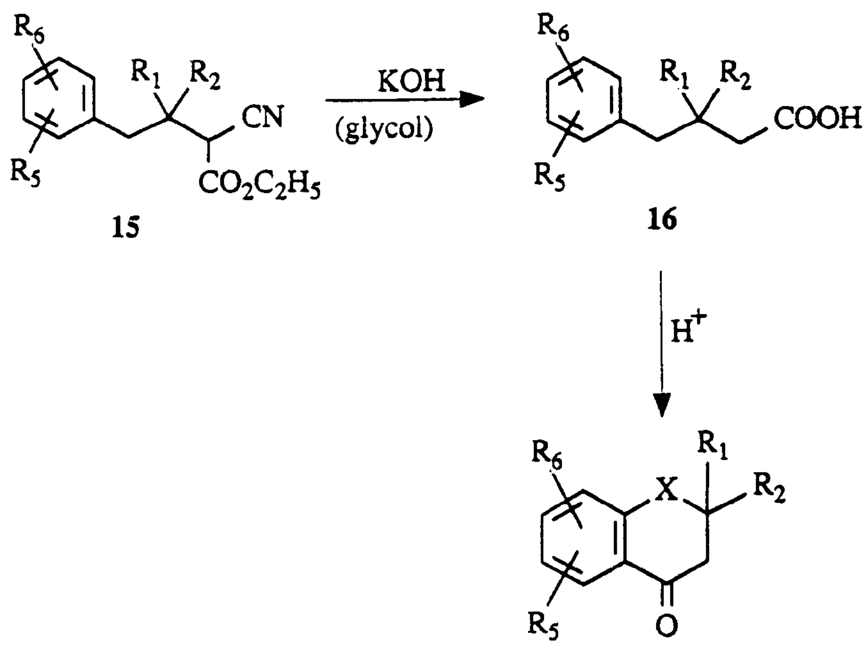


15 16 12

Comme l'indique le schéma VII, les cyanoesters de formule **15**, dans laquelle $R_1$, $R_2$, $R_5$ et $R_6$ ont les significations générales ou particulières déjà définies, sont hydrolysés et décarboxylés par la potasse glycolique en les acides **16** correspondants lesquels sont cyclisés en les composés **12** par chauffage en milieu acide. Les composés de formule **15** sont préparés selon le procédé décrit par PROUT et Coll., Org. Synth., Coll. vol. **IV**, 93, 1963.

Les composés de formule **1** sous forme ester peuvent être également préparés par transestérification de composés de formule **1**, différents par le substituant $R_{11}$, ou bien par alcoolyse de composés de formule **1** sous forme δ-lactone, ou encore par O-alkylation des composés de formule **1** sous forme de sels, de préférence les sels de sodium, par un bromure ou un iodure de formule $R_{11}$-Br(I), dans laquelle, $R_{11}$ a les significations générales ou particulières définies précédemment.

Les esters répondant à la formule **1**, dans laquelle, les substituants $R_7$ et $R_5$ représentent chacun un atome d'hydrogène peuvent aussi être préparés par hydrogénation des esters correspondants de formule **1**, dans laquelle, les substituants $R_7$ et $R_8$ forment une liaison, de préférence sous faible pression, en présence d'un catalyseur métallique comme le palladium ou l'oxyde de platine dans un solvant approprié comme le THF par exemple.

Les composés de formule **1** sous forme de sels, peuvent être préparés par hydrolyse basique, par exemple, par la soude ou la potasse, dans un solvant adapté, de préférence un alcool, des composés correspondants de formule **1** sous forme ester, de préférence les esters méthyliques ou éthyliques, ou bien sous forme δ-lactone.

Les composés δ-lactoniques de formule **1**, dans laquelle, les substituants $R_7$ et $R_8$ forment ensemble une liaison peuvent être préparés en faisant réagir les composés correspondants de formule **1** sous forme de sels, de préférence les sels de sodium, avec une chloro amine, de préférence la chloro-2-éthyl-N,N-diéthylamine dans un solvant approprié, de préférence carbonylé comme l'acétone ou la butanone.

Les composés δ-lactoniques de formule **1**, dans laquelle $R_7$, $R_8$ et $R_{10}$ représentent chacun un atome d'hydrogène, sont préparés par lactonisation des acides de formule **1** correspondants c'est à dire dans la formule desquels $R_{11}$ représente un radical hydroxyle : la réaction de lactonisation est de préférence réalisée par chauffage des acides dans un solvant inerte aromatique, tel que le benzène, le toluène, le xylène ou leur mélange, le cas échéant en présence d'un agent déshydratant, comme, par exemple, l'acide paratoluènesulfonique ;

un autre procédé très apprécié pour préparer les composés δ-lactoniques de formule **1**, dans laquelle, $R_7$, $R_8$ et $R_{10}$ représentent un atome d'hydrogène, consiste à hydrogéner, en phase hétérogène, les composés de formule **1** insaturés correspondants c'est à dire dans la formule desquels, $R_7$ et $R_8$, respectivement $R_9$ et $R_{11}$ forment ensemble une liaison, en présence d'un catalyseur métallique, de préférence, le palladium dispersé sur charbon ou bien l'oxyde de platine, au sein d'un solvant approprié, de préférence un éther comme le THF ou un alcool comme le méthanol ou l'éthanol.

Les composés acides de formule **1** dans laquelle $R_9$ et $R_{10}$ désignent chacun un atome d'hydrogène, peuvent être préparés par acidification des composés correspondants de formule **1** sous forme de sels ou encore par hydrogénolyse, en présence de palladium dispersé sur carbone, des esters benzyliques correspondants c'est-à-dire des composés de formule **1** dans laquelle $R_{11}$ représente un reste benzyloxy.

Les composés de formule **1** sous forme amide sont préparés par aminolyse par un excès d'amine au sein d'un solvant polaire de préférence le méthanol ou l'éthanol, des esters correspondants de formule **1** de préférence les esters méthyliques ou éthyliques ou des composés correspondants de formule **1** sous forme δ-lactone.

Les composés de formule **1**, dans laquelle les substituants $R_9$ et $R_{10}$ forment ensemble un groupe dialkylméthylène en C1-3, sont préparés par réaction des composés de formule **1** correspondants, dans laquelle $R_9$ et $R_{10}$ désignent chacun un atome d'hydrogène avec l'alkoxyalcène approprié (le terme alkoxyalcène signifiant, un alcène de 3 à 7 atomes de carbone substitué sur la double liaison par un radical alkoxy, tel que défini ci-dessus, de préférence méthoxy) dans un solvant approprié comme le N,N-diméthylformamide par exemple et en présence d'un catalyseur acide comme l'acide paratoluènesulfonique.

Il convient de remarquer que les composés de l'invention sous leurs différentes formes : acide, sel, ester, amide ou lactone, peuvent être interconvertis selon les procédés décrits ci-dessus, en conséquence ces différentes formes constituent également des intermédiaires utiles pour la synthèse des composés selon l'invention.

Les mélanges de stéréoisomères (cis, trans, thréo, érythro, énantiomères) peuvent être séparés, par les méthodes usuelles, à celui des stades de la synthèse qui est le plus approprié.

Par méthodes usuelles, on entend l'ensemble des procédés familiers au spécialiste comme par exemple, la recristallisation, la chromatographie ou la formation de combinaisons avec des composés optiquement actifs.

Les composés intermédiaires de synthèse répondant aux formules **2, 4, 5, 6, 7, 8, 9, 10, 11, 14** font également partie de l'invention, à l'exception des produits de formule **5** où $R_1$ et $R_2$ représentent H et X représente S ou $CH_2$, certains de ces composés étant connus d'après Degani, Ann. Chem. (Rome) 1971, 61(2), p 793 à 813 et d'après Penn, J.Magn. Reson. 1975, 18 (1), p 6 à 11.

La présente invention concerne également les procédés de préparation de ces intermédiaires de synthèse.

Les composés de formule **1** ont la propriété d'inhiber l'hydroxy-3 méthyl-3 glutaryl coenzyme A réductase, et par voie de conséquence, exercent un puissant effet hypocholestérolémiant.

Les composés de formule **1** sont également capables d'antagoniser les récepteurs du thromboxane $A_2$, propriété qui se traduit par une action antiagrégante plaquettaire.

Ces propriétés des composés de l'invention, rendent particulièrement intéressante leur utilisation comme médicament pour le traitement d'affections cardiovasculaires variées comme par exemple, les manifestations thrombotiques du diabète, l'athérosclérose, les hyperlipoprotéinémies.

Par ailleurs, les composés de l'invention sont doués de propriétés antifongiques qui leur confèrent un grand intérêt en tant que médicaments à action antimycotique.

Les propriétés pharmacologiques des composés selon l'invention ont été démontrées par les tests suivants,

Test A : mesure de la $^{14}C$-cholestérogénèse hépatique, in vivo, chez le rat selon la méthode décrite par BUCHER et Coll., J. Biol. Chem., **222**, 1-15, 1956 et ALBERTS et Coll., Proc. Natl. Acad. Sci. USA, **77** (7), 3957-3961, 1980.

Test B : mesure de la cholestérolémie totale, "in vivo", chez le rat traité en I.V. par le triton WR 1339 selon la méthode décrite par ENDO et coll., Biochim. Biophys. Acta, **575**, 226-276, 1979.

Test C : mesure de l'agrégation plaquettaire induite par un agoniste des récepteurs du $TXA_2$, chez le cobaye de la façon suivante : des plasmas riches en plaquettes (PRP) sont préparés par ponction aortique d'animaux anesthésiés ; le sang (9 vol.) est recueilli sur une solution 106 mM de citrate trisodique (1 vol.) pour empêcher la coagulation. Les PRP sont isolés par centrifugation lente (10 mn., 380 g), et mis en incubation pendant au moins 3 mn. (37°C, sous agitation lente) dans un agrégomètre de type CHRONOLOG-400. L'agrégation des plaquettes est induite par addition d'un agoniste direct des récepteurs du $TXA_2$, comme par exemple le composé connu sous le nom de code U 46619 (à 20 nM) selon MALMSTEIN, Life Sci., **18**, 169-178, 1976 ; les composés de l'invention sont testés "in vitro".

Par exemple on a obtenu pour les composés n° 1, 2, 19 et 65, sur le test A, respectivement les DE 50 de

0,17mg/kg, 0,6 mg/kg, 0,09 mg/kg et 0,38 mg/kg et pour la Lovastatine, dans les mêmes conditions, la DE 50 de 0,39mg/kg.

On a obtenu pour les mêmes composés, sur le test B, respectivement les DE 25 de 29, 100, 110 mg/kg et 74 mg/kg et pour la Lovastatine dans les mêmes conditions, la DE 25 de 130 mg/kg.

Sur le test C on a obtenu pour les composés n° 1 et 19 les CI 50 de 91 et 28 x $10^{-6}$ Mole/litre.

La présente invention concerne également les médicaments constitués par les composés de formule générale **1** pris tels que, à l'état pur ou sous forme d'associations avec tout autre produit acceptable du point de vue pharmaceutique lequel peut être inerte ou physiologiquement actif.

Ces médicaments peuvent être administrés selon une grande variété de formes posologiques différentes telles que comprimés, gélules, capsules, poudres, granulés, etc... Dans ces compositions, le principe actif est mélangé à un ou plusieurs diluants inertes tels que lactose ou amidon, en outre ces compositions peuvent comprendre des substances autres que diluants par exemple des lubrifiants tels que talc ou stéarate de magnésium ; lorsqu'on désire des suspensions aqueuses, élixirs ou sirops pour administration orale, l'ingrédient actif essentiel peut y être associé à divers édulcorants et/ou aromatisants, le cas échéant des émulsionnants et/ou des agents de mise en suspension en même temps que des diluants tels que l'eau, l'éthanol, le propylène glycol, et diverses associations similaires.

Les compositions pharmaceutiques selon l'invention, se prêtant à l'administration par voie orale et présentées sous forme de dose unitaire, contiennent de 1 à 500 mg de principe actif.

L'exemple suivant, donné à titre non limitatif, illustre une composition de ce type.

EXEMPLE :

| | | |
|---|---|---|
| Principe actif.......................... | 10 | mg |
| Lactose................................. | 104 | mg |
| Amidon de blé........................... | 30 | mg |
| Talc.................................... | 2,5 | mg |
| Polyvidone excipient.................... | 3 | mg |
| Stéarate de magnésium................... | 0,5 | mg |

L'invention est illustrée par les exemples non limitatifs ci-après dans lesquels :

- Toutes les évaporations sont exécutées, sauf indication contraire, dans un évaporateur rotatif sous pression réduite.
- Les températures sont exprimées en degrés centigrades (°C).
- Lorsqu'il est indiqué "température ambiante", il s'agit d'une température comprise entre 18 et 25°C.
- Sauf indication contraire, le degré d'avancement de la réaction est contrôlé par chromatographie en couche mince (CCM).
- Les nouveaux composés sont le cas échéant caractérisés par leurs constantes physiques, point de fusion noté PF ou point d'ébullition noté Eb suivi éventuellement de l'indication de la pression exprimée en millibars.
- Les rendements éventuellement indiqués sont donnés à titre uniquement illustratif et ne sont pas les plus élevés possible.
- Les spectres de résonnance magnétique nucléaire, sauf indication contraire, sont ceux des protons et sont relevés à 60 MHz en présence de tétraméthylsilane comme étalon interne ; les déplacements chimiques sont indiqués en ppm. ; les signaux sont décrits par les abréviations suivantes : s = singulet, d = doublet, dd = doublet de doublet, t = triplet, q = quartet, m = multiplet.
- Les spectres infrarouges des composés sont enregistrés à partir d'échantillons dispersés dans le bromure de potassium dans le cas de composés solides ou bien en film dans le cas des liquides.

**EXEMPLE 1** :

(+,-)-6E-Erythro-((fluoro-4-phényl)-4-spiro(2H-benzopyranne-1-2,1'-cyclopentyl)-3)-7-dihydroxy-3,5-heptène-6-oate d'éthyle (composé n° 1 - formule 1 : X = O, $R_1$-$R_2$ = $-(CH_2)_4-$, $R_3$ = 4'F, $R_4$ = $R_5$ = $R_6$ = $R_9$ = $R_{10}$ = H, $R_7$-$R_8$ = liaison, $R_{11}$ = $OC_2H_5$).

Stade 1 :

(Fluoro-4-phényl)-4-dihydro-3,4-hydroxy-4-spiro(2H-benzopyranne-1-2,1'-cyclopentane) - (Schéma VI - formule **14**)

On opère sous atmosphère d'azote dans un réacteur protégé de l'humidité. A 6,07 g (0,25 atome gr.) de magnésium en tournures en suspension dans 130 $cm^3$ de diéthyléther, on ajoute de façon à maintenir un doux reflux de l'éther, 43,75 g (0,25 mole) de bromo-1-fluoro-4-benzène en solution dans 200 $cm^3$ de diéthyléther. On maintient le mélange au reflux pendant 4 heures puis on refroidit à température ambiante et ajoute à cette température, une solution de 20 g (0,1 mole) de spiro(2H-benzopyranne-1-2,1'-cyclopentanone-4) [préparé selon Kabbe et coll., Synthesis **12**, 886, 1978] dans 60 $cm^3$ de diéthyléther, avec un débit nécessaire à maintenir un doux reflux de l'éther.

On agite le mélange pendant 2 heures à température ambiante puis on le verse sur une solution aqueuse glacée de chlorure d'ammonium en gros excès par rapport à la stoechiométrie ; on ajoute la quantité suffisante d'acide chlorhydrique dilué pour dissoudre complètement la suspension, puis on décante la phase organique ; on la lave à l'eau jusqu'à pH neutre, on la sèche sur sulfate de sodium, filtre et on évapore le solvant ; on obtient 30 g d'huile qui est utilisée telle quelle dans la suite de la synthèse.

Stade 2 :

(Fluoro-4-phényl)-4-spiro(2H-benzopyranne-1-2,1'-cyclopentane) (Schéma VI - formule **5**).

On chauffe sous pression réduite (16 mm de Hg) à la température de 120°C un mélange de 30 g (0,1 mole) du produit brut de l'étape 1 et de 3 g de $KHSO_4$ (1/10 du poids de composé à traiter) pendant 30 minutes. On dissout le résidu dans la quantité suffisante de dichlorométhane, on lave cette solution à l'eau, puis on la sèche sur sulfate de sodium, on la filtre et on évapore le solvant ; le solide obtenu est dispersé dans l'alcool méthylique.

PF = 76-8°C (diisopropyléther) - Rendement = 75 %.

(CCM : gel de silice : Acétate d'éthyle (AcOEt)-hexane : 5-95 : 1 tache

| Analyse centésimale : $C_{19}H_{17}FO$ | | PM = 280,33 | |
|---|---|---|---|
| | C | H | F |
| % calculé | 81,40 | 6,11 | 6,78 |
| % trouvé | 81,53 | 6,20 | 6,76 |

R.M.N. ($CDCl_3$) :
1,25-2,70 (m,8H) ; 5,73 (s,1H) ; 6,50-7,87 (m,8H)

Stade 3 :

2E-((Fluoro-4-phényl)-4-spiro(2H-benzopyranne-1-2,1'-cyclopentyl)-3)-3-propène-2-al. (Schéma II - formule **2**)

On opère sous atmosphère d'azote, dans un réacteur, protégé de l'humidité. Sauf indication contraire, pendant la durée des opérations suivantes, la température du mélange réactionnel est maintenue à -20°C. A une solution de 20,9 g (0,2 mole) de N,N-diméthylamino-3 acroléïne dans 200 $cm^3$ d'acétonitrile, on ajoute en l'espace de 30 mn, sous agitation, 29,1 g (0,19 mole) d'oxychlorure de phosphore ; on poursuit l'agitation pendant 10 mn, puis on ajoute en 15 mn, 11,2 g (0,04 mole) de composé du stade 2 en solution dans 100 $cm^3$ d'acétonitrile. On laisse ensuite le mélange revenir température ambiante puis on le chauffe au reflux jusqu'à disparition du produit de départ contrôlée par chromatographie couche mince ; dans le cas présent la durée nécessaire de chauffage est de 40 heures.

On évapore le mélange puis on verse le résidu dans 1000 $cm^3$ d'eau glacée, on neutralise (pH = 9) par addition de la quantité suffisante de soude concentrée, on agite la solution pendant 15 mn puis on l'extrait par 1000 $cm^3$ de chlorure de méthylène (2 x 500 $cm^3$).

On lave la couche organique à l'eau, on la sèche sur sulfate de sodium, on la filtre, on y ajoute 1000 $cm^3$ d' hexane et on l'agite en présence de 100 g de gel de silice. Après filtration et évaporation on obtient une huile qui cristallise en un solide jaune par dispersion dans l'hexane.

P.F. = 108-11°C (diisopropyléther) - Rendement 8,4 g = 63%

(CCM : gel de silice : AcOEt-hexane : 1-9 : 1 tache)

| Analyse centésimale : $C_{22}H_{19}FO_2$ | PM = 334,37 | | |
|---|---|---|---|
| | C | H | F |
| % calculé | 79,02 | 5,73 | 5,68 |
| % trouvé | 78,82 | 5,75 | 5,65 |

I.R. :
νCHO : 1670cm$^{-1}$
R.M.N. ($CDCl_3$) :
1,25-2,87 (m,8H) ; 5,98 (dd,1H J = 17 Hz et 7 Hz) ; 6,37-7,5 (m,9H) ; 9,25 (d,1H J = 7Hz)

Stade 4 :

(+,-)-6E-((Fluoro-4-phényl)-4-spiro(2H-benzopyranne-1-2,1'-cyclo pentyl)-3)-7-hydroxy-5-oxo-3-heptène-6-oate d'éthyle (Schéma I - formule **4**).
On opère sous atmosphère d'azote et à l'abri de l'humidité, en maintenant sauf indication contraire, la température du mélange à -20°C.
A une suspension de 1,2 g (0,05 mole) d'hydrure de sodium dans 300 cm³ de tétrahydrofuranne (THF) on ajoute 5,6 g (0,043 mole) d'acétoacétate d'éthyle en solution dans 25 cm³ de THF ; on agite le mélange pendant 20 mn puis on y ajoute en l'espace de 15-20 mn, 27 cm³ d'une solution 1,6 N de butyllithium dans l'hexane (soit 0,043 mole de BuLi) ; on poursuit l'agitation du mélange pendant 20 mn puis on y ajoute goutte à goutte, une solution de 8,7 g (0,026 mole) d'aldéhyde du stade 3 dans 140 cm³ de THF. On poursuit l'agitation du mélange pendant 3 heures, puis on y ajoute goutte à goutte entre -20°C et 10°C 40 ml d'une solution d'acide chlorhydrique 3N. On extrait à l'acétate d'éthyle, on lave à l'eau la phase organique jusqu'à pH 7, on la sèche sur sulfate de sodium, puis on l'évapore à sec ; on obtient une huile qui cristallise par dispersion dans l'hexane.
P.F. = 87-90°C (acétate d'éthyle) - Rendement = 110 g = 90,9 %
(CCM : gel de silice : AcOEt-hexane : 1-2 : 1 tache)

| Analyse centésimale : $C_{28}H_{29}FO_5$ | PM = 464,51 | | |
|---|---|---|---|
| | C | H | F |
| % calculé | 72,40 | 6,29 | 4,09 |
| % trouvé | 72,33 | 6,31 | 4,05 |

I.R. :
νOH : 3420 cm$^{-1}$
νCO : 1740 et 1710 cm$^{-1}$
R.M.N. ($CDCl_3$) :
1,32 (t, 3H) ; 1,5-2,35 (m,8H) ; 2,52 (d,2H) ; 3,92-4,65 (m,3H) ; 5,30 (dd,1H J = 15,8 Hz et 5,5 Hz) ; 6,00 (d,1H J = 15,8 Hz) ; 6,25-7,50 (m,8H).

Stade 5 :

(+,-)-6E-Erythro-((fluoro-4-phényl)-4-spiro(2H-benzopyranne-1-2, 1'-cyclopentyl)-3)-7-dihydroxy-3,5-heptène-6-oate d'éthyle (composé n° 1).
On opère sous atmosphère d'azote, dans un réacteur protégé de l'humidité.
Sauf indication contraire, pendant la durée des opérations suivantes, la température du mélange réactionnel est maintenue à -70°C.
A une solution de 3,25 g (0,007 mole) du céto ester du stade 4, dans un mélange de 65 cm³ de THF et de 15 cm³ de méthanol, on ajoute en l'espace de 5 mn, sous agitation, 7,7 cm³ d'une solution 1N de diéthylméthoxyborane dans le THF (soit 0,007 mole + 10 % de borane).
On poursuit l'agitation du mélange pendant 40 mn puis on y ajoute 0,29 g (0,007 mole + 10 %) de borohydrure de sodium. On poursuit l'agitation du mélange pendant 5 heures puis on l'acidifie par addition de 6,5 cm³ d'acide acétique, après quoi on y ajoute 80 cm³ d'acétate d'éthyle.
On laisse la température du mélange revenir à la température ambiante ; on lave ensuite le mélange par une solution aqueuse saturée de bicarbonate de sodium (2 x 200 cm³) puis par l'eau ; on décante la phase organique, on la sèche sur sulfate de sodium puis on évapore le solvant. L'huile résiduelle obtenue est dissoute dans 60 $cm^3$ de méthanol ; on agite cette solution pendant 20 mn à 35°C, puis on l'évapore à sec. On renouvelle cette opération jusqu'à poids constant, dans le cas présent l'opération est renouvelée 4 fois.
On obtient ainsi 3,1 g d'un solide que l'on disperse dans du diisopropyléther.
P.F. = 112-4°C (acétate d'éthyle) - Rendement = 2,48 g = 76 %
(CCM : gel de silice : AcOEt-hexane : 1-1 : 1 tache)

<u>Analyse centésimale</u> : $C_{28}H_{31}FO_5$ PM = 466,55

| | C | H | F |
|---|---|---|---|
| % calculé | 72,08 | 6,70 | 4,07 |
| % trouvé | 71,82 | 6,83 | 3,90 |

<u>I.R.</u> :
νOH : 3390 $cm^{-1}$
νCO : 1715 $cm^{-1}$
<u>R.M.N.</u> ($CDCl_3$) - 200 MHz :
1,27 (t,3H) ; 1,20-1,58 (m,2H) ; 1,60-2,29 (m,8H) ; 2,36-2,44 (m,2H) ; 4,17 (q,2H) ; 4,00-4,29 (m,2H) ; 5,34 (dd,1H J = 16,1 Hz et J = 6,3 Hz) ; 5,94 (dd,1H J = 16,1 Hz et 1,2 Hz) ; 6,54 (dd,1H J = 7,7 Hz et 1,5 Hz) ; 6,70-6,85 (m,2H) ; 7,00-7,20 (m,5H).
<u>H.P.L.C.</u> :
- Colonne silice 5 u sphérosyl 25 cm
- Détection UV - 254 nm
- Phase mobile : AcOEt-hexane-AcOH : 35-65-0,01
  1 pic : (11,1 mn)

**<u>EXEMPLE 2</u>** :

(±)-6E-Erythro-(dihydro-1,2-diméthyl-2,2-phényl-4-naphtyl-3)-7-dihydroxy-3,5-heptène-6-oate de méthyle (composé n° 2 - formule **1** : X = $CH_2$ ; $R_1$=$R_2$=$CH_3$ ; $R_3$=$R_4$=$R_5$=$R_5$=$R_9$=$R_{10}$=H ; $R_7$ et $R_8$ = liaison ; $R_{11}$ = $OCH_3$)

<u>Stade 1</u> :

(Diméthyl-2,2-phényl-3-propyl)-2-cyanacétate d'éthyle (Schéma VII - formule **15**).
A une suspension de 19,2 g (0,79 atome gramme) de magnésium en tournures dans 100 $cm^3$ d'éther, on ajoute de façon à maintenir un doux reflux une solution de 100 g (0,79 mole) de chlorure de benzyle dans 400 $cm^3$ d'éther.
On chauffe le mélange à reflux pendant 15 mn puis on ajoute de façon à maintenir un doux reflux, une solution de 101,1 g (0,66 mole) d'isopropylidène cyanacétate d'éthyle dans 130 $cm^3$ d'éther.
On chauffe à reflux pendant 2 heures puis on refroidit et ajoute lentement 400 $cm^3$ d'eau puis 100 $cm^3$ de $H_2SO_4$ à 20 %.
On agite le mélange pendant 30 mn puis on décante la phase organique.
On extrait la phase aqueuse à l'éther, on sèche les phases éthérées réunies sur sulfate de sodium, on filtre et on distille.
$Eb_{0,3}$ = 115-121°C - Rendement = 147 g = 90 %
<u>I.R.</u> :
νC=O : 1740 $cm^{-1}$ ; νCN : 2250 $cm^{-1}$
<u>R.M.N.</u> ($CDCl_3$) :
1-1,5(m,9H) ; 2,75 (s,2H) ; 3,25 (s,1H) ; 4,2 (q,2H) ; 6,7-7,6 (m,5H).

<u>Stade 2</u> :

Acide diméthyl-3,3-phényl-4-butanoïque (Schéma VII - formule **16**).
A 70 g (0,285 mole) du composé du stade 1, on ajoute, sans refroidir, une solution de 70 g de potasse dans 230 $cm^3$ d'éthylèneglycol.
On chauffe le mélange au reflux (140°C) pendant 3 h 15. On évapore les solvants volatils sous une pression

de 15 mm de mercure, puis on chauffe à reflux (197°C) pendant 6 heures.
On refroidit le mélange, on y ajoute 500 $cm^3$ d'eau et on extrait à l'éther.
On acidifie la phase aqueuse par addition d'acide chlorhydrique concentré et on extrait au benzène.
On lave à l'eau la phase benzénique, on sèche sur sulfate de sodium, on filtre, on évapore à sec.
On isole ainsi 48,7 g d'une huile qui est utilisée telle quelle dans la suite de la synthèse. Rendement = 89 %.
I.R. : νC=O : 1700 $cm^{-1}$
R.M.N. ($CDCl_3$) :
1 (s,6H) ; 2,2 (s,2H) ; 2,65 (s,2H) ; 6,8-7,5 (m,5H)

Stade 3 :

Tétrahydro-1,2,3,4-diméthyl-3,3-oxo-1-naphtalène (Schéma VII - formule **12**).
On chauffe entre 70 et 80°C un mélange de 292 g d'acide polyphosphorique et de 860 $cm^3$ de xylène, et on y ajoute à cette température, 48,7 g (0,253 mole) du composé du stade 2 en solution dans 550 $cm^3$ de xylène.
On chauffe à reflux le mélange pendant 6 h 30, puis on le refroidit, on décante la phase xylénique ; on rajoute sur la phase minérale restante, 2000 $cm^3$ d'eau et on extrait au xylène.
On réunit les phases organiques, on les lave avec une solution aqueuse de soude à 10 % puis à l'eau, puis on sèche sur sulfate de sodium. On filtre, on évapore le solvant et on distille l'huile résiduelle.
$Eb_{0,2}$ = 72-90°C - Rendement = 87,5 %
I.R. :
νC=O : 1680 $cm^{-1}$
R.M.N. ($CDCl_3$) :
0,8-1,2 (m,6H) ; 2,5 (s,2H) ; 2,8 (s,2H) ; 7-7,7 (m,3H) ; 8 (dd,1H).

Stade 4 :

Tétrahydro-1,2,3,4-hydroxy-1-diméthyl-3,3-phényl-1 naphtalène (Schéma VI - formule **14**).
A 8,07 g (1,16 atome gramme) de lithium en suspension dans 200 $cm^3$ d'éther, on ajoute de façon à maintenir un doux reflux de l'éther, une solution de 86,95 g (0,55 mole) de bromobenzène dans 150 $cm^3$ d'éther.
On chauffe le mélange au reflux pendant 45 mn puis on refroidit et y ajoute entre 5 et 10°C, 38,6 g (0,19 mole) de composé du stade 3 en solution dans 150 $cm^3$ d'éther.
On agite le mélange pendant 4 heures à la température ambiante puis on y ajoute 150 $cm^3$ d'eau en maintenant la température entre 5 et 10°C.
On agite pendant 15 mn, puis on décante la phase organique, on la lave à l'eau, on sèche sur sulfate de sodium, on filtre, on évapore le solvant.
On obtient un solide jaune qu'on disperse sous hexane, essore et sèche.
P.F. = 107-110°C - Rendement = 47,4 g = 85 %

Stade 5 :

Dihydro-1,2-diméthyl-2,2-phényl-4-naphtalène (Schéma VI - formule **5**) préparé à partir du composé du stade 4 selon le procédé du stade 2 de l'exemple 1.
$Eb_{0,1}$ = 103-105°C P.F. = 82-84°C Rendement = 89 %

Analyse centésimale : $C_{18}H_{18}$ P.M. = 234,34

| | C | H |
|---|---|---|
| % calculé | 92,26 | 7,74 |
| % trouvé | 92,42 | 7,64 |

R.M.N. ($CDCl_3$) :
1,05 (s,6H) ; 2,7 (s,2H) ; 5,7 (s,1H) ; 6,75-7,4 (m,9H) .

Stade 6 :

Bromo-3-dihydro-1,2-diméthyl-2,2-phényl-4-naphtalène (Schéma III - formule **6**).
A une suspension convenablement agitée de 27,3 g (0,117 mole) du composé du stade 5 dans 120 $cm^3$ de

DMF, on ajoute avec un débit tel que la température du mélange n'excède pas 30°C, 25 g (0,14 mole) de N-bromosuccinimide en solution dans 120 $cm^3$ de DMF.
On poursuit l'agitation du mélange à température ambiante pendant 24 heures puis on laisse reposer pendant 48 heures ; on verse ensuite le mélange sur 800 $cm^3$ d'eau glacée, on extrait par 400 $cm^3$ d'éther (2 x 200 $cm^3$), on lave les extraits réunis à l'eau, puis on sèche sur sulfate de sodium ; on évapore l'éther et on chromatographie l'huile résiduelle sur gel de silice en utilisant l'hexane comme éluant. On obtient après évaporation de l'hexane, une huile qui est utilisée telle quelle dans la suite de la synthèse.
Rendement = 32,4 g = 89 %.
R.M.N. ($CDCl_3$) :
1,2 (s,6H) ; 2,95 (s,2H) ; 6,3-7,5 (m,9H).

Stade 7 :

3E-(Dihydro-1,2-diméthyl-2,2-phényl-4-naphtyl-3)-3-propène-2-al (Schéma III - formule **2**).
Sauf indication contraire les opérations qui suivent sont effectuées à la température de -50°C.
A une solution de 6,26 g (0,02 mole) du composé du stade 6 dans 100 $cm^3$ d'éther refroidie à -50° et convenablement agitée, on ajoute 13,8 $cm^3$ d'une solution 1,6 N de butyllithium dans l'hexane (soit 0,022 mole de BuLi). Ensuite on laisse remonter la température du mélange à la température ambiante, on le chauffe au reflux pendant 1 h 20 puis on le refroidit entre -50 et -60°C et y ajoute 2 g de N,N-diméthylamino-3 acroléïne en solution dans 30 $cm^3$ d'éther.
On poursuit l'agitation du mélange à -50° pendant 1 h 30 puis on laisse remonter sa température à température ambiante ; on poursuit l'agitation pendant 30 mn puis on verse le tout sur 300 $cm^3$ d'acide chlorhydrique à 10 % sous agitation. On décante la phase organique puis on la lave à l'eau ; on extrait la phase aqueuse au chlorure de méthylène, on lave l'extrait à l'eau, on réunit toute les phases organiques ; on les sèche sur sulfate de sodium ; on évapore les solvants et on cristallise l'huile résiduelle dans le pentane.
P.F = 105-108°C - Rendement = 2,6 g = 45 %
I.R. :
$\nu C{=}O$ : 1680 $cm^{-1}$
R.M.N. ($CDCl_3$) :
1,2 (s,6H) ; 2,8 (s,2H) ; 6,05 (dd : J = 16,5 et 7,5, 1H) ; 6,4-7,5 (m,10H) ; 9,5 (d : J = 7,5, 1H).

Stade 8 :

(±)-6E-(Dihydro-1,2-diméthyl-2,2-phényl-4-naphtyl-3)-7-hydroxy-5 -oxo-3-heptène-6-oate de méthyle (Schéma I - formule **4**).
Préparé à partir du composé du stade 7 selon le procédé du stade 4 de l'exemple 1.
Le composé se présente sous forme d'huile. Rendement = 87%.
R.M.N. ($CDCl_3$) :
1,1 (s,6H) ; 1,9-2,6 (m,3H) ; 2,72 (s,2H) ; 3,3 (s,2H) ; 3,65 (s,3H) ; 3,45-4,5 (m,2H) ; 4,8-5,35 (m,1H) ; 6 (d : J = 15,75, 1H) ; 6,35-7,5 (m,5H).

Stade 9 :

(+,-)-6E-Erythro-(dihydro-1,2-diméthyl-2,2-phényl-4-naphtyl-3)-7-dihydroxy-3,5-heptène-6-oate de méthyle (composé n° 2).
Préparé à partir du composé du stade 8 selon le procédé du stade 5 de l'exemple 1.
Solide de couleur jaune.
P.F. = 101-103°C - Rendement = 2,2 g = 67 %

Analyse centésimale : $C_{26}H_{30}O_4$ PM = 406,52

| | C | N |
|---|---|---|
| % calculé | 76,82 | 7,44 |
| % trouvé | 76,57 | 7,43 |

**EXEMPLE 3** :

(±)-6E-Erythro-((chloro-4-phényl)-4-diméthyl-2,2-2H-benzothiapyranne-3-yl)-7-dihydroxy-3,5-heptène-6 -oate de méthyle (composé n° 3 - formule **1** : X = S, $R_1$, $R_2$ = $CH_3$, $R_3$ = 4'Cl, $R_4$ = $R_5$ = $R_6$ = $R_9$ = $R_{10}$ = H, $R_7$ et $R_8$ = liaison, $R_{11}$ = $OCH_3$).

Stade 1 :

(Chloro-4-phényl)-4-dihydro-3,4-hydroxy-4-diméthyl-2,2,2H-benzothiapyranne - (Schéma VI - formule **14**) préparé à partir de la dihydro-2,3-diméthyl-2,2-4H- benzothiapyrannone-4 selon le procédé du stade 4 de l'exemple 2 ; utilisé brut dans la suite de la synthèse.

Stade 2 :

(Chloro-4-phényl)-4-diméthyl-2,2-2H-benzothiapyranne - (Schéma VI - formule **5**) préparé à partir du composé du stade 1 selon le procédé du stade 2 de l'exemple 1).
P.F. = 100-102°C (diisopropyléther) - Rendement = 50 %

Analyse centésimale : $C_{17}H_{15}ClS$ PM = 286,81

| | C | H | Cl | S |
|---|---|---|---|---|
| % calculé | 71,19 | 5,27 | 12,36 | 11,18 |
| % trouvé | 70,94 | 5,42 | 12,60 | 10,90 |

R.M.N. ($CDCl_3$) :
2 (s,6H) ; 5,75 (s,1H) ; 6,75-7,5 (m,8H) .

Stade 3 :

Bromo-3-(chloro-4-phényl)-4-diméthyl-2,2-2H-benzothiapyranne - (Schéma III - formule **6**) préparé à partir du composé du stade 2 selon le procédé du stade 6 de l'exemple 2 ; après évaporation de l'éther on obtient un solide qu'on disperse dans la quantité suffisante de méthanol refroidi à -20°C puis on essore et sèche le solide formé.
P.F. = 150-156°C - Rendement = 88 %
R.M.N. ($CDCl_3$) :
R.M.N. ($CDCl_3$) :
1,6 (s,6H) ; 6,2-7,8 (m,8H).

Stade 4 :

2E-((chloro-4-phényl)-4-diméthyl-2,2-2H-benzothiapyranne-3-yl)-3 -propène-2-oate d'éthyle (Schéma IV - formule **8**).
On opère sous atmosphère d'azote, dans un réacteur protégé de l'humidité ambiante.
On chauffe à reflux pendant 4 heures un mélange de 20,1 g (0,055 mole) de composé du stade 3, 27,5 $cm^3$ (0,254 mole) d'acrylate d'éthyle, 130 $cm^3$ de N,N-diméthylformamide, 130 $cm^3$ de triéthylamine, 1 g de tri-orthotolyl phosphine et 0,25 g de diacétate de palladium ; on verse le mélange sur un mélange glace-eau et on extrait avec la quantité suffisante d'éther ; on lave alternativement cet extrait éthéré à l'acide chlorhydrique et à l'eau jusqu'à pH = 7 puis on le sèche sur sulfate de sodium, on filtre, on évapore l'éther, on disperse le résidu sous hexane refroidi à -20°C puis on essore le solide formé et on le sèche.
P.F. = 97-99°C ($CH_3OH$) - Rendement = 91 %

Analyse centésimale : $C_{22}H_{21}ClO_2S$ PM = 384,91

| | C | H | Cl | S |
|---|---|---|---|---|
| % calculé | 68,65 | 5,50 | 9,21 | 8,33 |
| % trouvé | 68,58 | 5,79 | 9,45 | 8,30 |

R.M.N. ($CDCl_3$) :
1-1,75 (m,9H) ; 3,75-4,5 (9,2H) ; 5,3-5,75 (d,1H) ; 6,5-7,5 (m,9H).

Stade 5 :

2E-((chloro-4-phényl)-4-diméthyl-2,2-2H-benzothiapyranne-3-yl)-3 -propène-2-ol (Schéma IV - formule **9**).
On opère sous atmosphère d'azote dans un réacteur protégé de l'humidité ambiante.
A une solution de 18,9 g (0,049 mole) de composé du stade 4 dans 180 $cm^3$ de tétrahydrofuranne refroidie à -20°C, on ajoute 196 $cm^3$ d'une solution 1N d'hydrure de diisobutylaluminium dans le tétrahydrofuranne (soit : 0,049 x 4 mole de DIBAL) ; on agite le mélange à température ambiante pendant 1 heure, puis on ajoute au mélange 200 $cm^3$ d'eau ; cette addition est exothermique, le débit est réglé de façon à maintenir la température du mélange inférieure à 30°C ; on acidifie ensuite le mélange jusqu'à pH = 1 par addition de la quantité suffisante d'acide chlorhydrique concentré puis on l'extrait à l'éther.
On lave ensuite la phase éthérée par l'eau jusqu'à neutralité puis on la sèche sur sulfate de sodium ; après filtration puis évaporation de l'éther, on obtient une huile qu'on disperse dans la quantité suffisante d'hexane refroidie à 5°C ; on essore et sèche le solide formé.
P.F. = 105-108°C (hexane) - Rendement = 87 %

Analyse centésimale : $C_{20}H_{19}ClOS$ PM = 342,87

| | C | H | Cl | S |
|---|---|---|---|---|
| % calculé | 70,06 | 5,59 | 10,34 | 9,35 |
| % trouvé | 69,91 | 5,78 | 10,52 | 9,30 |

Stade 6 :

2E-((chloro-4-phényl)-4-diméthyl-2,2-2H-benzothiapyranne-3-yl)-3 -propène-2-al (Schéma IV - formule **2**).
On opère dans un réacteur protégé de l'humidité ambiante ; on agite à température ambiante pendant 48 h un mélange de 14,63 g (0,0427 mole) de composé du stade 5, de 22,26 g (0,0427 x 6 moles) de dioxyde de manganèse et 300 $cm^3$ de dichlorométhane ; on filtre ensuite le mélange, on évapore le dichlorométhane du filtrat, on disperse le résidu solide dans la quantité suffisante de diisopropyléther, on l'essore et le sèche.
P.F. = 132-134°C ($CH_3CO_2C_2H_5$) - Rendement = 82 %

Analyse centésimale : $C_{20}H_{17}ClOS$ PM = 340,86

| | C | H | Cl | S |
|---|---|---|---|---|
| % calculé | 70,47 | 5,03 | 10,40 | 9,41 |
| % trouvé | 70,27 | 4,98 | 10,51 | 9,85 |

R.M.N. ($CDCl_3$) :
1,5 (s,6H) ; 5,75-6,25 (9,1H) ; 6,75-7,5 (m,9H) ; 9,25-9,5 (d,1H).

Stade 7 :

(+,-)-6E-((chloro-4-phényl)-4-diméthyl-2,2-2H-benzothiapyranne-3-yl)-7-hydroxy-5-oxo-3-heptène-6-oate de méthyle (Schéma I - formule **4**).
Préparé à partir du composé du stade 6 selon le procédé du stade 4 de l'exemple 1.
P.F. = 86-88°C ($CH_3OH$) - Rendement = 94 %
I.R. :
νC=O : 1740 et 1700 $cm^{-1}$
R.M.N. ($CDCl_3$) :
1,5 (s,6H) ; 2,25-2,75 (m,3H) ; 3,3 (s,2H) ; 3,75 (s,3H) ; 4,25-4,5 (m,1H) ; 5-5,55 (dd,1H) ; 5,75-6,25 (d,1H) ; 6,5-7,5 (m,8H).

Stade 8 :

(±)-6E-Erythro-((chloro-4-phényl)-4-diméthyl-2,2-2H-benzothiapyranne-3-yl)-7-dihydroxy-3,5-heptène-6-oate de méthyle (Schéma I - formule **1**).
Préparé à partir du composé du stade 7 selon le procédé du stade 5 de l'exemple 1.
P.F. = 115-117°C (diisopropyléther) - Rendement = 58 %

Analyse centésimale : $C_{25}H_{27}ClO_4S$ PM = 458,99

| | C | H | Cl | S |
|---|---|---|---|---|
| % calculé | 65,42 | 5,93 | 7,72 | 6,98 |
| % trouvé | 65,52 | 6,02 | 7,80 | 7,09 |

I.R. :
νCO = 1720 $cm^{-1}$
νOH = 3400 $cm^{-1}$
R.M.N. ($CDCl_3$) :
1,5 (s,6H) ; 2,5 (d,2H) ; 3-3,75 (m,2H) ; 3,75 (s,3H) ; 4-4,5 (m,2H) ; 5-5,5 (dd,1H) ; 5,75-6,25 (d,1H) ; 6,5-7,5 (m,8H).

**EXEMPLE 4** :

(+,-)-Erythro-((fluoro-4-phényl)-4-spiro(2H-benzopyranne-1-2,1'-cyclopentyl)-3)-7-dihydroxy-3,5-hepta noate d'éthyle (composé 4 - formule **1** : X = 0, $R_1$-$R_2$ = $(CH_2)_4$-, $R_3$= 4'F, $R_4$= $R_5$ = $R_5$ = $R_7$ = $R_5$ = $R_9$ = $R_{10}$ = H, $R_{11}$ = $OC_2H_5$).

Stade 1 :

3E-((Fluoro-4-phényl)-4-spiro(2H-benzopyranne-1-2,1'-cyclopentyl)-3)-3 propène-2-al, diméthylacétal (Schéma V - formule **10**).
On opère sous atmosphère d'azote, dans un réacteur protégé de l'humidité.
On agite pendant 7 heures à température ambiante un mélange de 16,75 g (0,05 mole) de 3E((Fluoro-4-phényl)-4-spiro(2H-benzopyranne-1-2,1'-cyclopentyl)-3)-3-propène-2-al lui même préparé à partir de la spiro(2H-benzopyranne-1-2,1'-cyclopentanone-4) selon les procédés des stades 1,2 et 3 de l'exemple 1 et de 2,25 g de résine Amberlyst 15, dans 625 $cm^3$ d'orthoformiate de méthyle.
On ajoute à nouveau 2,25 g de résine Amberlyst et on agite le mélange à température ambiante pendant 12 heures. On renouvelle ce traitement 4 fois de suite puis on filtre, on évapore l'excès d'orthoformiate de méthyle ; on isole un solide blanc qu'on disperse sous hexane ; on essore et sèche.
P.F. = 112-114°C - Rendement = 16,3 g = 88 %
R.M.N. ($CDCl_3$) :
2,1-2,2 (m-8H) ; 3,07 (s,6H) ; 6,05 (d,1H, J = 5 Hz) ; 5,3 (dd,1H,J = 5Hz et 16 Hz) ; 6,05 (d,1H, J = 16 Hz) ; 6,3-7,2 (m,8H) .

Stade 2 :

((Fluoro-4-phényl)-4-spiro(2H-benzopyranne-1-2,1'-cyclopentyl) -3)-3-propanal diméthylacétal (Schéma V - formule **11**).
On hydrogène à pression normale et à température ambiante, 3,8 g (0,01 mole) du composé du stade 1 en solution dans 100 $cm^3$ de THF, en présence de 0,5 g de palladium dispersé sur carbone.
Lorsque le volume d'hydrogène théorique à absorber est atteint, on filtre le mélange et on évapore le THF ; on obtient une huile qui cristallise à la longue ; on disperse le solide ainsi obtenu dans l'hexane puis on l'essore et on le sèche.
P.F. = 83-85°C - Rendement = 3,2 g = 84 %
(CCM : gel de silice : AcOEt-hexane : 1-9 : 1 tache)
R.M.N. ($CDCl_3$) :
1,9-2,5 (m,12H) ; 3,2 (s,6H) ; 4,15 (t, 1H, J = 5Hz) ; 6,3-7,25 (m,8H).

Stade 3 :

((Fluoro-4-phényl)-4-spiro (2H-benzopyranne-1-2,1'-cyclopentyl)-3)-3 propanal (Schéma V - formule **2**). On agite à température ambiante pendant 96 heures, un mélange de 2 g (0,0052 mole) de composé du stade 2 et 2 g de résine Amberlyst 15 ; on filtre la résine, on évapore le filtrat à sec, on reprend le résidu par 40 $cm^3$ de chlorure de méthylène, on lave à l'eau et on sèche sur sulfate de sodium, on filtre et on évapore à sec, on obtient ainsi 0,9 g d'huile.
P.F. = 118-120°C (diisopropyléther)
I.R. :
$\nu$C=O : 1720 $cm^{-1}$
R.M.N. ($CDCl_3$) :
1-2,5 (m,10H) ; 6,2-7,2 (m,8H) ; 9,55 (s,1H)

Analyse centésimale : $C_{22}H_{21}FO_2$ PM = 336,39

| | C | H | F |
|---|---|---|---|
| % calculé | 78,55 | 6,29 | 5,65 |
| % trouvé | 78,33 | 6,04 | 5,57 |

Stade 4 :

(±)-Fluoro-4-phenyl)-4-spiro (24-benzopyranne-1-2,1'- cyclopentyl-3)-7-hydroxy-5-oxo-3-heptanoate d'éthyle (Schéma I - formule **4** - $R_7$ = $R_8$ = H).
Préparé à partir du composé du stade 3 et selon le procédé du stade 4 de l'exemple 1.
Le composé est obtenu sous forme d'huile (rendement = 51 %) ; il est utilisé tel quel dans la suite de la synthèse.

Stade 5 :

(+,-)-Erythro-((fluoro-4-phényl)-4-spiro(2H-benzopyranne-1-2,1'-cyclopentyl)-3)-7-dihydroxy-3,5-hepta noate d'éthyle (composé n° 4).
Préparé par réduction du composé du stade 4 selon le procédé du stade 5 de l'exemple 1.
Composé blanc.
P.F. = 103-105°C (diisopropyléther) - Rendement = 65 %

Analyse centésimale : $C_{28}H_{33}FO_5$ PM = 468,54

| | C | H | F |
|---|---|---|---|
| % calculé | 71,77 | 7,10 | 4,05 |
| % trouvé | 71,63 | 7,11 | 3,98 |

R.M.N. ($CDCl_3$) :
0,9-2,25 (m,17H) ; 2,35 (d,2H, J = 6 Hz) ; 3-3,5 (m,2H) ; 3,5-3,9 (m,1H) ; 3,9-4,4 (m,3H) ; 6,2-7,2 (m,8H).

**EXEMPLE 5** :

(+,-)-6E-Erythro-((fluoro-4-phényl)-4-spiro(2H-benzopyranne-1-2,1'-cyclopentyl)-3)-7-dihydroxy-3,5 heptène-6-oate de sodium (composé n° 5 - formule 1 : X = O, $R_1$-$R_2$ =-$(CH_2)_4$- ; $R_3$ = 4'F ; $R_4$ = $R_5$ = $R_6$ = $R_9$ = $R_{10}$ = H ; $R_7$ et $R_8$ = liaison; $R_{11}$ = $-O^-Na^+$).
On chauffe à 60°C de façon à obtenir une solution limpide, 2,14 g (0,0046 mole) de composé n° 4 dans 30 $cm^3$ d'éthanol, puis on refroidit cette solution à la température ambiante et on y ajoute une solution aqueuse de soude préalablement préparée par dissolution de 0,18 g (0,0046 mole) de soude en perles dans 100 $cm^3$ d'eau. On agite le mélange pendant 30 mn, on le filtre et on évapore à sec ; le résidu est séché par chauffage à 60°C sous pression réduite (0,5 mm Hg) pendant 1 heure.
P.F. = non défini - Rendement = 2,04 g = 96 %
(CCM : gel de silice : AcOEt-hexane : 1-1 + 3 % AcOH : 1 tache)

| Analyse centésimale : $C_{26}H_{26}FNaO_5$ - PM = 460,47 | | | | |
|---|---|---|---|---|
| | C | H | F | Na |
| % calculé | 67,82 | 5,69 | 4,13 | 4,99 |
| % trouvé | 67,44 | 5,71 | 3,91 | 5,11 |

**EXEMPLE 6**

(+,-)-6E-Erythro-((fluoro-4-phényl)-4-spiro(2H-benzopyranne-1-2, 1'-cyclopentyl)-3)-7-dihydroxy-3,5-heptène-6-oate de benzyle (composé n° 6 - formule **1** : X = 0, $R_1$-$R_2$ =-$(CH_2)_4$-, $R_3$ = 4'F, $R_4$ = $R_5$= $R_6$ = $R_9$ = $R_{10}$ = H, $R_7$ et $R_8$ = liaison, $R_{11}$ = $C_6H_5CH_2O$-).
A une solution de 1,55 g (0,00337 mole) de composé n° 5 dans 40 cm³ de méthyl éthyl cétone, on ajoute 0,638 g (O,OO337 mole + 10 %) de bromure de benzyle puis on chauffe le mélange au reflux pendant 3 heures.
On ajoute à nouveau 0,638 g de bromure de benzyle et on chauffe le mélange au reflux pendant 8 heures.
On filtre le mélange, on évapore le filtrat, on dissout le résidu dans la quantité suffisante d'acétate d'éthyle, on lave à l'eau cette solution, on sèche sur sulfate de sodium, on filtre et on évapore à sec.
On chromatographie l'huile résiduelle sur 15 g de gel de silice en utilisant un mélange acétate d'éthyle-hexane : 30 - 70 %.
La fraction II recueillie, fournit après évaporation des solvants, un solide qui est dispersé dans le diisopropyléther.
On essore le solide et on le sèche.
P.F. = 81 - 83°C - Rendement = 1,17 g = 66 %

| Analyse centésimale : $C_{33}H_{33}FO_5$ PM = 528,59 | | | |
|---|---|---|---|
| | C | H | F |
| % calculé | 74,98 | 6,29 | 3,59 |
| % trouvé | 74,91 | 6,23 | 3,55 |

R.M.N. ($CDCl_3$) :
1,1-2,3 (m,10H) ; 2,45 (d,2H J = 6,7 Hz) ; 2,7-3,2 (m,1H ($D_2O$)) ; 3,3-3,6 (m,1H ($D_2O$)) ; 3,8-4,5 (m,2H) ; 5,0-5,6 (s+d,3H) ; 5,9 (d,1H J = 15,7 Hz) ; 6,3-7,7 (m, 13H).

**EXEMPLE 7** :

(+,-)-6E-Erythro-((fluoro-4-phényl)-4-spiro(2H-benzopyranne-1-2, 1'-cyclopentyl)-3)-7-dihydroxy-3,5-N-méthyl-heptène-6-amide (composé n° 7 - formule **1** : X = 0, $R_1$-$R_2$ = -$(CH_2)_4$-, $R_3$ = 4'F, $R_4$ = $R_5$ = $R_6$ = $R_9$ = $R_{10}$ = H, $R_7$ et $R_6$ = liaison, $R_{11}$ = $CH_3NH$-).
On agite pendant 24 heures à température ambiante une solution de 0,67 g (0,00165 mole) de composé n° 1 dans 25 cm³ d'une solution de méthylamine à 33 % dans l'éthanol.
On évapore le solvant et on disperse le résidu dans 8 cm³ d'acétate d'éthyle, on essore et sèche.
P.F. = 150-152°C - Rendement = 0,52 g = 70 %

| Analyse centésimale : $C_{27}H_{30}FNO_4$ PM = 451,54 | | | | |
|---|---|---|---|---|
| | C | H | N | F |
| % calculé | 71,66 | 6,90 | 3,10 | 4,20 |
| % trouvé | 71,47 | 6,76 | 2,93 | 4,08 |

**EXEMPLE 8** :

(+,-)-Trans-(1E-((fluoro-4-phényl)-4-spiro(2H-benzopyranne-1-2,1'-cyclopentyl)-3)-2-éthényl)-6-hydroxy-4-tétrahydro-3,4,5,6-2H-pyrannone-2 (composé n° 8 - formule **1** : X = 0, $R_1$-$R_2$ = -$(CH_2)_4$-, $R_3$ = 4'F, $R_4$ = $R_6$ = $R_6$ = $R_{10}$ = H, $R_7$ et $R_6$= liaison, $R_9$ et $R_{11}$ = liaison).
On opère à l'abri de l'humidité.

On chauffe au reflux pendant 3 heures, un mélange de 6 g (0,0135 mole) de composé n° 4 et de 1,94 g (0,0135 mole) de chloro-2 éthyl diéthyl amine dans 120 $cm^3$ d'acétone.
On évapore l'acétone et on dissout le résidu dans la quantité suffisante d'acétate d'éthyle.
On lave à l'eau la solution jusqu'à pH 7 puis on la sèche sur sulfate de sodium.
On filtre, on évapore l'acétate d'éthyle.
On obtient un solide qu'on recristallise directement dans 120 $cm^3$ d'acétate d'éthyle.
P.F. = 188-191°C - Rendement = 4,27 g = 79 %

Analyse centésimale : $C_{26}H_{25}FO_4$ PM = 420,49

| | C | H | F |
|---|---|---|---|
| % calculé | 74,27 | 5,99 | 4,52 |
| % trouvé | 74,07 | 6,04 | 4,49 |

R.M.N. ($DMSOd_6$) - 200 MHz :
1,45-2,20 (m,10H) ; 2,34 (dd,1H J = 16 Hz et 3,45 Hz) ; 3,93-4,10 (m,1H) ; 4,85-5,0 (m,1H) ; 5,11 (d,1H J = 3,3 Hz) ; 4,42 (dd, 1H J = 16,3 Hz et 6,7 Hz) ; 6,02 (d,1H J = 16,3 Hz) ; 6,42-6,54 (m,1H) ; 6,72-6,83 (m,2H) ; 7,07-7,34 (m,5H).

**EXEMPLE 9** :

(+,-)-Trans-hydroxy-4-tétrahydro-3,4,5,6-(((fluoro-4-phényl)-4-spiro (2H-benzopyranne-1-2,1'-cyclopentyl)-3)-2-éthyl)-6-pyrannone-2 (composé n° 9 - formule **1** : X = 0, $R_1$-$R_2$ = $-(CH_2)_4-$, $R_3$ = 4'F, $R_4$ = $R_5$ = $R_6$ = $R_7$ = $R_8$ = $R_{10}$ = H, $R_9$ et $R_{11}$ = liaison).
On hydrogène à pression normale et à température ambiante, 1,72 g (0,00409 mole) de composé n° 8 dans 70 $cm^3$ de THF en présence de 0,1 g de palladium dispersé sur carbone.
Lorsque le volume théorique d'hydrogène à absorber est atteint, on filtre le mélange, on évapore le filtrat et on disperse le résidu obtenu dans le diisopropyléther.
On essore le solide ainsi obtenu et on le sèche.
P.F. = 156-157°C (diisopropyléther) - Rendement = 0,7 g = 41 %

Analyse centésimale : $C_{26}H_{27}FO_4$ PM = 422,50

| | C | H | F |
|---|---|---|---|
| % calculé | 73,91 | 6,44 | 4,50 |
| % trouvé | 73,83 | 6,32 | 4,40 |

R.M.N. ($DMSOd_6$) - 200 MHz :
1,38-2,21 (m,14H) ; 2,39 (dd,1H H = 17,2 Hz et 2,8 Hz) ; 2,57 (dd,1H J= 17,2 Hz et 4,6 Hz) ; 3,96-4,11 (m,1H) ; 4,25-4,46 (m,1H) ; 5,04 (d,1H J= 3,2 Hz) ; 6,31-6,41 (m,1H) ; 6,69-6,85 (m,2H) ; 7,02-7,34 (m,5H).

**EXEMPLE 10** :

(+,-)-Erythro-((fluoro-4-phényl)-4-spiro(2H-benzopyranne-1-2,1'-cyclopentyl)-3)-7-dihydroxy-3,5-heptanoate de sodium (composé n° 10 - formule **1** : X = 0, $R_1$-$R_2$ = $-(CH_2)_4-$,$R_3$ = 4'F, $R_4$ = $R_5$ = $R_6$ = $R_7$ = $R_8$ = $R_9$ = $R_{10}$ = H, $R_{11}$ = $-O^-Na^+$).
On agite à température ambiante un mélange de 0,22 g (0,00052 mole) de composé n° 9 et de 0,52 $cm^3$ (0,00052 mole - 5 %) de soude aqueuse 1N dans 25 $cm^3$ d'éthanol.
On évapore l'éthanol, on disperse le résidu dans l'éther, on essore et on sèche le solide ainsi obtenu.
(CCM : gel de silice : AcOEt-hexane : 1-1 + 3 % AcOH : 1 tache)
P.F. = non défini - Rendement = 0,12 g = 50 %

Analyse centésimale : $C_{26}H_{28}FNaO_5$ PM = 462,48

| | C | H | F | Na |
|---|---|---|---|---|
| % calculé | 67,52 | 6,10 | 4,11 | 4,97 |
| % trouvé | 67,30 | 5,92 | 3,90 | 5,00 |

**EXEMPLE 11** :

(+,-)-Erythro-((fluoro-4-phényl)-4-spiro(2H-benzopyranne-1-2,1'-cyclopentyl-3)-7-dihydroxy-3,5-heptanoate d'éthyle (composé n° 4).

On hydrogène à pression normale, 1,4 g (0,003 mole) de composé n° 1 en solution dans 100 $cm^3$ de THF et en présence de 0,6 g de palladium dispersé sur carbone.

Lorsque le volume théorique d'hydrogène à absorber est atteint, on filtre le mélange, on évapore le filtrat à sec et on disperse le résidu dans l' hexane, on l'essore et on le sèche.

P.F. = 103-105°C (diisopropyléther) - Rendement = 1 g = 71 %

**EXEMPLE 12** :

(+,-)-Trans-((1E-((fluoro-4-phényl)-4-spiro(2H-benzopyranne-1-2,1'-cyclopentyl)-3)-2-éthényl)-6-diméthyl-2,2-dioxane-1,3-yl-4) acétate d'éthyle (composé n° 11 - formule **1** : X = 0, $R_1$-$R_2$ = $-(CH_2)_4-$, $R_3$ = 4'F, $R_4$ = $R_5$ = $R_6$ = H, $R_7$ et $R_6$ = liaison, $R_9$-$R_{10}$ = $(CH_3)_2C-$, $R_{11}$ = $OC_2H_5$).

On agite à température ambiante pendant 48 heures, un mélange de 2 g (0,0043 mole) de composé n° 1, de 0,7 g de méthoxy-2 propène et de 8 mg d'acide paratoluènesulfoniquemonohydraté dans 25 $cm^3$ de DMF.

On ajoute 125 $cm^3$ d'éther et on lave par une solution aqueuse saturée de bicarbonate de sodium puis à l'eau.

On sèche la phase organique sur sulfate de sodium, on filtre et on évapore à sec.

On obtient une huile qu'on cristallise dans l'hexane. On essore, on sèche et on chromatographie sur 20 g de gel de silice en utilisant un mélange hexane-acétate d'éthyle : 1-1 comme solvant et éluant.

Les fractions I et II réunies sont évaporées ; on cristallise le résidu obtenu dans l'hexane ; on essore et on sèche.

P.F. = 103 - 105°C - Rendement = 0,95 g = 44 %

Analyse centésimale : $C_{31}H_{35}FO_5$ PM = 506,61

| | C | H | F |
|---|---|---|---|
| % calculé | 73,50 | 6,96 | 3,75 |
| % trouvé | 73,64 | 6,98 | 3,73 |

R.M.N. ($CDCl_3$) :

0,9-2,5 (m,16H) ; 3,9-4,5 (m,2H) ; 4,05 (q,2H) ; 5,15 (dd,1H) ; 5,95 (d,1H) ; 6,2-7,25 (m,8H).

Dans les exemples de composés de l'invention qui suivent, les abréviations utilisées ont les significations suivantes : Me = méthyl, Et = éthyl, Pr = propyl, Bz = benzyl, Pe = n.pentyl, tBu = tertiobutyl, iPr = isopropyl, Phe = phényl, Pyr = pyrrolidino.

**EXEMPLE 13** :

En utilisant les procédés appropriés des exemples 1 à 12, on a préparé les composés (tableau ci-dessous), de formula 1 dans laquelle X = O, $R_1$ et $R_2$ forment une chaîne $-(CH_2)_n-$, $R_7$ et $R_8$ forment ensemble une liaison simple, $R_9$ et $R_{10}$ désignent chacun un atome d'hydrogène :

| Composé n° | R3 | R4 | R5 | R6 | n | R11 | P.F. (°c) |
|---|---|---|---|---|---|---|---|
| 12 | H | H | H | H | 4 | EtO | 119-121 |
| 13 | 4'-Et | H | H | H | 4 | EtO | liquide |
| 14 | H | H | 6-Me | H | 4 | EtO | 96-98 |
| 15 | H | H | 7-F | H | 4 | MeO | 89-96 |
| 16 | 4'-Me | H | H | H | 4 | EtO | 120-122 |
| 17 | 3'-F | H | H | H | 4 | EtO | 97-99 |
| 18 | 4'-MeO | H | H | H | 4 | EtO | 86-88 |
| 19 | 4'-Cl | H | H | H | 4 | EtO | 128-130 |
| 20 | 2',3'-CH=CH-CH=CH | | H | H | 4 | EtO | 115-117 |
| 21 | 3'-Me | 5'-Me | H | H | 4 | EtO | liquide |
| 22 | 4'-F | H | 5-Me | 7-Me | 4 | MeO | 111-115 |
| 23 | 4'-EtO | H | H | H | 4 | MeO | liquide |
| 24 | 4'-iPrO | H | H | H | 4 | MeO | 78-80 |
| 25 | 4'-F | H | 6-MeO | H | 4 | MeO | liquide |
| 26 | 4'-CF3 | H | H | H | 4 | MeO | 141-143 |
| 27 | 4'-PeO | H | H | H | 4 | MeO | 69-71 |
| 28 | 4'-F | H | 7,8-CH=CH-CH=CH | | 4 | MeO | 109-111 |
| 29 | 4'-MeS | H | H | H | 4 | MeO | 69-71 |
| 30 | 4'-tBu | H | H | H | 4 | EtO | liquide |
| 31 | 4'-F | H | H | H | 5 | EtO | 128-131 |
| 32 | 4'-F | H | 7-iPrO | H | 4 | MeO | 90-91 |
| 33 | 4'-F | H | H | H | 4 | NH2 | 166-168 |
| 34 | 4'-F | H | H | H | 4 | iPrNH | 118-120 |
| 35 | 4'-Phe | H | H | H | 4 | Pyr | 115-117 |
| 36 | 4'-F | H | H | H | 4 | c-C6H11NH | 104-106 |

**Exemple 13 (suite)** :

| Composé n° | R3 | R4 | R5 | R6 | n | R11 | PF (°C) |
|---|---|---|---|---|---|---|---|
| 37 | 4'-F | H | H | H | 4 | BzNH | 90-92 |
| 38 | 4'-Et | H | H | H | 4 | O-Na+ | non défini |
| 39 | H | H | 6-Me | H | 4 | O-Na+ | non défini |
| 40 | 4'-iPr | H | H | H | 4 | O-Na+ | non défini |
| 41 | H | H | H | H | 4 | O-Na+ | non défini |
| 42 | 4'-Cl | H | H | H | 4 | O-Na+ | non défini |

**EXEMPLE 14** :

En utilisant les procédés appropriés des exemples 1 à 12, on a préparé les composés (tableau ci-dessous) de formule **1** dans laquelle X = O, $R_4 = R_5 = R_6 = R_9 = R_{10}$ = H et $R_7$ et $R_8$ forment une liaison :

| Composé n° | R1 | R2 | R3 | R11 | PF (°c) |
|---|---|---|---|---|---|
| 43 | H | H | H | EtO | 124-125 |
| 44 | Me | Me | 4'-F | MeO | 97-100 |
| 45 | H | iPr | 4'-F | EtO | liquide |

**EXEMPLE 15** :

En utilisant les procédés appropriés des exemples 1 à 12 on a préparé les composés (tableau ci-dessous) de formule **1** dans laquelle $R_4 = R_5 = R_{10}$ = H et $R_7$ et $R_5$ respectivement, $R_9$ et $R_{11}$ forment ensemble une liaison simple :

| Composé n° | X | R1 | R2 | R3 | R5 | PF (°C) |
|---|---|---|---|---|---|---|
| 46 | O | -(CH2)4- | | H | H | 180-181 |
| 47 | O | -(CH2)4- | | 4'-F | 6-MeO | 152-154 |
| 48 | CH2 | Me | Me | H | H | 156-158 |
| 49 | S | Me | Me | H | H | 135-137 |

**EXEMPLE 16** :

En utilisant les procédés appropriés des exemples 1 à 12, on a préparé les composés (tableau ci-dessous) de formule **1** dans laquelle X = $CH_2$ ou S, $R_4 = R_6 = R_9 = R_{10}$ = H, $R_7$ et $R_8$ forment une liaison simple :

| Composé n° | X | R1 | R2 | R3 | R5 | R11 | PF (°C) |
|---|---|---|---|---|---|---|---|
| 50 | CH2 | -(CH2)4- | | H | H | MeO | 110-112 |
| 51 | CH2 | Me | Me | 4'-F | H | MeO | 126-128 |
| 52 | CH2 | Me | Me | H | H | MeO | 101-103 |
| 53 | CH2 | -(CH2)4- | | 4'-F | H | MeO | 104-106 |
| 54 | CH2 | -(CH2)4- | | 4'-Cl | H | MeO | 96-98 |
| 55 | CH2 | Me | Me | 4'-Cl | H | MeO | 105-106 |
| 56 | CH2 | -(CH2)4- | | 3'-F | H | MeO | liquide |
| 57 | CH2 | H | H | 4'-F | H | O-Na+ | non défini |
| 58 | CH2 | iPr | H | H | H | MeO | 108-111 |
| 59 | CH2 | Me | H | H | H | MeO | liquide |
| 60 | CH2 | -(CH2)4- | | 4'-Me | 8-Me | MeO | 113-116 |
| 61 | CH2 | -(CH2)4- | | 4'-Cl | 6-Cl | MeO | 93-96 |
| 62 | CH2 | -(CH2)4- | | 4'-MeO | 8-Me | MeO | 103-106 |
| 63 | CH2 | -(CH2)4- | | 4'-F | 6-Cl | MeO | liquide |
| 64 | CH2 | Me | Me | H | 7-MeO | MeO | liquide |
| 65 | S | -(CH2)4- | | H | H | O-Na+ | non défini |
| 66 | S | -(CH2)4- | | 4'-Me | H | MeO | liquide |
| 67 | S | Me | Me | H | H | MeO | 75-77 |
| 68 | S | Me | Me | H | H | O-Na+ | non défini |

En utilisant les procédés des stades intermédiaires des exemples 1 à 4 appropriés on a préparé les composés intermédiaires rassemblés dans les exemples non limitatifs qui suivent.

**EXEMPLE 17** :

Composés intermédiaires de formule générale **4** dans laquelle X = 0, $R_1$ et $R_2$ forment une chaîne : $-(CH_2)_n-$, $R_7$ et $R_8$ forment ensemble une liaison, $R_9$ désigne un atome d'hydrogène :

| Composé intermédiaire n° | R3 | R4 | R5 | R6 | n | R11 | PF (°c) |
|---|---|---|---|---|---|---|---|
| 69 | H | H | H | H | 4 | EtO | 84-88 |
| 70 | 4'-Et | H | H | H | 4 | EtO | liquide |
| 71 | H | H | 6-Me | H | 4 | EtO | liquide |
| 72 | H | H | 7-F | H | 4 | MeO | 50-62 |
| 73 | 4'-Me | H | H | H | 4 | EtO | liquide |
| 74 | 3'-F | H | H | H | 4 | EtO | liquide |
| 75 | 4'-MeO | H | H | H | 4 | EtO | liquide |
| 76 | 4'-Cl | H | H | H | 4 | EtO | liquide |
| 77 | 2',3'-CH=CH-CH=CH | | H | H | 4 | EtO | liquide |
| 78 | 3'-Me | 5'-Me | H | H | 4 | EtO | liquide |
| 79 | 4'-F | H | 5-Me | 7-Me | 4 | MeO | liquide |
| 80 | 4'-EtO | H | H | H | 4 | MeO | liquide |
| 81 | 4'-iPrO | H | H | H | 4 | MeO | liquide |
| 82 | 4'-F | H | 6-MeO | H | 4. | MeO | liquide |
| 83 | 4'-CF3 | H | H | H | 4 | MeO | liquide |
| 84 | 4'-PeO | H | H | H | 4 | MeO | liquide |
| 85 | 4'-F | H | 7,8-CH=CH-CH=CH | | 4 | MeO | liquide |
| 86 | 4'-MeS | H | H | H | 4 | MeO | 105-107 |
| 87 | 4'-tBu | H | H | H | 4 | EtO | liquide |
| 88 | 4'-F | H | H | H | 5 | EtO | 95-97 |
| 89 | 4'-F | H | 7-iPrO | H | 4 | MeO | liquide |
| 90 | 4'-Phe | H | H | H | 4 | EtO | liquide |
| 91 | 4'-iPr | H | H | H | 4 | MeO | liquide |

**EXEMPLE 18** :

Composés intermédiaires de formule générale **4** dans laquelle X = 0, $R_4 = R_5 = R_6 = R_9 = H$ et $R_7$ et $R_8$ forment une liaison :

| Composé intermédiaire n° | R1 | R2 | R3 | R11 | PF (°c) |
|---|---|---|---|---|---|
| 92 | H | H | H | EtO | liquide |
| 93 | Me | Me | 4'-F | MeO | 72-79 |
| 94 | H | iPr | 4'-F | EtO | liquide |

**EXEMPLE 19** :

Composés intermédiaires de formule générale **4** dans laquelle, X = $CH_2$ ou S, $R_4$ = $R_6$ = $R_9$ = H, $R_7$ et $R_8$ forment une liaison et $R_{11}$ = méthoxy :

| Composé intermed.no | X | R1 | R2 | R3 | R5 | PF (°C) |
|---|---|---|---|---|---|---|
| 95 | CH2 | -(CH2)4- | | H | H | liquide |
| 96 | CH2 | Me | Me | 4'-F | H | 88-91 |
| 97 | CH2 | Me | Me | H | H | liquide |
| 98 | CH2 | -(CH2)4- | | 4'-F | H | liquide |
| 99 | CH2 | -(CH2)4- | | 4'-Cl | H | liquide |
| 100 | CH2 | Me | Me | 4'-Cl | H | liquide |
| 101 | CH2 | -(CH2)4- | | 3'-F | H | liquide |
| 102 | CH2 | H | H | 4'-F | H | liquide |
| 103 | CH2 | iPr | H | H | H | liquide |
| 104 | CH2 | Me | H | H | H | liquide |
| 105 | CH2 | -(CH2)4- | | 4'-Me | 8-Me | liquide |
| 106 | CH2 | -(CH2)4- | | 4'-Cl | 6-Cl | liquide |
| 107 | CH2 | -(CH2)4- | | 4'-MeO | 8-Me | liquide |
| 108 | CH2 | -(CH2)4- | | 4'-F | 6-Cl | liquide |
| 109 | CH2 | Me | Me | H | 7-MeO | liquide |
| 110 | S | -(CH2)4- | | H | H | liquide |
| 111 | S | -(CH2)4- | | 4'-Me | H | liquide |
| 112 | S | Me | Me | H | H | liquide |

**EXEMPLE 20** :

Composés intermédiaires de formule générale **2** dans laquelle X = 0, $R_1$ et $R_2$ forment une chaîne $-(CH_2)_n-$, et $R_7$ et $R_8$ forment une liaison :

| Composé intermédiaire n° | R3 | R4 | R5 | R6 | n | PF (°c) |
|---|---|---|---|---|---|---|
| 113 | H | H | H | H | 4 | 108-110 |
| 114 | 4'-Et | H | H | H | 4 | 164-166 |
| 115 | H | H | 6-Me | H | 4 | 126-128 |
| 116 | H | H | 7-F | H | 4 | 94-98 |
| 117 | 4'-Me | H | H | H | 4 | 146-148 |
| 118 | 3'-F | H | H | H | 4 | 113-114 |
| 119 | 4'-MeO | H | H | H | 4 | 122-124 |
| 120 | 4'-Cl | H | H | H | 4 | 144-145 |
| 121 | 2',3'-CH=CH-CH=CH- | | H | H | 4 | 138-140 |
| 122 | 3'-Me | 5'-Me | H | H | 4 | 124-126 |
| 123 | 4'-F | H | 5-Me | 7-Me | 4 | 152-156 |
| 124 | 4'-EtO | H | H | H | 4 | 134-136 |
| 125 | 4'-iPrO | H | H | H | 4 | 141-142 |
| 126 | 4'-F | H | 6-MeO | H | 4 | 126-130 |
| 127 | 4'-CF3 | H | H | H | 4 | 172-173 |
| 128 | 4'-PeO | H | H | H | 4 | 68-70 |
| 129 | 4'-F | H | 7,8-CH=CH-CH=CH- | | 4 | 198-201 |
| 130 | 4'-MeS | H | H | H | 4 | 181-183 |
| 131 | 4'-tBu | H | H | H | 4 | 129-131 |
| 132 | 4'-F | H | H | H | 5 | 125-127 |
| 133 | 4'-F | H | 7-iPrO | H | 4 | 134-136 |
| 134 | 4'-Phe | H | H | H | 4 | 147-149 |
| 135 | 4'-iPr | H | H | H | 4 | 172-174 |

**EXEMPLE 21** :

Composés intermédiaires de formule générale **2** dans laquelle X = $CH_2$, O ou S, $R_4$ = $R_6$ = H et $R_7$ et $R_6$ forment ensemble une liaison :

| Composé intermédno | X | R1 | R2 | R3 | R5 | PF (°C) |
|---|---|---|---|---|---|---|
| 136 | O | H | H | H | H | 121-123 |
| 137 | O | Me | Me | 4'-F | H | 149-153 |
| 138 | O | H | iPr | 4'-F | H | 159-160 |
| 139 | CH2 | -(CH2)4- | | H | H | 98-103 |
| 140 | CH2 | Me | Me | 4'-F | H | 92-95 |
| 141 | CH2 | Me | Me | H | H | 115-117 |
| 142 | CH2 | -(CH2)4- | | 4'-F | H | - |
| 143 | CH2 | -(CH2)4- | | 4'-Cl | H | 139-141 |
| 144 | CH2 | Me | Me | 4'-Cl | H | 131-133 |
| 145 | CH2 | -(CH2)4- | | 3'-F | H | 100-102 |
| 146 | CH2 | H | H | 4'-F | H | liquide |
| 147 | CH2 | iPr | H | H | H | 110-115 |
| 148 | CH2 | Me | H | H | H | 140-145 |
| 149 | CH2 | -(CH2)4- | | 4'-Me | 8-Me | 122-124 |
| 150 | CH2 | -(CH2)4- | | 4'-Cl | 6-Cl | 150-152 |
| 151 | CH2 | -(CH2)4- | | 4'-MeO | 8-Me | liquide |
| 152 | CH2 | -(CH2)4- | | 4'-F | 6-Cl | 118-120 |
| 153 | CH2 | Me | Me | H | 7-MeO | 112-114 |
| 154 | S | -(CH2)4- | | H | H | 100-111 |
| 155 | S | -(CH2)4- | | 4'-Me | H | 117-119 |
| 156 | S | Me | Me | H | H | 110-112 |

**EXEMPLE 22** :

Composés intermédiaires de formule générale **6** dans laquelle X = $CH_2$ ou S, $R_4$ = $R_6$ = H.

| Composé n° | X | R1 | R2 | R3 | R5 | PF (°C) |
|---|---|---|---|---|---|---|
| 157 | CH2 | -(CH2)4- | | H | H | 51-56 |
| 158 | CH2 | Me | Me | 4'-F | H | 128-131 |
| 159 | CH2 | Me | Me | H | H | liquide |
| 160 | CH2 | -(CH2)4- | | 4'-F | H | 86-89 |
| 161 | CH2 | -(CH2)4- | | 4'-Cl | H | 90-92 |
| 162 | CH2 | Me | Me | 4'-Cl | H | 153-156 |
| 163 | CH2 | -(CH2)4- | | 3'-F | H | 73-75 |
| 164 | CH2 | H | H | 4'-F | H | 89-90 |
| 165 | CH2 | iPr | H | H | H | liquide |
| 166 | CH2 | Me | H | H | H | liquide |
| 167 | CH2 | -(CH2)4- | | 4'-Me | 8-Me | 135-137 |
| 168 | CH2 | -(CH2)4- | | 4'-Cl | 6-Cl | 147-149 |
| 169 | CH2 | -(CH2)4- | | 4'-MeO | 8-Me | liquide |
| 170 | CH2 | -(CH2)4- | | 4'-F | 6-Cl | 117-119 |
| 171 | CH2 | Me | Me | H | 7-MeO | 71-73 |
| 172 | S | -(CH2)4- | | H | H | 95-97 |
| 173 | S | -(CH2)4- | | 4'-Me | H | 112-114 |
| 174 | S | Me | Me | H | H | 79-81 |

**EXEMPLE 23** :

Composés intermédiaires de formule générale **5** dans laquelle X = 0 et $R_1$ et $R_2$ forment une chaîne tétraméthylène : $-(CH_2)_4-$ :

| Composé intermédiaire n° | R3 | R4 | R5 | R6 | PF (°c) |
|---|---|---|---|---|---|
| 175 | H | H | H | H | 68-71 |
| 176 | 4'-Et | H | H | H | liquide |
| 177 | H | H | 6-Me | H | 66-67 |
| 178 | H | H | 7-F | H | liquide |
| 179 | 4'-Me | H | H | H | 56-58 |
| 180 | 3'-F | H | H | H | 76-78 |
| 181 | 4'-MeO | H | H | H | 58-60 |
| 182 | 4'-Cl | H | H | H | 81-83 |
| 183 | 2',3'-CH=CH-CH=CH | | H | H | 121-123 |
| 184 | 3'-Me | 5'Me | H | H | 74-76 |
| 185 | 4'-F | H | 5-Me | 7-Me | liquide |
| 186 | 4'-EtO | H | H | H | liquide |
| 187 | 4'-iPrO | H | H | H | 73-74 |
| 188 | 4'-F | H | 6-MeO | H | 57-61 |
| 189 | 4'-CF3 | H | H | H | 92-94 |
| 190 | 4'-PeO | H | H | H | liquide |
| 191 | 4'-F | H | 7,8-CH=CH-CH=CH | | liquide |
| 192 | 4'-MeS | H | H | H | 66-68 |
| 193 | 4'-tBu | H | H | H | 72-74 |
| 194 | 4'-F | H | 7-iPrO | H | liquide |
| 195 | 4'-Phe | H | H | H | 113-115 |
| 196 | 4'-iPr | H | H | H | liquide |

**EXEMPLE 24** :

Composés intermédiaires de formule générale **5** dans laquelle $R_4$ = $R_6$ = H :

| Composé intermed.no | X | R1 | R2 | R3 | R5 | PF (°C) |
|---|---|---|---|---|---|---|
| 197 | O | -(CH2)5- | | 4'-F | H | 111-114 |
| 198 | O | H | H | H | H | liquide |
| 199 | O | Me | Me | 4'-F | H | 48-50 |
| 200 | O | H | iPr | 4'-F | H | 97-99 |
| 201 | CH2 | -(CH2)4- | | H | H | 135-145 * |
| 202 | CH2 | Me | Me | 4'-F | H | 70-73 |
| 203 | CH2 | Me | Me | H | H | liquide |
| 204 | CH2 | -(CH2)4- | | 4'-F | H | liquide |
| 205 | CH2 | -(CH2)4- | | 4'-Cl | H | 53-55 |
| 206 | CH2 | Me | Me | 4'-Cl | H | 92-94 |
| 207 | CH2 | -(CH2)4- | | 3'-F | H | liquide |
| 208 | CH2 | H | H | 4'-F | H | liquide |
| 209 | CH2 | iPr | H | H | H | liquide |
| 210 | CH2 | Me | H | H | H | liquide |
| 211 | CH2 | -(CH2)4- | | 4'-Me | 8-Me | liquide |
| 212 | CH2 | -(CH2)4- | | 4'-Cl | 6-Cl | 127-129 |
| 213 | CH2 | -(CH2)4- | | 4'-MeO | 8-Me | liquide |
| 214 | CH2 | -(CH2)4- | | 4'-F | 6-Cl | 109-111 |
| 215 | CH2 | Me | Me | H | 7-MeO | liquide |
| 216 | S | -(CH2)4- | | H | H | liquide |
| 217 | S | -(CH2)4- | | 4'-Me | H | liquide |
| 218 | S | Me | Me | H | H | liquide |

*Ebl

**EXEMPLE 25** :

Composés intermédiaires de formule générale **9** dans laquelle X = S et $R_4 = R_6 = H$ :

| Composé intremédiaire no | R1 | R2 | R3 | R5 | PF (°C) |
|---|---|---|---|---|---|
| 219 | -(CH2)4- | | H | H | liquide |
| 220 | -(CH2)4- | | 4'-Me | H | 125-126 |
| 221 | Me | Me | H | H | liquide |

**EXEMPLE 26** :

Composés intermédiaires de formule générale **8** dans laquelle X = S, $R_4 = R_6 = H$, $R = C_2H_5$ :

| Composé intremédiaire no | R1 | R2 | R3 | R5 | PF (°C) |
|---|---|---|---|---|---|
| 222 | -(CH2)4- | | H | H | 126-129 |
| 223 | -(CH2)4- | | 4'-Me | H | 106-107 |
| 224 | Me | Me | H | H | 125-127 |

DONNEES ANALYTIQUES :

| Composé n° | | C | H | F | N | Na | Cl | S |
|---|---|---|---|---|---|---|---|---|
| 12 | % Calc.<br>% Tr. | 74,98<br>74,99 | 7,19<br>7,16 | | | | | |
| 13 | % Calc.<br>% Tr. | 75,60<br>75,58 | 7,61<br>7,40 | | | | | |
| 14 | % Calc.<br>% Tr. | 75,30<br>75,04 | 7,41<br>7,18 | | | | | |
| 15 | % Calc.<br>% Tr. | 71,66<br>71,87 | 6,46<br>6,31 | 4,20<br>4,20 | | | | |
| 16 | % Calc.<br>% Tr. | 75,30<br>75,27 | 7,41<br>7,61 | | | | | |
| 17 | % Calc.<br>% Tr. | 72,08<br>72,04 | 6,70<br>6,66 | 4,07<br>3,99 | | | | |
| 18 | % Calc.<br>% Tr. | 72,78<br>73,00 | 7,10<br>6,90 | | | | | |
| 19 | % Calc.<br>% Tr. | 69,63<br>69,72 | 6,47<br>6,51 | | | | 7,34<br>7,44 | |
| 20 | % Calc.<br>% Tr. | 77,08<br>76,90 | 6,87<br>6,92 | | | | | |
| 21 | % Calc.*<br>% Tr. | 74,20<br>74,45 | 7,68<br>7,65 | | | | | |
| 22 | % Calc.<br>% Tr. | 72,48<br>72,72 | 6,92<br>6,91 | 3,95<br>3,94 | | | | |
| 23 | % Calc.<br>% Tr. | 72,78<br>72,51 | 7,16<br>7,11 | | | | | |
| 24 | % Calc.<br>% Tr. | 73,15<br>73,41 | 7,37<br>7,20 | | | | | |
| 25 | % Calc.<br>% Tr. | 69,69<br>69,81 | 6,47<br>6,49 | 3,94<br>4,17 | | | | |
| 26 | % Calc.<br>% Tr. | 66,92<br>66,96 | 5,82<br>5,94 | 11,34<br>11,48 | | | | |

* Calculé avec 1/2 molécule d'eau.

| Composé n° | | C | H | F | N | Na | Cl | S |
|---|---|---|---|---|---|---|---|---|
| 27 | % Calc.<br>% Tr. | 73,82<br>74,10 | 7,74<br>7,73 | | | | | |
| 28 | % Calc.<br>% Tr. | 74,09<br>73,95 | 6,22<br>6,35 | 3,78<br>3,78 | | | | |
| 29 | % Calc.<br>% Tr. | 69,97<br>70,04 | 6,71<br>6,77 | | | | | 6,67<br>6,79 |
| 30 | % Calc.<br>% Tr. | 76,16<br>76,21 | 7,99<br>7,94 | | | | | |
| 31 | % Calc.<br>% Tr. | 72,48<br>72,23 | 6,92<br>6,99 | 3,95<br>3,86 | | | | |
| 32 | % Calc.<br>% Tr. | 70,57<br>70,70 | 6,91<br>6,82 | 3,72<br>3,70 | | | | |
| 33 | % Calc.<br>% Tr. | 71,21<br>71,03 | 6,67<br>6,47 | 4,33<br>4,26 | 3,19<br>3,04 | | | |
| 34 | % Calc.<br>% Tr. | 72,63<br>72,37 | 7,15<br>7,04 | 3,96<br>3,87 | 2,92<br>2,79 | | | |
| 35 | % Calc.<br>% Tr. | 78,66<br>78,47 | 7,15<br>7,10 | | 2,55<br>2,49 | | | |
| 36 | % Calc.<br>% Tr. | 73,96<br>73,70 | 7,37<br>7,46 | 3,66<br>3,76 | 2,70<br>2,64 | | | |
| 37 | % Calc.<br>% Tr. | 75,12<br>74,83 | 6,49<br>6,67 | 3,60<br>3,58 | 2,65<br>2,59 | | | |
| 38 | % Calc.*<br>% Tr. | 70,13<br>70,00 | 6,73<br>6,78 | | | 4,79<br>5,05 | | |
| 40 | % Calc.*<br>% Tr. | 70,57<br>70,73 | 6,94<br>6,82 | | | 4,66<br>4,71 | | |
| 43 | % Calc.<br>% Tr. | 73,08<br>72,84 | 6,64<br>6,60 | | | | | |

* Calculé avec 1/2 molécule d'eau

| Composé n° | | C | H | F | N | Na | Cl | S |
|---|---|---|---|---|---|---|---|---|
| 44 | % Calc.<br>% Tr. | 70,41<br>70,23 | 6,38<br>6,25 | 4,45<br>4,42 | | | | |
| 45 | % Calc.<br>% Tr. | 71,35<br>70,90 | 6,87<br>6,92 | 4,18<br>4,05 | | | | |
| 46 | % Calc.<br>% Tr. | 77,59<br>77,55 | 6,51<br>6,52 | | | | | |
| 47 | % Calc.<br>% Tr. | 71,99<br>72,10 | 6,04<br>6,15 | 4,22<br>4,18 | | | | |
| 48 | % Calc.<br>% Tr. | 78,30<br>78,40 | 7,46<br>7,30 | | | | | |
| 49 | % Calc.<br>% Tr. | 73,44<br>73,75 | 6,16<br>6,05 | | | | | 8,17<br>8,18 |
| 50 | % Calc.<br>% Tr. | 77,75<br>77,86 | 7,46<br>7,30 | | | | | |
| 51 | % Calc.<br>% Tr. | 73,56<br>73,41 | 6,89<br>6,89 | 4,48<br>4,25 | | | | |
| 52 | % Calc.<br>% Tr. | 76,82<br>76,57 | 7,44<br>7,43 | | | | | |
| 53 | % Calc.<br>% Tr. | 74,64<br>74,61 | 6,93<br>7,07 | 4,22<br>4,20 | | | | |
| 54 | % Calc.<br>% Tr. | 72,01<br>72,01 | 6,69<br>6,54 | | | | 7,59<br>7,64 | |
| 55 | % Calc.*<br>% Tr. | 69,40<br>69,24 | 6,72<br>6,55 | | | | 7,88<br>7,88 | |
| 56 | % Calc.<br>% Tr. | 74,64<br>74,36 | 6,93<br>7,19 | 4,22<br>4,13 | | | | |
| 57 | % Calc.*<br>% Tr. | 66,82<br>66,99 | 5,61<br>5,58 | 4,60<br>4,45 | | | | |
| 58 | % Calc.<br>% Tr. | 77,11<br>77,11 | 7,67<br>7,58 | | | | | |
| 59 | % Calc.<br>% Tr. | 76,50<br>76,72 | 7,19<br>7,25 | | | | | |
| 60 | % Calc.<br>% Tr. | 78,23<br>78,42 | 7,88<br>7,80 | | | | | |

* Calculé avec 1/2 molécule d'eau

| Composé n° | | C | H | F | N | Na | Cl | S |
|---|---|---|---|---|---|---|---|---|
| 61 | % Calc.<br>% Tr. | 67,07<br>66,90 | 6,03<br>5,83 | | | | 14,14<br>14,04 | |
| 62 | % Calc.<br>% Tr. | 75,60<br>75,90 | 7,61<br>7,45 | | | | | |
| 63 | % Calc.<br>% Tr. | 69,34<br>69,11 | 6,23<br>6,15 | 3,92<br>4,16 | | | 7,31<br>7,16 | |
| 64 | % Calc.*<br>% Tr. | 73,29<br>73,25 | 7,44<br>7,57 | | | | | |
| 65 | % Calc.°<br>% Tr. | 71,97<br>71,76 | 6,71<br>6,89 | | | | | 7,12<br>7,05 |
| 66 | % Calc.<br>% Tr. | 72,38<br>72,42 | 6,94<br>6,66 | | | | | 6,90<br>6,92 |
| 67 | % Calc.<br>% Tr. | 70,73<br>70,38 | 6,65<br>6,77 | | | | | 7,55<br>7,33 |
| 68 | % Calc.**<br>% Tr. | 65,96<br>65,89 | 5,88<br>6,10 | | | 5,26<br>5,16 | | 7,34<br>7,19 |

* Calculé avec 1/3 molécule d'eau

° Analyse de l'ester méthylique correspondant

** Calculé avec 3/4 molécule d'eau

## Revendications

**Revendications pour les Etats contractants suivants : AT, BE, CH, DE, DK, FR, GB, IT, LI, LU, NL, SE**

1. Dérivés d'acides benzocycloalcényl dihydroxy alcanoïques de formule **1** suivante,

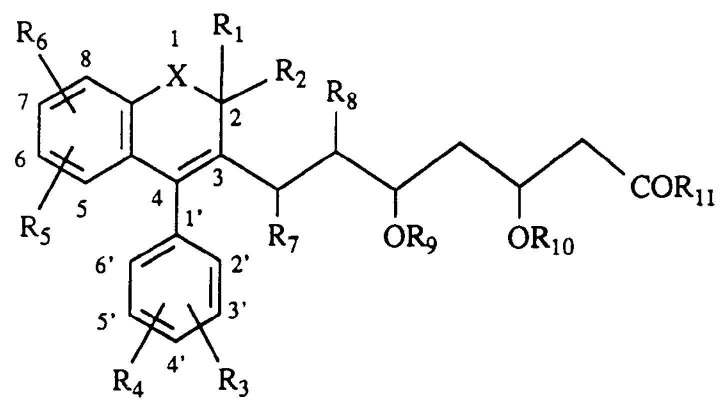

1

dans laquelle, X représente un chaînon méthylène $-CH_2-$, un atome d'oxygène ou de soufre ; $R_1$ et $R_2$, identiques ou différents, représentent des atomes d'hydrogène ou des radicaux alkyle de 1 à 3 atomes de carbone ; $R_1$ et $R_2$ peuvent aussi former ensemble une chaîne alkylène $-(CH_2)_n-$, dont le nombre

de n chaînons peut être égal à 4 ou 5 et le cas échéant substituée symétriquement par un ou deux radicaux alkyle de 1 à 3 atomes de carbone ; $R_3$ et $R_4$ qui peuvent être identiques ou différents, représentent des atomes d'hydrogène, de fluor, de chlore ou le brome, des radicaux $CF_3$, N,N-dialkylamino de 1 à 3 atomes de carbone, alkyle de 1 à 4 atomes de carbone, alkoxy de 1 à 5 atomes de carbone, phényle éventuellement substitué par au plus, deux substituants qui peuvent être identiques ou différents et représenter des radicaux alkyle en C1-3, ou des atomes de fluor ou de chlore, étant entendu que lorsque l'un des substituants $R_3$ ou $R_4$ représente un radical $CF_3$, N,N-dialkylamino, phényle ou phényle substitué, il se trouve sur les sommets 3', 4' ou 5' et l'autre substituant représente un atome d'hydrogène ; $R_5$ et $R_6$ qui peuvent être identiques ou différents, représentent des atomes d'hydrogène, de fluor, de chlore ou de brome, des radicaux : $CF_3$, alkyle en C1-3, alkoxy en C1-3 ou phényle le cas échéant substitué par au plus deux radicaux alkyle en C1-3, alkoxy en C1-3 ou atomes de fluor ou de chlore, à condition que lorsque l'un des substituants $R_6$ ou $R_6$ représente les radicaux $CF_3$, phényle ou phényle substitué, il se trouve sur les sommets 6 ou 7 et l'autre désigne un atome d'hydrogène ; les substituants $R_3$ et $R_4$ respectivement $R_5$ et $R_6$ peuvent aussi former ensemble, à condition d'être sur deux sommets contigus, les diradicaux de formules : -CH=CH-CH=CH-, $-(CH_2)_m-$ ou $-O(CH_2)_pO-$, dans lesquelles m peut être égal à 3 ou 4 et p à 1 ou 2, étant entendu que lorsque $R_3$ et $R_4$, repectivement $R_5$ et $R_6$ représentent le diradical $-O(CH_2)_pO-$, celui-ci est relié aux sommets 3' et 4' ou 4' et 5' respectivement 6 et 7 ; les substituants $R_7$ et $R_6$ représentent chacun un atome d'hydrogène ou forment ensemble avec la liaison C-C existante une double liaison de géométrie trans (E) ; les substituants $R_9$ et $R_{10}$ représentent chacun un atome d'hydrogène ou forment ensemble un reste dialkylméthylène de 1 à 3 atomes de carbone, $R_{11}$ représentant avec le groupe CO auquel il est lié une fonction acide libre, ester, amide, sel d'acide ou formant avec $R_9$ un cycle δ-lactone, ainsi que leurs isomères optiquement actifs.

2. Composé selon la revendication 1), caractérisé en ce que $R_{11}$ représente un radical hydroxyle, alkoxy de 1 à 4 atomes de carbone, benzyloxy, alkylamino ou N,N-dialkylamino de 1 à 3 atomes de carbone, imino de 4 à 6 atomes de carbone, cycloamino de 3 à 6 atomes de carbone, amino ou benzylamino ou $-O^- M^+$ où $M^+$ désigne un cation pharmaceutiquement acceptable, ou $R_{11}$ forme une simple liaison avec $R_9$.

3. Composés selon la revendication 1) ou 2) dans la formule desquels les substituants $R_1$ et $R_2$ représentent chacun un groupe méthyle ou forment ensemble une chaîne tétraméthylène, $R_3$ et $R_4$ respectivement $R_6$ et $R_6$ qui peuvent être identiques ou différents représentent des atomes d'hydrogène, de fluor, de chlore, des radicaux méthyle ou éthyle, méthoxy, méthylthio, phényle, fluoro-4-phényle, méthyl-4-phényle, méthoxy-4-phényle ou forment ensemble les diradicaux;

-CH=CH-CH=CH-, tétraméthylène : $-(CH_2)_4-$ ou méthylène dioxy : $-O(CH_2)O-$.

4. Composés selon la revendication 1) ou 2) caractérisés en ce que,

A) X désigne les atomes d'oxygène ou de soufre ou un chaînon méthylène, $R_1$ et $R_2$ désignent chacun un radical méthyle ou forment ensemble une chaîne tétraméthylène $-(CH_2)_4-$, seul l'un des radicaux $R_3$ ou $R_4$ respectivement $R_5$ ou $R_6$ désigne un atome d'hydrogène, $R_7$ et $R_6$ forment ensemble une liaison et $R_9$ et $R_{10}$ représentent chacun un atome d'hydrogène ou,

B) X, $R_1$, $R_2$, $R_7$, $R_8$, $R_9$ et $R_{10}$ ont les significations données immédiatement ci-dessus en A), l'un des substituants $R_3$ et $R_4$ désigne un atome d'hydrogène et l'autre un atome de fluor ou de chlore, et chacun des deux substituants $R_6$ et $R_6$ désigne un atome d'hydrogène, ou

C) X, $R_1$, $R_2$, $R_7$, $R_8$, $R_9$ et $R_{10}$ ont les significations qui viennent d'être définies ci-dessus en A), l'un des substituants $R_3$ et $R_4$ est un atome d'hydrogène et l'autre désigne un atome de fluor, et chacun des deux substituants $R_6$ et $R_6$ désigne un atome d'hydrogène ou,

D) X, $R_1$, $R_2$, $R_7$, $R_6$, $R_9$ et $R_{10}$ ont les significations particulières définies en A), les deux substituants $R_3$ et $R_4$ sont des atomes d'hydrogène, et seul l'un des radicaux $R_6$ et $R_6$ désigne un atome d'hydrogène ou,

E) X, $R_3$, $R_4$, $R_5$, $R_6$, $R_7$, $R_6$, $R_9$ et $R_{10}$ ont les significations ci-dessus en C) et $R_1$ et $R_2$ forment ensemble une chaîne tétraméthylène $-(CH_2)_4-$ ou,

F) X, $R_1$, $R_2$, $R_7$, $R_6$, $R_9$ et $R_{10}$ ont les significations indiquées immédiatement ci-dessus en E) et chacun des substituants $R_3$, $R_4$, $R_6$ et $R_6$ désigne un atome d'hydrogène.

5. Composés suivant la revendication 1) ou 2), nommés : (+,-)-6E-Erythro-((fluoro-4-phényl)-4-spiro(2H-benzopyranne-1-2,1'-cyclopentyl) -3)-7-dihydroxy-3,5-heptène-6-oate d'éthyle, (+,-)-6E-Erythro-((fluoro -4-phényl)-4-spiro(2H-benzopyranne-1-2,1'-cyclopentyl)-3)-7-dihydroxy-3,5-heptène-6-oate de sodium, (+,-) -6E-Erythro((dihydro-1,2-diméthyl -2,2-phényl-4-naphtyl -3)-7-dihydroxy-3,5-heptène-6-oate de

méthyle, (+,-)-6E-Erythro-((chloro-4-phényl)-4-spiro(2H-benzopyranne-1-2,1'-cyclopentyl)-3)-7-dihydroxy-3,5-heptène-6-oate d'éthyle, (+,-)-6E- Erythro-((chloro-4-phényl)-4-spiro(2H-benzopyranne-1-2,1'-cyclopentyl)-3)-7-dihydroxy-3,5-heptène-6-oate de sodium, (+,-)-6E-Erythro-dihydroxy-3,5-(phényl-4-diméthyl-2,2-2H-benzothiapyrannyl -3)-7-heptène-6-oate de méthyle, (+,-)-6E-Erythro-dihydroxy-3,5-(phényl-4-diméthyl-2,2-2H-benzothiapyrannyl-3)-7-heptène-6-oate de sodium, (+,-)-6E-Erythro-dihydroxy-3,5-(phényl-4-spiro(2H-benzothiapyranne-1-2,1'-cyclopentyl)-3)-7-heptène-6-oate de sodium, (+,-)-Erythro-((fluoro-4-phényl)-4-spiro (2H-benzopyranne-1-2,1'-cyclopentyl)-3)-7-dihydroxy- 3,5-heptanoate d'éthyle.

6. Procédés de préparation des composés de formule **1** tels que définis à la revendication 1) ou 2), caractérisés en ce qu'ils comportent au moins,

a) la réduction d'un céto ester de formule **4**, éventuellement optiquement actif

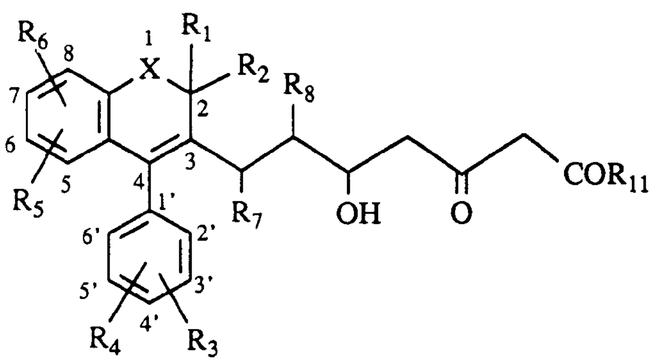

**4**

et le cas échéant,

b) La transestérification de composés de formule **1** ou l'alcoolyse de composés de formule **1** sous forme δ-lactone ou bien l'alkylation de composés de formule **1** sous forme de sels et ou,

c) L'hydrolyse de composés de formule **1** sous forme ester ou δ-lactone ou,

d) Lorsque $R_7$ et $R_8$ respectivement $R_9$ et $R_{11}$ forment une liaison simple : le traitement des composés correspondants de formule **1** sous forme de sels par une chloroamine tertiaire ou,

e) lorsque $R_7$, $R_8$ et $R_{10}$ désignent un atome d'hydrogène et $R_9$ et $R_{11}$ forment ensemble une liaison : la réduction catalytique des composés δ-lactoniques de formule **1** dans laquelle $R_7$ et $R_8$ forment une liaison ou la lactonisation des composés acides correspondants de formule **1** dans laquelle $R_7$, $R_8$ et $R_{10}$ désignent un atome d'hydrogène ou,

f) L'aminolyse des composés de formule **1** sous forme ester ou δ-lactone ou,

g) Lorsque $R_9$ et $R_{10}$ forment ensemble un reste dialkylalkylène : la cyclisation des composés de formule **1** correspondants dans laquelle $R_9$ et $R_{10}$ désignent chacun un atome d'hydrogène par un alkoxyalcène, et

h) éventuellement le dédoublement du racémate obtenu.

7. Composés intermédiaires dans la préparation des composés de formule **1** tels que définis à la revendication 1) ou 2), représentés par les formules générales,

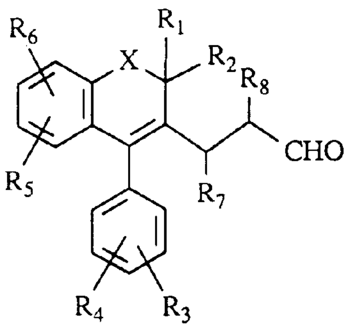

2

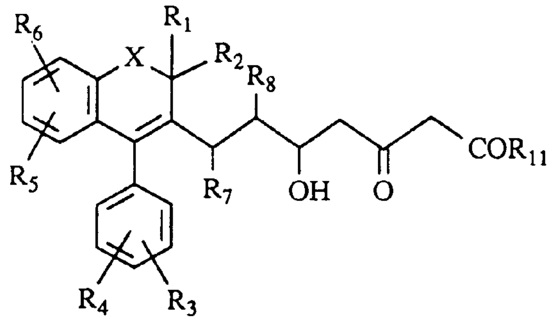

4

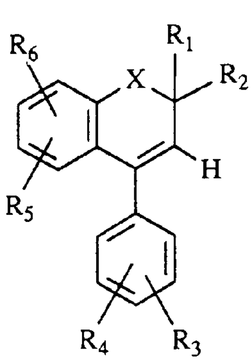

5

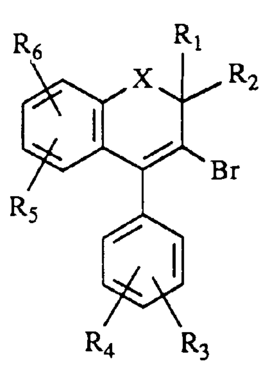

6

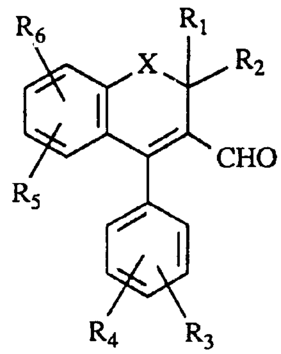

7

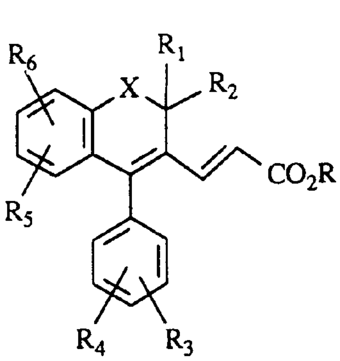

8

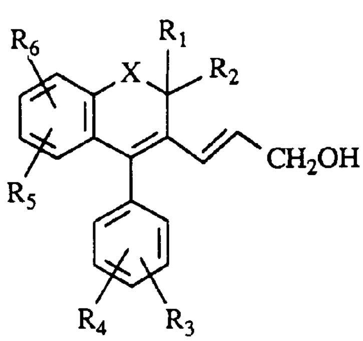

9

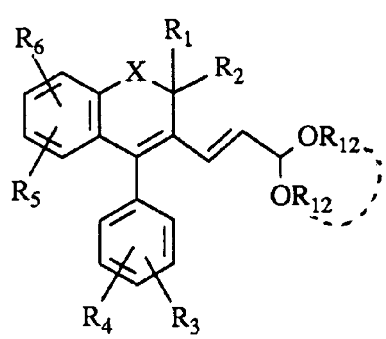

10

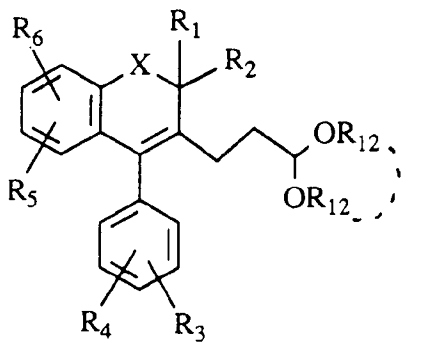

11

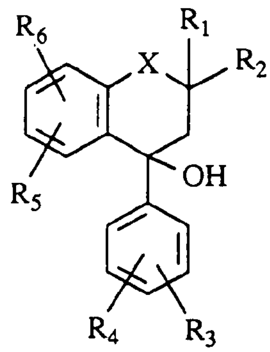

14

dans lesquelles $R_1$, $R_2$, $R_3$, $R_4$, $R_5$, $R_6$, $R_7$, $R_8$, $R_9$, $R_{10}$ et $R_{11}$ sont définis à la revendication 1 et $R_{12}$ représente un radical alkyle de 1 à 4 atomes de carbone ou $R_{12}$....$R_{12}$ représente un biradical méthylène $-(CH_2)q-$ où q représente 2 ou 3 et forme avec les atomes d'oxygènes auxquels il est rattaché un cycle à 5 ou 6 chaînons, à l'exception des produits de formule **5** où $R_1$ et $R_2$ représentent H et X représente S ou $CH_2$.

**8.** Procédé de préparation des composés intermédiaires de formule **4** tels que définis à la revendication 7, caractérisé en ce qu'il comporte la condensation aldolique de l'acétoacétate $LiCH_2COCH(Na)COR_{11}$ correspondant dans un solvant approprié, en présence d'un complexant approprié, avec le composé de formule **2** correspondant.

**9.** Procédé de préparation des composés intermédiaires de formule **2** définis à la revendication 7 où $R_7$ et $R_8$ représentent une double liaison, caractérisé en ce qu'il comporte la réaction du composé de formule **5** correspondant

A) avec la N,N'-diméthylamino-3-acroléïne dans un solvant inerte à une température comprise entre 20°C et la température de reflux pour former le composé de formule **2** correspondant sous forme trans ; ou

B) avec le N-bromosuccinimide dans un solvant approprié pour produire le composé de formule **6** 3-bromé correspondant,

a) qui réagit avec le butyllithium dans un solvant approprié puis l'éthoxy-3-acroléïne ou la N,N-diméthylamino-3-acroléïne pour former le composé de formule **2** correspondant sous forme trans ; ou

b) qui réagit avec un acrylate d'alkyle en présence d'un agent basique et d'un dérivé du palladium dans un solvant approprié pour former le composé **8** correspondant,

ou

C) le N,N-diméthylformamide pour produire l'aldéhyde de formule **7** correspondant qui réagit avec l'éthoxy- ou le méthoxy-carboxyméthylène triphényl phosphorane ou le triphényl phosphonoacétate de méthyle ou d'éthyle pour donner l'ester propenoïque 3-substitué de formule **8** sous forme trans correspondant, le composé **8** correspondant étant réduit sous forme du propénol 3-substitué de formule **9** sous forme trans correspondant qui subit une oxydation ménagée dans un solvant approprié pour produire le composé de formule **2** correspondant sous forme trans.

**10.** Procédé de préparation des composés de formule **2** définis à la revendication 7, où $R_7$ et $R_6$ représentent l'hydrogène, caractérisé en ce que l'on soumet le composé de formule **11** correspondant à une désacétalysation en catalyse acide dans un solvant approprié, le composé **11** correspondant étant préparé par hydrogénation catalytique du composé de formule **10** correspondant où $R_7$ et $R_6$ représentent une double liaison, obtenu par acétalisation de l'aldéhyde de formule **2** correspondant, ou en ce que l'on traite le composé correspondant de formule **6** avec l'alcool allylique en présence d'un agent basique approprié pour obtenir l'aldéhyde de formule **2** correspondant.

**11.** Médicament comportant comme principe actif un composé de formule 1 selon l'une des revendications 1 à 5.

**12.** Médicament selon la revendication 11) sous forme d'unité de dosage dans laquelle, chaque unité de dosage contient de 1 à 500 mg de principe actif en mélange à un excipient pharmaceutiquement acceptable.

**13.** Utilisation des composés de formule 1 seelon l'une des revendications 1 à 5 pour la préparation de médicaments utiles comme agents hypocholestérolémiant, anti-athéroscléreux et anti-thrombotique dans le traitement d'affections cardiovasculaires et de manifestations thrombotiques du diabète, de l'athérosclérose, ou des hyperlipoprotéinémies.

**14.** Utilisation des composés de formule 1 selon l'une des revendications 1 à 5 pour la préparation de médicaments utiles comme agents antifongiques.

**15.** Compositions pharmaceutiques caractérisées en ce qu'elles comportent au moins un composé de formule 1 telle que définie à la revendication 1 et un excipient pharmaceutiquement acceptable.

**Revendications pour l'Etat contractant suivant : ES**

**1.** Procédé de préparation de dérivés d'acides benzocycloalcényl dihydroxy alcanoïque de formule 1 suivante,

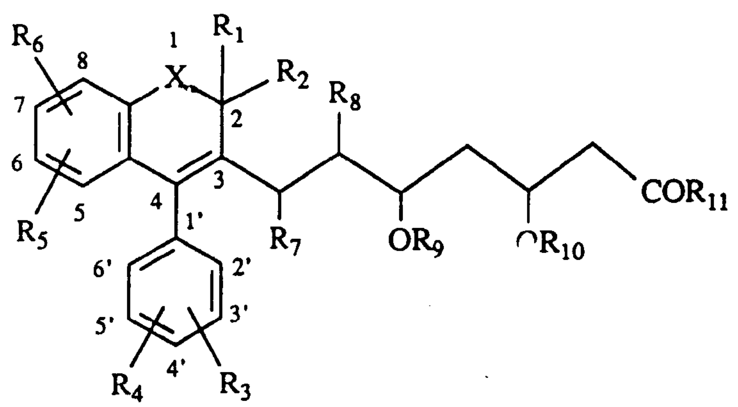

1

ainsi que leurs isomères optiquement actifs.

dans laquelle, X représente un chaînon méthylène $-CH_2-$, un atome d'oxygène ou de soufre ; $R_1$ et $R_2$, identiques ou différents, représentent des atomes d'hydrogène ou des radicaux alkyle de 1 à 3 atomes de carbone ; $R_1$ et $R_2$ peuvent aussi former ensemble une chaîne alkylène $-(CH_2)_n-$, dont le nombre de n chaînons peut être égal à 4 ou 5 et le cas échéant substituée symétriquement par un ou deux radicaux alkyle de 1 à 3 atomes de carbone ; $R_3$ et $R_4$ qui peuvent être identiques ou différents, représentent des atomes d'hydrogène, de fluor, de chlore ou le brome, des radicaux $CF_3$, N,N-dialkylamino de 1 à 3 atomes de carbone, alkyle de 1 à 4 atomes de carbone, alkoxy de 1 à 5 atomes de carbone, phényle éventuellement substitué par au plus, deux substituants qui peuvent être identiques ou différents et représenter des radicaux alkyle en C1-3, ou des atomes de fluor ou de chlore, étant entendu que lorsque l'un des substituants $R_3$ ou $R_4$ représente un radical $CF_3$, N,N-dialkylamino, phényle ou phényle substitué, il se trouve sur les sommets 3', 4' ou 5' et l'autre substituant représente un atome d'hydrogène ; $R_5$ et $R_6$ qui peuvent être identiques ou différents, représentent des atomes d'hydrogène, de fluor, de chlore ou de brome, des radicaux : $CF_3$, alkyle en C1-3, alkoxy en C1-3 ou phényle le cas échéant substitué par au plus deux radicaux alkyle en C1-3, alkoxy en C1-3 ou atomes de fluor ou de chlore, à condition que lorsque l'un des substituants $R_6$ ou $R_6$ représente les radicaux $CF_3$, phényle ou phényle substitué, il se trouve sur les sommets 6 ou 7 et l'autre désigne un atome d'hydrogène ; les substituants $R_3$ et $R_4$ respectivement $R_5$ et $R_6$ peuvent aussi former ensemble, à condition d'être sur deux sommets contigus, les diradicaux de formules : -CH=CH-CH=CH-, $-(CH_2)_m-$ ou $-O(CH_2)_pO-$, dans lesquelles m peut être égal à 3 ou 4 et p à 1 ou 2, étant entendu que lorsque $R_3$ et $R_4$, repectivement $R_5$ et $R_6$ représentent le diradical $-O(CH_2)_pO-$, celui-ci est relié aux sommets 3' et 4' ou 4' et 5' respectivement 6 et 7 ; les substituants $R_7$ et $R_6$ représentent chacun un atome d'hydrogène ou forment ensemble avec la liaison C-C existante une double liaison de géométrie trans (E) ; les substituants $R_9$ et $R_{10}$ représentent chacun un atome d'hydrogène ou forment ensemble un reste dialkylméthylène de 1 à 3 atomes de carbone, $R_{11}$ représentant avec le groupe CO auquel il est lié une fonction acide libre, ester, amide, sel d'acide ou formant avec $R_9$ un cycle δ-lactone , caractérisé en ce qu'il comporte au moins,

a) la réduction d'un céto ester de formule **4**, éventuellement optiquement actif,

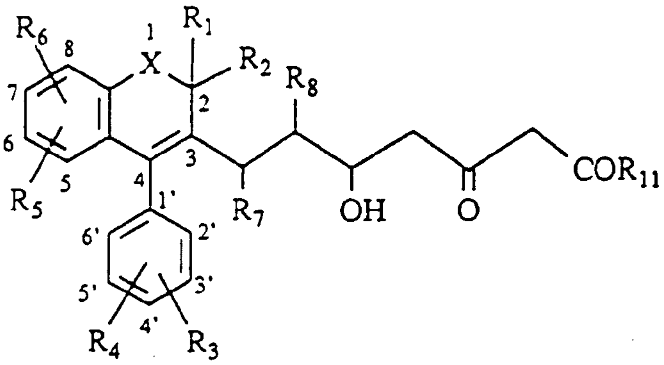

**4**

et le cas échéant,

b) La transestérification de composés de formule **1** ou l'alcoolyse de composés de formule **1** sous forme δ-lactone ou bien l'alkylation de composés de formule **1** sous forme de sels et ou,

c) L'hydrolyse de composés de formule **1** sous forme ester ou δ-lactone ou,

d) Lorsque $R_7$ et $R_8$ respectivement $R_9$ et $R_{11}$ forment une liaison simple : le traitement des composés correspondants de formule **1** sous forme de sels par une chloroamine tertiaire ou,

e) lorsque $R_7$, $R_8$ et $R_{10}$ désignent un atome d'hydrogène et $R_9$ et $R_{11}$ forment ensemble une liaison : la réduction catalytique des composés δ-lactoniques de formule **1** dans laquelle $R_7$ et $R_8$ forment une liaison ou la lactonisation des composés acides correspondants de formule 1 dans laquelle $R_7$, $R_8$ et $R_{10}$ désignent un atome d'hydrogène ou,

f) L'aminolyse des composés de formule **1** sous forme ester ou δ-lactone ou,

g) Lorsque $R_9$ et $R_{10}$ forment ensemble un reste dialkylalkylène : la cyclisation des composés de formule **1** correspondants dans laquelle $R_9$ et $R_{10}$ désignent chacun un atome d'hydrogène par un alkoxyalcène, et

h) éventuellement le dédoublement du racémate obtenu.

**2.** Procédé selon la revendication 1), caractérisé en ce que $R_{11}$ représente un radical hydroxyle, alkoxy de 1 à 4 atomes de carbone, benzyloxy, alkylamino ou N,N-dialkylamino de 1 à 3 atomes de carbone, imino de 4 à 6 atomes de carbone, cycloamino de 3 à 6 atomes de carbone, amino ou benzylamino ou $-O^- M^+$ où $M^+$ désigne un cation pharmaceutiquement acceptable, ou $R_{11}$ forme une simple liaison avec $R_9$.

**3.** Procédé selon la revendication 1) ou 2), caractérisé en ce que les substituants $R_1$ et $R_2$ représentent chacun un groupe méthyle ou forment ensemble une chaîne tétraméthylène, $R_3$ et $R_4$ respectivement $R_8$ et $R_6$ qui peuvent être identiques ou différents représentent des atomes d'hydrogène, de fluor, de chlore, des radicaux méthyle ou éthyle, méthoxy, méthylthio, phényle, fluoro-4-phényle, méthyl-4-phényle, méthoxy-4-phényle ou forment ensemble les diradicaux:

$-CH=CH-CH=CH-$, tétraméthylène : $-(CH_2)_4-$ ou méthylène dioxy : $-O(CH_2)O-$.

**4.** Procédé selon la revendication 1) ou 2) caractérisé en ce que,

A) X désigne les atomes d'oxygène ou de soufre ou un chaînon méthylène, $R_1$ et $R_2$ désignent chacun un radical méthyle ou forment ensemble une chaîne tétraméthylène $-(CH_2)_4-$, seul l'un des radicaux $R_3$ ou $R_4$ respectivement $R_8$ ou $R_6$ dégigne un atome d'hydrogène, $R_7$ et $R_8$ forment ensemble une liaison et $R_9$ et $R_{10}$ représentent chacun un atome d'hydrogène ou,

B) X, $R_1$, $R_2$, $R_7$, $R_8$, $R_9$ et $R_{10}$ ont les significations données immédiatement ci-dessus en A), l'un des substituants $R_3$ et $R_4$ désigne un atome d'hydrogène et l'autre un atome de fluor ou de chlore, et chacun des deux substituants $R_8$ et $R_6$ désigne un atome d'hydrogène, ou

C) X, $R_1$, $R_2$, $R_7$, $R_8$, $R_9$ et $R_{10}$ ont les significations qui viennent d'être définies ci-dessus en A), l'un des substituants $R_3$ et $R_4$ est un atome d'hydrogène et l'autre désigne un atome de fluor, et chacun des deux substituants $R_8$ et $R_6$ désigne un atome d'hydrogène ou,

D) X, $R_1$, $R_2$, $R_7$, $R_8$, $R_9$ et $R_{10}$ ont les significations particulières définies en A), les deux substituants $R_3$ et $R_4$ sont des atomes d'hydrogène, et seul l'un des radicaux $R_8$ et $R_8$ désigne un atome d'hydrogène ou,

E) X, $R_3$, $R_4$, $R_5$, $R_6$, $R_7$, $R_6$, $R_9$ et $R_{10}$ ont les significations ci-dessus en C) et $R_1$ et $R_2$ forment ensemble une chaîne tétraméthylène $-(CH_2)_4-$ ou,

F) X, $R_1$, $R_2$, $R_7$, $R_6$, $R_9$ et $R_{10}$ ont les significations indiquées immédiatement ci-dessus en E) et chacun des substituants $R_3$, $R_4$, $R_6$ et $R_6$ désigne un atome d'hydrogène.

**5.** Procédé suivant la revendication 1) ou 2), caractérisé en ce que les produits préparés sont (+,-)-6E-Erythro-((fluoro-4-pényl)-4-spiro(2H-benzopyranne-1-2,1'-cyclopentyl) -3)-7-dihydroxy-3,5-heptène-6-oate d'éthyle, (+,-)-6E-Erythro-((fluoro -4-phényl)-4-spiro(2H-benzopyranne-1-2,1'-cyclopentyl)-3)-7-dihydroxy-3,5-heptène-6-oate de sodium, (+,-)-6E-Erythro ((dihydro-1,2-diméthyl -2,2-phényl-4-naphtyl -3)-7-dihydroxy-3,5-heptène-6-oate de méthyle, (+,-)-6E-Erythro-((chloro-4-phényl)-4-spiro(2H-benzopyranne-1-2,1'-cyclopentyl)-3)-7-dihydroxy-3,5-heptène-6-oate d'éthyle, (+,-)-6E-Erythro-((chloro-4-phényl)-4-spiro (2H-benzopyranne-1-2,1'-cyclopentyl)-3)-7-dihydroxy-3,5-heptène-6-oate de sodium, (+,-)-6E- Erythro-dihydroxy-3,5-(phényl-4-diméthyl-2,2-2H-benzothiapyrannyl -3)-7-heptène-6-oate de méthyle, (+,-)-6E-Erythro-dihydroxy-3,5-(phényl-4-diméthyl-2,2-2H-benzothiapyrannyl-3)-7-heptène-6-oate de sodium, (+,-)-6E-Erythro-dihydroxy-3,5-(phényl-4-spiro(2H-benzothiapyranne-1-2,1'-cyclopentyl)-3)-7-heptène-6-oate de sodium, ou(+,-)-Erythro-((fluoro-4-phényl)-4-spiro(2H-benzopyranne-1-2,1'-cyclopentyl)-3)-7-dihydroxy-3,5-heptanoate d'éthyle.

**6.** Procédé de préparation de composés intermédiaires dans la préparation des composés de formule **1** tels que définis à la revendication 1) ou 2), représentés par les formules générales,

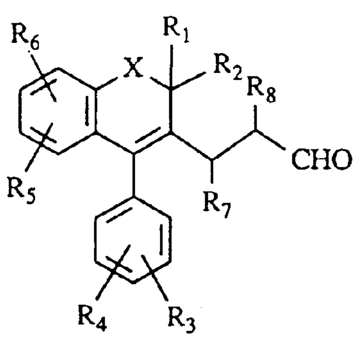

*2*

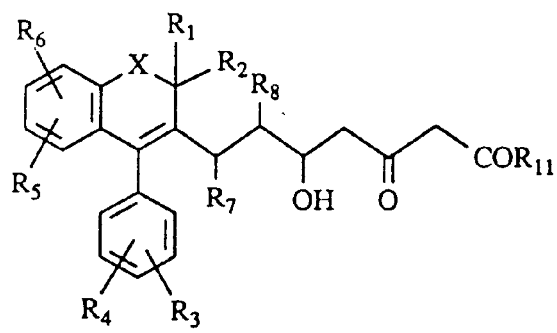

**4**

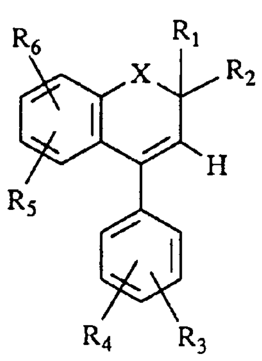

**5**

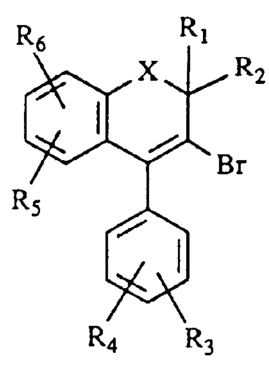

**6**

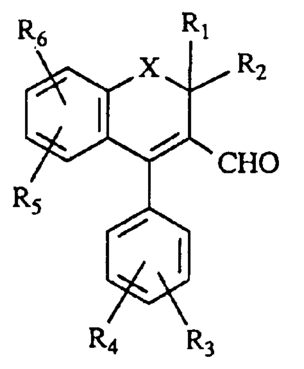

**7**

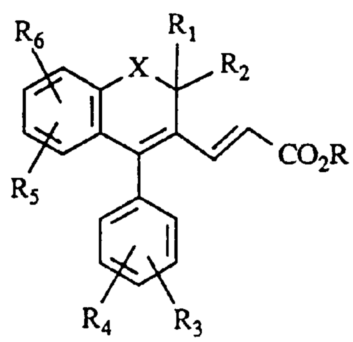

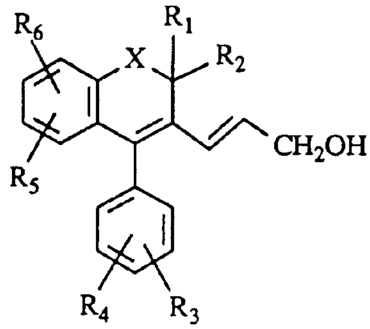

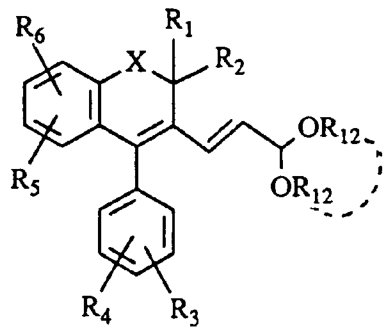

8 9 10

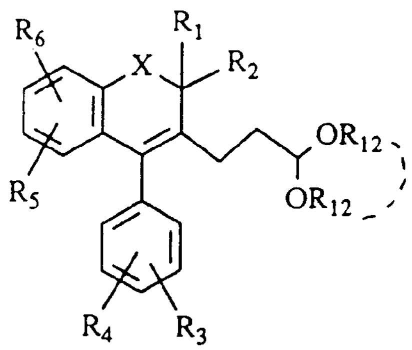

11

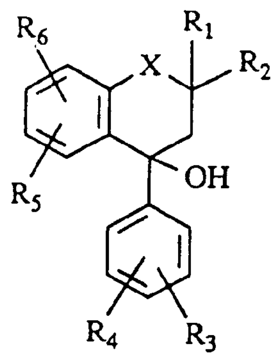

14

dans lesquelles $R_1$, $R_2$, $R_3$, $R_4$, $R_5$, $R_6$, $R_7$, $R_8$, $R_9$, $R_{10}$ et $R_{11}$ sont définis à la revendication 1 et $R_{12}$ représente un radical alkyle de 1 à 4 atomes de carbone ou $R_{12}$.... $R_{12}$ représente un biradical méthylène -$(CH_2)$q- où q représente 2 ou 3 et forme avec les atomes d'oxygènes auxquels il est rattaché un cycle à 5 ou 6 chaînons, à l'exception des produits de formule **5** où $R_1$ et $R_2$ représentent H et X représente S ou $CH_2$, caractérisé en ce qu'il comporte au moins

α) la condensation aldolique de l'acétoacétate $LiCH_2COCH(Na)COR_{11}$ correspondant dans un solvant approprié, en présence d'un complexant approprié, avec le composé de formule **2** correspondant, ou

β) pour la préparation des composés intermédiaires de formule **1** où $R_7$ et $R_6$ représentent une double liaison, la réaction du composé de formule **5** correspondant

A) avec la N,N'-diméthylamino-3-acroléïne dans un solvant inerte à une température comprise entre 20°C et la température de reflux pour former le composé de formule **2** correspondant sous forme trans ; ou

B) avec le N-bromosuccinimide dans un solvant approprié pour produire le composé de formule **6** 3-bromé correspondant,

a) qui réagit avec le butyllithium dans un solvant approprié puis l'éthoxy-3-acroléïne ou la N,N-diméthylamino-3-acroléïne pour former le composé de formule **2** correspondant sous forme trans ; ou

b) qui réagit avec un acrylate d'alkyle en présence d'un agent basique et d'un dérivé du palladium dans un solvant approprié pour former le composé **8** correspondant,

ou

C) le N,N-diméthylformamide pour produire l'aldéhyde de formule **7** correspondant qui réagit avec l'éthoxy- ou le méthoxy-carboxyméthylène triphényl phosphorane ou le triphényl phosphonoacétate de méthyle ou d'éthyle pour donner l'ester propenoïque 3-substitué de formule **8** sous forme trans correspondant, le composé **8** correspondant étant réduit sous forme du propénol 3-substitué de formule **9** sous forme trans correspondant qui subit une oxydation ménagée dans un solvant approprié pour produire le composé de formule **2** correspondant sous forme trans , ou

γ) pour la préparation des composés de formule **2** où $R_7$ et $R_8$ représentent l'hydrogène, le traitement du composé de formule **11** correspondant par une désacétalysation en catalyse acide dans un solvant approprié, le composé **11** correspondant étant préparé par hydrogénation catalytique du composé de formule **10** correspondant où $R_7$ et $R_8$ représentent une double liaison, obtenu par acétalisation de l'aldéhyde de formule **2** correspondant, ou le traitement du composé correspondant de formule **6** avec l'alcool allylique en présence d'un agent basique approprié pour obtenir l'aldéhyde de formule **2** correspondant.

**7.** Procédé de préparation de compositions pharmaceutiques caractérisé en ce qu'au moins un composé de formule **1** tel que défini à la revendication 1) est mélangé à un excipient pharmaceutiquement acceptable.

**Revendications pour l'Etat contractant suivant : GR**

**1.** Dérivés d'acides benzocycloalcényl dihydroxy alcanoïques de formule **1** suivante,

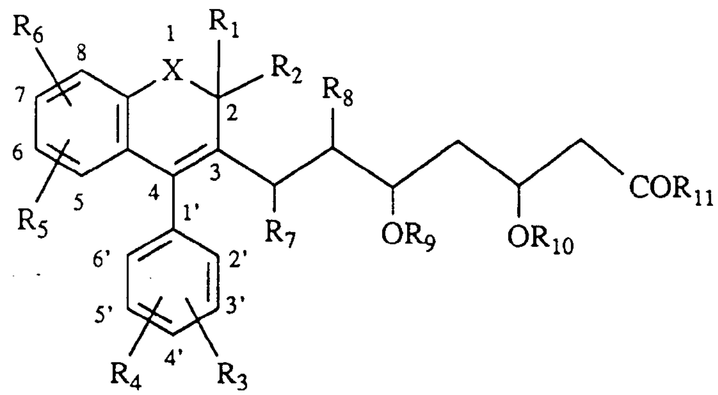

**1**

dans laquelle, X représente un chaînon méthylène $-CH_2-$, un atome d'oxygène ou de soufre ; $R_1$ et $R_2$, identiques ou différents, représentent des atomes d'hydrogène ou des radicaux alkyle de 1 à 3 atomes de carbone ; $R_1$ et $R_2$ peuvent aussi former ensemble une chaîne alkylène $-(CH_2)_n-$, dont le nombre de n chaînons peut être égal à 4 ou 5 et le cas échéant substituée symétriquement par un ou deux radicaux alkyle de 1 à 3 atomes de carbone ; $R_3$ et $R_4$ qui peuvent être identiques ou différents, représentent des atomes d'hydrogène, de fluor, de chlore ou le brome, des radicaux $CF_3$, N,N-dialkylamino de 1 à 3 atomes de carbone, alkyle de 1 à 4 atomes de carbone, alkoxy de 1 à 5 atomes de carbone, phényle éventuellement substitué par au plus, deux substituants qui peuvent être identiques ou différents et représenter des radicaux alkyle en C1-3, ou des atomes de fluor ou de chlore, étant entendu que lorsque l'un des substituants $R_3$ ou $R_4$ représente un radical $CF_3$, N,N-dialkylamino, phényle ou phényle substitué, il se trouve sur les sommets 3', 4' ou 5' et l'autre substituant représente un atome d'hydrogène ; $R_5$ et $R_8$ qui peuvent être identiques ou différents, représentent des atomes d'hydrogène, de fluor, de chlore ou de brome, des radicaux : $CF_3$, alkyle en C1-3, alkoxy en C1-3 ou phényle le cas échéant substitué par au plus deux radicaux alkyle en C1-3, alkoxy en C1-3 ou atomes de fluor ou de chlore, à condition que lorsque l'un des substituants $R_8$ ou $R_6$ représente les radicaux $CF_3$, phényle ou phényle substitué, il se trouve sur les sommets 6 ou 7 et l'autre désigne un atome d'hydrogène ; les substituants $R_3$ et $R_4$ respectivement $R_5$ et $R_8$ peuvent aussi former ensemble, à condition d'être sur deux sommets contigus, les diradicaux de formules : -CH=CH-CH=CH-, $-(CH_2)_m-$ ou $-O(CH_2)_pO-$, dans lesquelles m peut être égal à 3 ou 4 et p à 1 ou 2, étant entendu que lorsque $R_3$ et $R_4$, repectivement $R_5$ et $R_6$ représentent le diradical $-O(CH_2)_pO-$, celui-ci est relié aux sommets 3' et 4' ou 4' et 5' respectivement 6 et 7 ; les substituants $R_7$ et $R_8$ représentent chacun un atome d'hydrogène ou forment ensemble avec la liaison C-C existante une double liaison de géométrie trans (E) ; les substituants $R_9$ et $R_{10}$ représentent chacun un atome d'hydrogène ou forment ensemble un reste dialkylméthylène de 1 à 3 atomes de carbone, $R_{11}$ représentant avec le groupe CO auquel il est lié une fonction acide libre, ester, amide, sel d'acide ou formant avec $R_9$ un cycle δ-lactone ainsi que leurs isomères optiquement actifs.

**2.** Composé selon la revendication 1), caractérisé en ce que $R_{11}$ représente un radical hydroxyle, alkoxy de

1 à 4 atomes de carbone, benzyloxy, alkylamino ou N,N-dialkylamino de 1 à 3 atomes de carbone, imino de 4 à 6 atomes de carbone, cycloamino de 3 à 6 atomes de carbone, amino ou benzylamino ou $-O^- M^+$ où $M^+$ désigne un cation pharmaceutiquement acceptable, ou $R_{11}$ forme une simple liaison avec $R_9$.

3. Composés selon la revendication 1) ou 2) dans la formule desquels les substituants $R_1$ et $R_2$ représentent chacun un groupe méthyle ou forment ensemble une chaîne tétraméthylène, $R_3$ et $R_4$ respectivement $R_5$ et $R_5$ qui peuvent être identiques ou différents représentent des atomes d'hydrogène, de fluor, de chlore, des radicaux méthyle ou éthyle, méthoxy, méthylthio, phényle, fluoro-4-phényle, méthyl-4-phényle, méthoxy-4-phényle ou forment ensemble les diradicaux:
   -CH=CH-CH=CH-, tétraméthylène : $-(CH_2)_4-$ ou méthylène dioxy : $-O(CH_2)O-$.

4. Composés selon la revendication 1) ou 2) caractérisés en ce que,
   A) X désigne les atomes d'oxygène ou de soufre ou un chaînon méthylène, $R_1$ et $R_2$ désignent chacun un radical méthyle ou forment ensemble une chaîne tétraméthylène $-(CH_2)_4-$, seul l'un des radicaux $R_3$ ou $R_4$ respectivement $R_5$ ou $R_6$ désigne un atome d'hydrogène, $R_7$ et $R_5$ forment ensemble une liaison et $R_9$ et $R_{10}$ représentent chacun un atome d'hydrogène ou,
   B) X, $R_1$, $R_2$, $R_7$, $R_8$, $R_9$ et $R_{10}$ ont les significations données immédiatement ci-dessus en A), l'un des substituants $R_3$ et $R_4$ désigne un atome d'hydrogène et l'autre un atome de fluor ou de chlore, et chacun des deux substituants $R_5$ et $R_6$ désigne un atome d'hydrogène, ou
   C) X, $R_1$, $R_2$, $R_7$, $R_8$, $R_9$ et $R_{10}$ ont les significations qui viennent d'être définies ci-dessus en A), l'un des substituants $R_3$ et $R_4$ est un atome d'hydrogène et l'autre désigne un atome de fluor, et chacun des deux substituants $R_5$ et $R_6$ désigne un atome d'hydrogène ou,
   D) X, $R_1$, $R_2$, $R_7$, $R_5$, $R_9$ et $R_{10}$ ont les significations particulières définies en A), les deux substituants $R_3$ et $R_4$ sont des atomes d'hydrogène, et seul l'un des radicaux $R_5$ et $R_5$ désigne un atome d'hydrogène ou,
   E) X, $R_3$, $R_4$, $R_5$, $R_5$, $R_7$, $R_5$, $R_9$ et $R_{10}$ ont les significations ci-dessus en C) et $R_1$ et $R_2$ forment ensemble une chaîne tétraméthylène $-(CH_2)_4-$ ou,
   F) X, $R_1$, $R_2$, $R_7$, $R_5$, $R_9$ et $R_{10}$ ont les significations indiquées immédiatement ci-dessus en E) et chacun des substituants $R_3$, $R_4$, $R_5$ et $R_5$ désigne un atome d'hydrogène.

5. Composés suivant la revendication 1) ou 2), nommés : (+,-)-6E-Erythro-((fluoro-4-phényl)-4-spiro(2H-benzopyranne-1-2,1'-cyclopentyl) -3)-7-dihydroxy-3,5-heptène-6-oate d'éthyle, (+,-)-6E-Erythro-((fluoro -4-phényl)-4-spiro(2H-benzopyranne-1-2,1'-cyclopentyl)-3)-7-dihydroxy-3,5-heptène-6-oate de sodium, (+,-)-6E-Erythro((dihydro-1,2-diméthyl -2,2-phényl-4-naphtyl -3)-7-dihydroxy-3,5-heptène-6-oate de méthyle, (+,-)-6E-Erythro-((chloro-4-phényl)-4-spiro(2H-benzopyranne-1-2,1'-cyclopentyl)-3)-7-dihydroxy-3,5-heptène-6-oate d'éthyle, (+,-)-6E-Erythro-((chloro-4-phényl)-4-spiro(2H-benzopyranne-1-2,1'-cyclopentyl)-3)-7-dihydroxy-3,5-heptène-6-oate de sodium, (+,-)-6E-Erythro-dihydroxy-3,5-(phényl-4-diméthyl-2,2-2H-benzothiapyrannyl -3)-7-heptène-6-oate de méthyle, (+,-)-6E-Erythro-dihydroxy-3,5-(phényl-4-diméthyl-2,2-2H-benzothiapyrannyl-3)-7-heptène-6-oate de sodium, (+,-)-6E-Erythro-dihydroxy-3,5-(phényl-4- spiro(2H-benzothiapyranne-1-2,1'-cyclopentyl)-3)-7-heptène-6-oate de sodium, (+,-)-Erythro-((fluoro-4-phényl)-4-spiro(2H-benzopyranne-1-2,1'-cyclopentyl)-3)-7-dihydroxy-3,5-hepta noate d'éthyle.

6. Procédés de préparation des composés de formule **1** tels que définis à la revendication 1) ou 2), caractérisés en ce qu'ils comportent au moins,
   a) la réduction d'un céto ester de formule **4**, éventuellement optiquement actif,

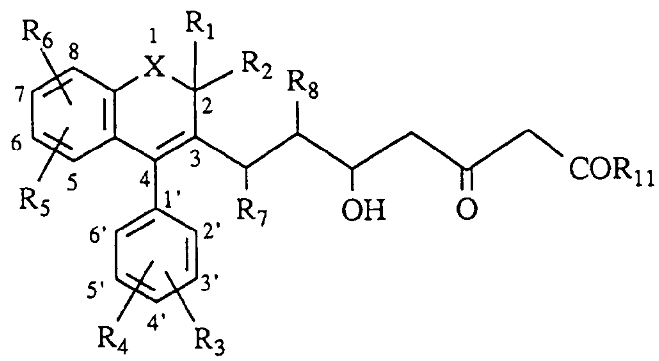

4

et le cas échéant,

b) La transestérification de composés de formule **1** ou l'alcoolyse de composés de formule 1 sous forme δ-lactone ou bien l'alkylation de composés de formule **1** sous forme de sels et ou,

c) L'hydrolyse de composés de formule **1** sous forme ester ou δ-lactone ou,

d) Lorsque $R_7$ et $R_8$ respectivement $R_9$ et $R_{11}$ forment une liaison simple : le traitement des composés correspondants de formule **1** sous forme de sels par une chloroamine tertiaire ou,

e) lorsque $R_7$, $R_8$ et $R_{10}$ désignent un atome d'hydrogène et $R_9$ et $R_{11}$ forment ensemble une liaison : la réduction catalytique des composés δ-lactoniques de formule **1** dans laquelle $R_7$ et $R_8$ forment une liaison ou la lactonisation des composés acides correspondants de formule **1** dans laquelle $R_7$, $R_8$ et $R_{10}$ désignent un atome d'hydrogène ou,

f) L'aminolyse des composés de formule **1** sous forme ester ou δ-lactone ou,

g) Lorsque $R_9$ et $R_{10}$ forment ensemble un reste dialkylalkylène : la cyclisation des composés de formule **1** correspondants dans laquelle $R_9$ et $R_{10}$ désignent chacun un atome d'hydrogène par un alkoxyalcène, et

h) éventuellement le dédoublement du racémate obtenu.

**7.** Composés intermédiaires dans la préparation des composés de formule **1** tels que définis à la revendication 1) ou 2), représentés par les formules générales,

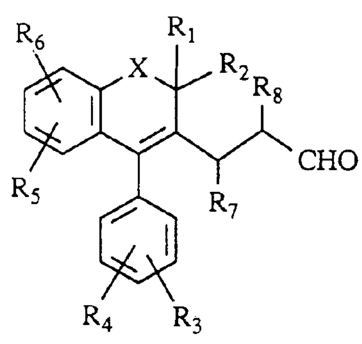

*2*

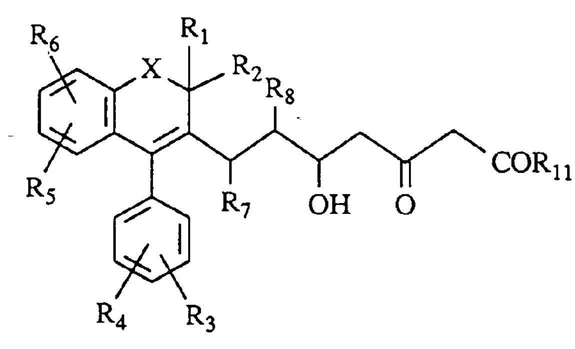

4

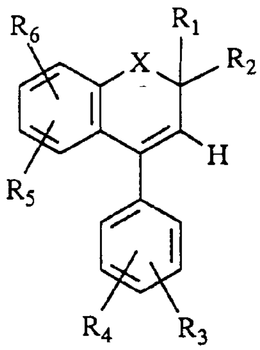

5

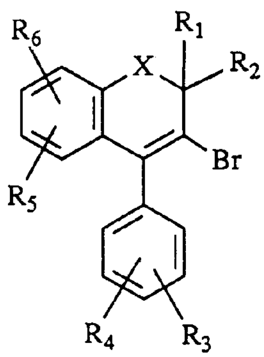

6

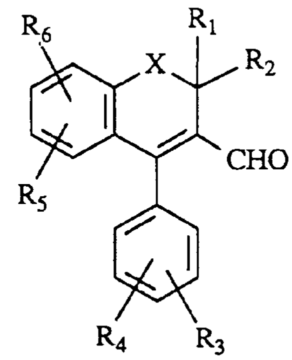

7

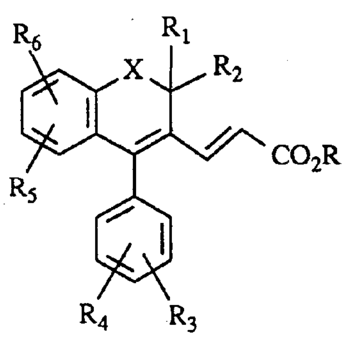

8

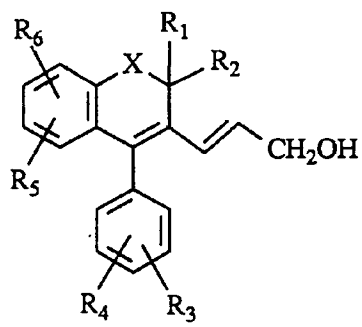

9

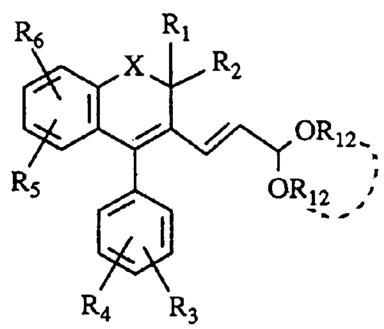

10

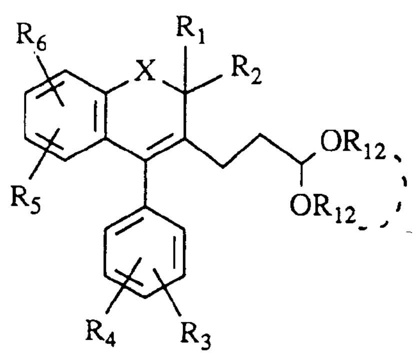

11

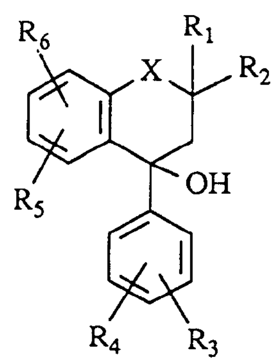

14

dans lesquelles $R_1$, $R_2$, $R_3$, $R_4$, $R_5$, $R_6$, $R_7$, $R_8$, $R_9$, $R_{10}$ et $R_{11}$ sont définis à la revendication 1 et $R_{12}$ représente un radical alkyle de 1 à 4 atomes de carbone ou $R_{12}$.... $R_{12}$ représente un biradical méthylène $-(CH_2)q-$ où q représente 2 ou 3 et forme avec les atomes d'oxygènes auxquels il est rattaché un cycle à 5 ou 6 chaînons, à l'exception des produits de formule **5** où $R_1$ et $R_2$ représentent H et X représente S ou $CH_2$.

**8.** Procédé de préparation des composés intermédiaires de formule **4** tels que définis à la revendication 7, caractérisé en ce qu'il comporte la condensation aldolique de l'acétoacétate $LiCH_2COCH(Na)COR_{11}$

correspondant dans un solvant approprié, en présence d'un complexant approprié, avec le composé de formule **2** correspondant.

9. Procédé de préparation des composés intermédiaires de formule **2** définis à la revendication 7 où $R_7$ et $R_8$ représentent une double liaison, caractérisé en ce qu'il comporte la réaction du composé de formule **5** correspondant

   A) avec la N,N'-diméthylamino-3-acroléïne dans un solvant inerte à une température comprise entre 20°C et la température de reflux pour former le composé de formule **2** correspondant sous forme trans ; ou

   B) avec le N-bromosuccinimide dans un solvant approprié pour produire le composé de formule **6** 3-bromé correspondant,

      a) qui réagit avec le butyllithium dans un solvant approprié puis l'éthoxy-3-acroléïne ou la N,N-diméthylamino-3-acroléïne pour former le composé de formule **2** correspondant sous forme trans ; ou

      b) qui réagit avec un acrylate d'alkyle en présence d'un agent basique et d'un dérivé du palladium dans un solvant approprié pour former le composé **8** correspondant,

   ou

   C) le N,N-diméthylformamide pour produire l'aldéhyde de formule **7** correspondant qui réagit avec l'éthoxy- ou le méthoxy-carboxyméthylène triphényl phosphorane ou le triphényl phosphonoacétate de méthyle ou d'éthyle pour donner l'ester propenoïque 3-substitué de formule **8** sous forme trans correspondant, le composé **8** correspondant étant réduit sous forme du propénol 3-substitué de formule **9** sous forme trans correspondant qui subit une oxydation ménagée dans un solvant approprié pour produire le composé de formule **2** correspondant sous forme trans.

10. Procédé de préparation des composés de formule **2** définis à la revendication 7, où $R_7$ et $R_8$ représentent l'hydrogène, caractérisé en ce que l'on soumet le composé de formule **11** correspondant à une désacétalysation en catalyse acide dans un solvant approprié, le composé **11** correspondant étant préparé par hydrogénation catalytique du composé de formule **10** correspondant où $R_7$ et $R_8$ représentent une double liaison, obtenu par acétalisation de l'aldéhyde de formule **2** correspondant, ou en ce que l'on traite le composé correspondant de formule **6** avec l'alcool allylique en présence d'un agent basique approprié pour obtenir l'aldéhyde de formule **2** correspondant.

11. Procédé de préparation de compositions pharmaceutiques, caractérisé en ce que l'on mélange au moins un composé de formule 1 tel que défini à la revendication 1 avec un excipient pharmaceutiquement acceptable.

## Patentansprüche

### Patentansprüche für folgende Vertragsstaaten : AT, BE, CH, DE, DK, FR, GB, IT, LI, LU, NL, SE

1. Benzocycloalkenyl-dihydroxy-alkansäure-Derivate der folgenden Formel 1

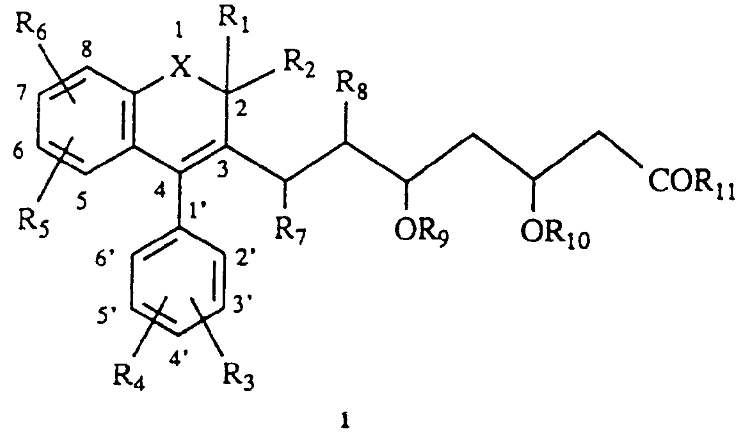

**1**

in der X eine Methylenkette $-CH_2-$, ein Sauerstoffatom oder ein Schwefelatom; $R_1$ und $R_2$, die gleichartig oder verschieden sein können, Wasserstoffatome oder Alkylgruppen mit 1 bis 3 Kohlenstoffatomen; oder $R_1$ und $R_2$ gemeinsam eine Alkylenkette $-(CH_2)_n-$, deren Anzahl der Kettenglieder n 4 oder 5 sein kann, und die gegebenenfalls durch eine oder zwei Alkylgruppen mit 1 bis 3 Kohlenstoffatomen substituiert sein kann; $R_3$ und $R_4$, die gleichartig oder verschieden sein können, Wasserstoffatome, Fluoratome, Chloratome oder Bromatome, $CF_3$-Gruppen, N,N-Dialkylaminogruppen mit 1 bis 3 Kohlenstoffatomen, Alkylgruppen mit 1 bis 4 Kohlenstoffatomen, Alkoxygruppen mit 1 bis 5 Kohlenstoffatomen, Phenylgruppen, die gegebenenfalls durch höchstens zwei Substituenten substituiert sein können, die gleichartig oder verschieden sein können, und $C_{1-3}$-Alkylgruppen, oder Fluor- oder Chloratome darstellen, mit der Maßgabe, daß, wenn einer der Substituenten $R_3$ oder $R_4$ eine $CF_3$-Gruppe, eine N,N-Dialkylaminogruppe, eine Phenylgruppe oder eine substituierte Phenylgruppe darstellt, er in der Stellung 3', 4' oder 5' steht und der andere Substituent ein Wasserstoffatom bedeutet; $R_5$ und $R_6$, die gleichartig oder verschieden sein können, Wasserstoffatome, Fluoratome, Chloratome, Bromatome, $CF_3$-Gruppen, $C_{1-3}$-Alkylgruppen, $C_{1-3}$-Alkoxygruppen oder Phenylgruppen, die gegebenenfalls durch höchstens zwei $C_{1-3}$-Alkylgruppen, $C_{1-3}$-Alkoxygruppen, Fluoratome oder Chloratome substituiert sein können, mit der Maßgabe, daß, wenn einer der Substituenten $R_5$ oder $R_6$ eine $CF_3$-Gruppe, eine Phenylgruppe oder eine substituierte Phenylgruppe darstellt, er in der Stellung 6 oder 7 steht und der andere Substituent ein Wasserstoffatom bedeutet; die Substituenten $R_3$ und $R_4$ bzw. $R_5$ und $R_6$, wenn sie an zwei benachbarten Stellungen stehen. auch gemeinsam zweiwertige Gruppen der Formeln -CH=CH-CH=CH-,$-(CH_2)_m$-oder-$O(CH_2)_pO$-, worin m 3 oder 4 und p 1 oder 2 darstellen, mit der Maßgabe bedeuten, daß, wenn $R_3$ und $R_4$ bzw. $R_6$ und $R_6$ eine zweiwertige Gruppe der Formel $-O(CH_2)_pO-$ darstellt, diese an die Stellungen 3' und 4' oder 4' und 5' bzw. 6 und 7 gebunden ist; die Substituenten $R_7$ und $R_6$ jeweils ein Wasserstoffatom oder gemeinsam mit der vorhandenen C-C-Bindung eine Doppelbindung der trans-Form (E); die Substituenten $R_9$ und $R_{10}$ jeweils ein Wasserstoffatom oder gemeinsam eine Dialkylmethylengruppe mit 1 bis 3 Kohlenstoffatomen; und $R_{11}$ mit der Gruppe CO, an die sie gebunden ist, eine freie Säuregruppe, eine Estergruppe, eine Amidgruppe, eine in Salzform vorliegende Säuregruppe oder zusammen mit $R_9$ einen δ-Lactonring bedeuten, sowie deren optisch aktive Isomeren.

2. Verbindung nach Anspruch 1, **dadurch gekennzeichnet**, daß $R_{11}$ eine Hydroxylgruppe, eine Alkoxygruppe mit 1 bis 4 Kohlenstoffatomen, eine Benzyloxygruppe, eine Alkylaminogruppe oder eine N,N-Dialkylaminogruppe mit 1 bis 3 Kohlenstoffatomen, eine Iminogruppe mit 4 bis 6 Kohlenstoffatomen, eine Cycloaminogruppe mit 3 bis 6 Kohlenstoffatomen, eine Aminogruppe oder eine Benzylaminogruppe oder -$O^- M^+$ bedeutet, worin $M^+$ ein pharmazeutisch annehmbares Kation darstellt, oder $R_{11}$ zusammen mit $R_9$ eine Einfachbindung bildet.

3. Verbindungen nach Anspruch 1 oder 2, in deren Formel die Substituenten $R_1$ und $R_2$ jeweils eine Methylgruppe oder gemeinsam eine Tetramethylenkette, $R_3$ und $R_4$ bzw. $R_5$ und $R_6$, die gleichartig oder verschieden sein können, Wasserstoffatome, Fluoratome, Chloratome, Methylgruppen, Ethylgruppen, Methoxygruppen, Methylthiogruppen, Phenylgruppen, 4-Fluor-phenylgruppen, 4-Methyl-phenylgruppen, 4-Methoxy-phenylgruppen oder gemeinsam die zweiwertigen Gruppen: -CH=CH-CH=CH-, Tetramethylen: $-(CH_2)_4-$ oder Methylendioxy: $-O(CH_2)O-$ bedeuten.

4. Verbindungen nach Anspruch 1 oder 2, **dadurch gekennzeichnet,** daß

A) X ein Sauerstoffatom oder ein Schwefelatom oder eine Methylengruppe, $R_1$ und $R_2$ jeweils eine Methylgruppe oder gemeinsam eine Tetramethylenkette $-(CH_2)_4-$, lediglich eine der Gruppen $R_3$ oder $R_4$ bzw. $R_6$ oder $R_6$ ein Wasserstoffatom, $R_7$ und $R_6$ gemeinsam eine Bindung und $R_9$ und $R_{10}$ jeweils ein Wasserstoffatom bedeuten, oder

B) X, $R_1$, $R_2$, $R_7$, $R_8$, $R_9$ und $R_{10}$ die oben unterA) angegebenen Bedeutungen besitzen und einer der Substituenten $R_3$ und $R_4$ ein Wasserstoffatom und der andere ein Fluoratom oder ein Chloratom und jeder der beiden Substituenten $R_6$ und $R_6$ ein Wasserstoffatom bedeutet, oder

C) X, $R_1$, $R_2$, $R_7$, $R_6$, $R_9$ und $R_{10}$ die oben unter A) angegebenen Bedeutungen besitzen und einer der Substituenten $R_3$ und $R_4$ ein Wasserstoffatom und der andere ein Fluoratom bedeuten und jeder der beiden Substituenten $R_6$ und $R_6$ ein Wasserstoffatom darstellt, oder

D) X, $R_1$, $R_2$, $R_7$, $R_6$, $R_9$ und $R_{10}$ die unter A) angegebenen Bedeutungen besitzen und die beiden Substituenten $R_3$ und $R_4$ Wasserstoffatome darstellen und lediglich eine der Gruppen $R_6$ und $R_6$ ein Wasserstoffatom bedeutet, oder

E) X, $R_3$, $R_4$, $R_5$, $R_6$, $R_7$, $R_8$, $R_9$ und $R_{10}$ die oben unter C) angegebenen Bedeutungen besitzen und $R_1$ und $R_2$ gemeinsam eine Tetramethylenkette $-(CH_2)_4-$ bilden, oder

F) X, $R_1$, $R_2$, $R_7$, $R_8$, $R_9$ und $R_{10}$ die oben unter E) angegebenen Bedeutungen besitzen undjeder der Substituenten $R_3$, $R_4$, $R_5$ und $R_5$ ein Wasserstoffatom bedeutet.

5. Verbindungen nach Anspruch 1 oder 2, nämlich: (+,-)-6E-Erythro-7-(4-(4-fluor-phenyl)-3-spiro(1-2H-benzopyran-2,1'-cyclopentyl))-3,5-dihydroxy-6- heptensäureethylester, (+,-)-6E-Erythro-7-(4-(4-fluor-phenyl)-3-spiro(1-2H- benzopyran-2,1'-cyclopentyl))-3,5-dihydroxy-6-heptensäure-natriumsalz, (+,-)- 6E-Erythro-7-(1,2-dihydro-2,2-dimethyl-4-phenyl-napth-3-yl)-3,5-dihydroxy-6-heptensäuremethylester, (+,-)-6E-Erythro-7-(4-(4-chlor-phenyl)-3-spiro(1-2H-benzopyran-2,1'-cyclopentyl))-3,5-dihydroxy-6-heptens äureethylester, (+,-)-6E-Erythro-7-(4-(4-chlor-phenyl)-3-spiro(1-2H-benzopyran-2,1'-cyclopentyl))-3,5-dihydroxy-6-heptensäure-natriumsalz, (+,-)-6E-Erythro-3,5-dihydroxy-7-(4-phenyl-2,2-dimethyl-2H-benzothiapyran-3-yl)-6-heptensäuremethylester, (+,-)-6E-Erythro-3,5-dihydroxy-7-(4-phenyl-2,2-dimethyl-2H-benzothiapyran-3-yl)-6-heptensäure-natriumsalz, (+,-)-6E-Erythro-3,5-dihydroxy-7- (4-phenyl-3-spiro(1-2H-benzothiapyran-2,1'-cyclopentyl))-6-heptensäure-natriumsalz, (+,-)-Erythro-7-(4-(4-fluor-phenyl)-3-spiro(1-2H-benzopyran-2,1'-cyclopentyl))-3,5-dihydroxy-heptansäureethylester.

6. Verfahren zur Herstellung der Verbindungen der Formel 1, wie sie in Anspruch 1 oder 2 definiert sind, **dadurch gekennzeichnet**, daß sie mindestens

a) die Reduktion eines gegebenenfalls optisch aktiven Ketoesters der Formel 4

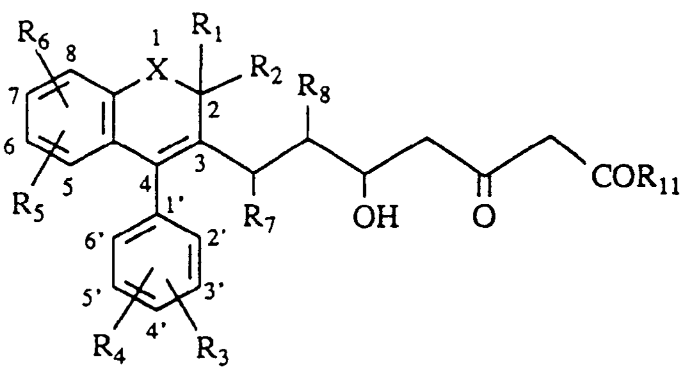

**4**

umfassen und

gegebenenfalls

b) die Umesterung der Verbindungen der Formel 1 oder die Alkoholyse der Verbindungen der Formel 1 in Form des δ-Lactons oder die Alkylierung der Verbindungen der Formel 1 in Form der Salze und/oder

c) die Hydrolyse der Verbindungen der Formel 1 in Form des Esters oder des δ-Lactons oder

d) wenn $R_7$ und $R_5$ bzw. $R_9$ und $R_{11}$ eine Einfachbindung bilden, die Behandlung der entsprechenden Verbindungen der Formel 1 in Form der Salze mit einem tertiären Chloramin oder

e) wenn $R_7$, $R_8$ und $R_{10}$ ein Wasserstoffatom und $R_9$ und $R_{11}$ gemeinsam eine Bindung bedeuten, die katalytische Reduktion der δ-Lactonverbindungen der Formel 1, in der $R_7$ und $R_8$ eine Bindung bilden, oder die Lactonisierung der entsprechenden Säureverbindungen der Formel 1, worin $R_7$, $R_8$ und $R_{10}$ ein Wasserstoffatom bedeuten, oder

f) die Aminolyse der Verbindungen der Formel 1 in Form des Esters oder des δ-Lactons oder

g) wenn $R_9$ und $R_{10}$ gemeinsam einen Dialkylalkylenrest bilden, die Cyclisierung der entsprechenden Verbindungen der Formel 1, in der $R_9$ und $R_{10}$ jeweils ein Wasserstoffatom bedeuten, mit einem Alkoxyalken, und

h) gegebenenfalls die Aufspaltung des erhaltenen Racemats.

7. Zwischenverbindungen zur Herstellung der Verbindungen der Formel 1, wie sie in Anspruch 1 oder 2 definiert sind, der folgenden allgemeinen Formeln

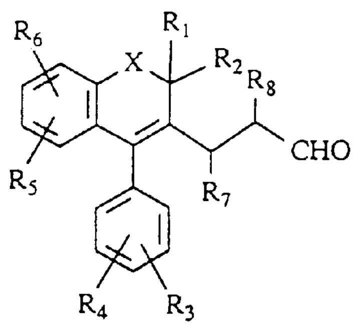

2

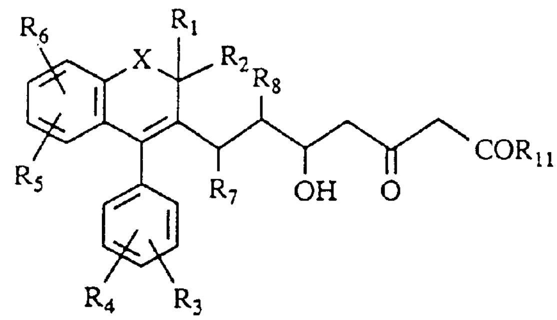

4

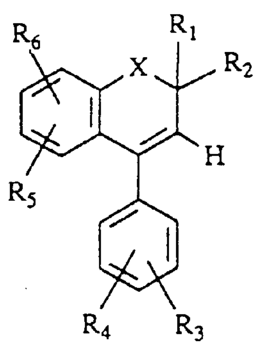

5

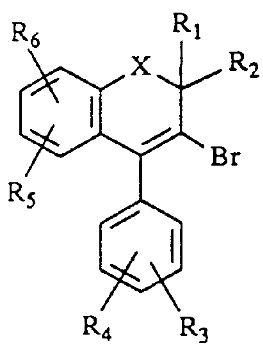

6

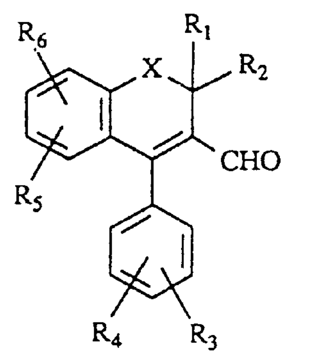

7

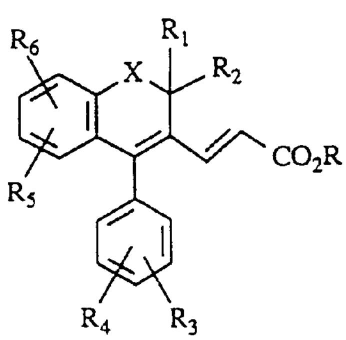

8

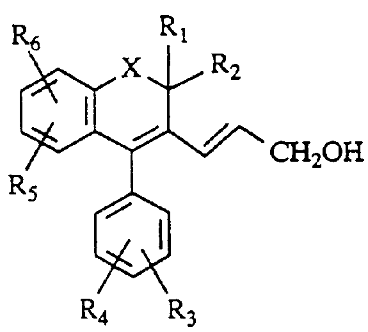

9

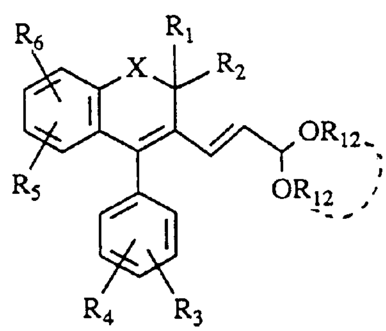

10

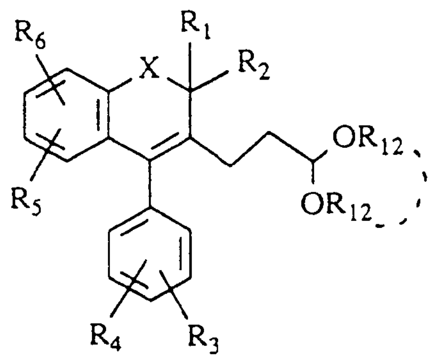

11

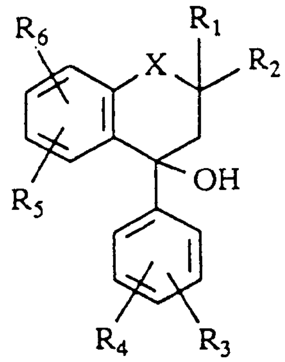

14

worin $R_1$, $R_2$, $R_3$, $R_4$, $R_5$, $R_6$, $R_7$, $R_8$, $R_9$, $R_{10}$ und $R_{11}$ die in Anspruch 1 angegebenen Bedeutungen besitzen und $R_{12}$ eine Alkylgruppe mit 1 bis 4 Kohlenstoffatomen bedeutet oder $R_{12}$...$R_{12}$ eine zweiwertige Methylengruppe $-(CH_2)_q-$, worin q 2 oder 3 darstellt, bedeuten und mit den Sauerstoffatomen, an die sie gebunden sind, einen Ring mit 5 oder 6 Kettengliedern bilden, mit Ausnahme der Produkte der Formel 5, worin $R_1$ und $R_2$ H und X S oder $CH_2$ bedeuten.

**8.** Verfahren zur Herstellung der Zwischenverbindungen der Formel 4, wie sie in Anspruch 7 definiert sind, **dadurch gekennzeichnet**, daß es die Aldolkondensation des entsprechenden Acetoacetats $LiCH_2COCH(Na)COR_{11}$ in einem geeigneten Lösungsmittel in Gegenwart eines geeigneten Komplexierungsmittels mit der entsprechenden Verbindung der Formel 2 umfaßt.

**9.** Verfahren zur Herstellung der Zwischenverbindungen der Formel 2, wie sie in Anspruch 7 definiert sind, worin $R_7$ und $R_8$ eine Doppelbindung bedeuten, **dadurch gekennzeichnet,** daß es die Reaktion der entsprechenden Verbindung der Formel 5

A) mit 3-N,N'-Dimethylamino-acrolein in einem inerten Lösungsmittel bei einer Temperatur zwischen 20°C und der Rückflußtemperatur zur Bildung der entsprechenden Verbindung der Formel 2 in der trans-Form; oder

B) mit N-Bromsuccinimid in einem geeigneten Lösungsmittel zur Bildung der entsprechenden 3-bromierten Verbindung der Formel 6,

a) welche mit Butyllithium in einem geeigneten Lösungsmittel und dann mit 3-Ethoxyacrolein oder 3-N,N-Dimethylamino-acrolein umgesetzt wird zur Bildung der entsprechenden Verbindung der Formel 2 in der trans-Form; oder

b) welche mit einem Acrylsäurealkylester in Gegenwart eines basischen Mittels und eines Palladiumderivats in einem geeigneten Lösungsmittel umgesetzt wird zur Bildung der entsprechenden Verbindung 8, oder

C) mit N,N-Dimethylformamid zur Bildung des entsprechenden Aldehyds der Formel 7, der mit Ethoxy- oder Methoxy-carboxymethylen-triphenylphosphoran oder Triphenyl-phosphonoessigsäuremethylester oder -ethylester umgesetzt wird zur Bildung des entsprechenden 3-substituierten Propensäureesters der Formel 8 in der trans-Form, wobei die entsprechende Verbindung 8 in Form des entsprechenden 3-substituierten Propenols der Formel 9 in der trans-Form reduziert wird, welches einer milden Oxidation in einem geeigneten Lösungsmittel unterworfen wird zur Bildung der entsprechenden Verbindung der Formel 2 in der trans-Form umfaßt.

**10.** Verfahren zur Herstellung der Verbindungen der Formel 2, wie sie in Anspruch 7 definiert sind, worin $R_7$ und $R_6$ Wasserstoff bedeuten, **dadurch gekennzeichnet**, daß man die entsprechende Verbindung der Formel 11 einer säurekatalysierten Desacetalisierung in einem geeigneten Lösungsmittel unterwirft, wobei die entsprechende Verbindung 11 durch katalytische Hydrierung der entsprechenden Verbindung der Formel 10, worin $R_7$ und $R_6$ eine Doppelbindung darstellen, erhalten wird, welche man durch Acetalisierung des entsprechenden Aldehyds der Formel 2 erhalten hat, oder indem man die entsprechende Verbindung der Formel 6 mit Allylalkohol in Gegenwart eines geeigneten basischen Mittels behandelt, um den entsprechenden Aldehyd der Formel 2 zu erhalten.

**11.** Arzneimittel enthaltend als Wirkstoff eine Verbindung der Formel 1 nach einem der Ansprüche 1 bis 5.

12. Arzneimittel nach Anspruch 11 in Dosiseinheitsform, worin jede Dosiseinheit 1 bis 500 mg des Wirkstoffs in Form einer Mischung mit einem pharmazeutisch annehmbaren Trägermaterial umfaßt.

13. Verwendung der Verbindungen der Formel 1 nach einem der Ansprüche 1 bis 5 zur Herstellung von Arzneimitteln, die als hypocholesterolämische Mittel, anti-artherosklerotische Mittel und anti-thrombotische Mittel bei der Behandlung von kardiovaskulären Erkrankungen und thrombotischen Zuständen des Diabetes, der Arteriosklerose und der Hyperlipoproteinämie geeignet sind.

14. Verwendung der Verbindungen der Formel 1 nach einem der Ansprüche 1 bis 5 zur Herstellung von Arzneimitteln, die als antifungistische Mittel geeignet sind.

15. Pharmazeutische Zubereitungen, **dadurch gekennzeichnet**, daß sie mindestens eine Verbindung der Formel 1, wie sie in Anspruch 1 definiert worden ist, und ein pharmazeutisch annehmbares Trägermaterial umfassen.

**Patentansprüche für folgenden Vertragsstat : ES**

1. Verfahren zur Herstellung der Benzocycloalkenyl-dihydroxy-alkansäure- Derivate der folgenden Formel 1

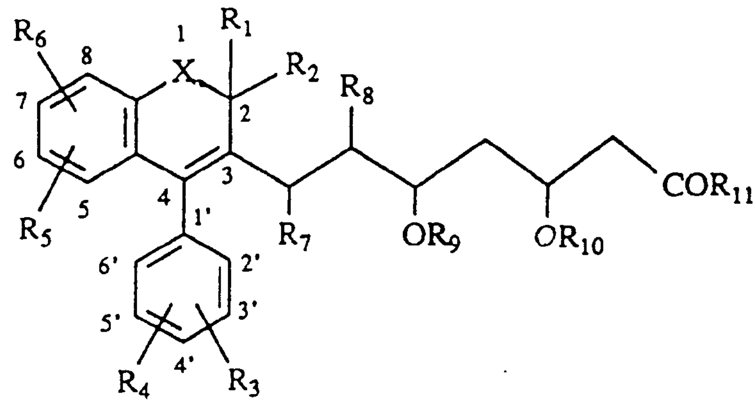

1

in der X eine Methylenkette-$CH_2$-, ein Sauerstoffatom oder ein Schwefelatom; $R_1$ und $R_2$, die gleichartig oder verschieden sein können, Wasserstoffatome oder Alkylgruppen mit 1 bis 3 Kohlenstoffatomen; oder $R_1$ und $R_2$ gemeinsam eine Alkylenkette $-(CH_2)_n-$, deren Anzahl der Kettenglieder n 4 oder 5 sein kann, und die gegebenenfalls durch eine oder zwei Alkylgruppen mit 1 bis 3 Kohlenstoffatomen substituiert sein kann; $R_3$ und $R_4$, die gleichartig oder verschieden sein können, Wasserstoffatome, Fluoratome, Chloratome oder Bromatome, $CF_3$-Gruppen, N,N-Dialkylaminogruppen mit 1 bis 3 Kohlenstoffatomen, Alkylgruppen mit 1 bis 4 Kohlenstoffatomen, Alkoxygruppen mit 1 bis 5 Kohlenstoffatomen, Phenylgruppen, die gegebenenfalls durch höchstens zwei Substituenten substituiert sein können, die gleichartig oder verschieden sein können, und $C_{1-3}$-Alkylgruppen, oder Fluor- oder Chloratome darstellen, mit der Maßgabe, daß, wenn einer der Substituenten $R_3$ oder $R_4$ eine $CF_3$-Gruppe, eine N,N-Dialkylaminogruppe, eine Phenylgruppe oder eine substituierte Phenylgruppe darstellt, er in der Stellung 3', 4' oder 5' steht und der andere Substituent ein Wasserstoffatom bedeutet; $R_5$ und $R_6$, die gleichartig oder verschieden sein können, Wasserstoffatome, Fluoratome, Chloratome, Bromatome, $CF_3$-Gruppen, $C_{1-3}$-Alkylgruppen, $C_{1-3}$-Alkoxygruppen oder Phenylgruppen, die gegebenenfalls durch höchstens zwei $C_{1-3}$-Alkylgruppen, $C_{1-3}$-Alkoxygruppen, Fluoratome oder Chloratome substituiert sein können, mit der Maßgabe, daß, wenn einer der Substituenten $R_5$ oder $R_6$ eine $CF_3$-Gruppe, eine Phenylgruppe oder eine substituierte Phenylgruppe darstellt, er in der Stellung 6 oder 7 steht und der andere Substituent ein Wasserstoffatom bedeutet; die Substituenten $R_3$ und $R_4$ bzw. $R_5$ und $R_6$, wenn sie an zwei benachbarten Stellungen stehen, auch gemeinsam zweiwertige Gruppen der Formeln -CH=CH-CH=CH-, $-(CH_2)_m-$ oder $-O(CH_2)_pO-$, worin m 3 oder 4 und p 1 oder 2 darstellen, mit der Maßgabe bedeuten, daß, wenn $R_3$ und $R_4$ bzw. $R_5$ und $R_6$ eine zweiwertige Gruppe der Formel $-O(CH_2)_pO-$ darstellt, diese an die Stellungen 3' und 4' oder 4' und 5' bzw. 6 und 7 gebunden ist; die Substituenten $R_7$ und $R_6$ jeweils ein Wasserstoffatom oder gemeinsam mit der vorhandenen C-C-Bindung eine Doppelbindung der trans-Form (E); die Substituenten $R_9$ und $R_{10}$ jeweils ein Wasserstoffatom oder gemeinsam eine Dialkylmethylengruppe mit 1 bis 3 Kohlenstoffatomen; und $R_{11}$

mit der Gruppe CO, an die sie gebunden ist, eine freie Säuregruppe, eine Estergruppe, eine Amidgruppe, eine in Salzform vorliegende Säuregruppe oder zusammen mit $R_9$ einen δ-Lactonring bedeuten, sowie ihrer optisch aktiven Isomeren, **dadurch gekennzeichnet**, daß es mindestens

a) die Reduktion eines gegebenenfalls optisch aktiven Ketoesters der Formel 4

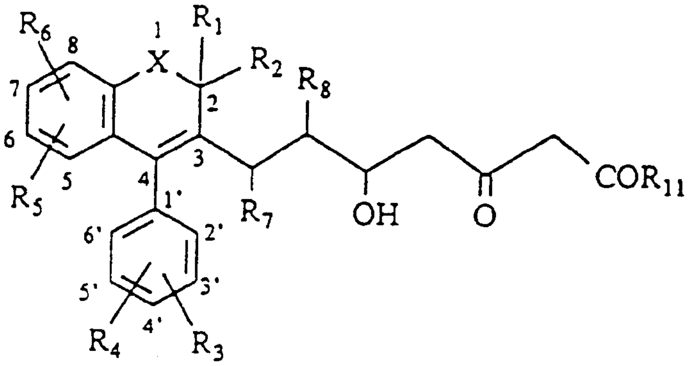

**4**

umfaßt und
gegebenenfalls

b) die Umesterung der Verbindungen der Formel 1 oder die Alkoholyse der Verbindungen der Formel 1 in Form des δ-Lactons oder die Alkylierung der Verbindungen der Formel 1 in Form der Salze und/oder

c) die Hydrolyse der Verbindungen der Formel 1 in Form des Esters oder des δ-Lactons oder

d) wenn $R_7$ und $R_8$ bzw. $R_9$ und $R_{11}$ eine Einfachbindung bilden, die Behandlung der entsprechenden Verbindungen der Formel 1 in Form der Salze mit einem tertiären Chloramin oder

e) wenn $R_7$, $R_8$ und $R_{10}$ ein Wasserstoffatom und $R_9$ und $R_{11}$ gemeinsam eine Bindung bedeuten, die katalytische Reduktion der δ-Lactonverbindungen der Formel 1, in der $R_7$ und $R_8$ eine Bindung bilden, oder die Lactonisierung der entsprechenden Säureverbindungen der Formel 1, worin $R_7$, $R_8$ und $R_{10}$ ein Wasserstoffatom bedeuten, oder

f) die Aminolyse der Verbindungen der Formel 1 in Form des Esters oder des δ-Lactons oder

g) wenn $R_9$ und $R_{10}$ gemeinsam einen Dialkylalkylenrest bilden, die Cyclisierung der entsprechenden Verbindungen der Formel 1, in der $R_9$ und $R_{10}$ jeweils ein Wasserstoffatom bedeuten, mit einem Alkoxyalken, und

h) gegebenenfalls die Aufspaltung des erhaltenen Racemats.

2. Verfahren nach Anspruch 1, **dadurch gekennzeichnet**, daß $R_{11}$ eine Hydroxylgruppe, eine Alkoxygruppe mit 1 bis 4 Kohlenstoffatomen, eine Benzyloxygruppe, eine Alkylaminogruppe oder eine N,N-Dialkylaminogruppe mit 1 bis 3 Kohlenstoffatomen, eine Iminogruppe mit 4 bis 6 Kohlenstoffatomen, eine Cycloaminogruppe mit 3 bis 6 Kohlenstoffatomen, eine Aminogruppe oder eine Benzylaminogruppe oder $-O^-$ $M^+$ bedeutet, worin $M^+$ ein pharmazeutisch annehmbares Kation darstellt, oder $R_{11}$ zusammen mit $R_9$ eine Einfachbindung bildet.

3. Verfahren nach Anspruch 1 oder 2, **dadurch gekennzeichnet,** daß die Substituenten $R_1$ und $R_2$ jeweils eine Methylgruppe oder gemeinsam eine Tetramethylenkette, $R_3$ und $R_4$ bzw. $R_8$ und $R_8$, die gleichartig oder verschieden sein können, Wasserstoffatome, Fluoratome, Chloratome, Methylgruppen, Ethylgruppen, Methoxygruppen, Methylthiogruppen, Phenylgruppen, 4-Fluor-phenylgruppen, 4-Methyl-phenylgruppen, 4-Methoxy-phenylgruppen oder gemeinsam die zweiwertigen Gruppen: -CH=CH-CH=CH-, Tetramethylen: $-(CH_2)_4-$ oder Methylendioxy: $-O(CH_2)O-$ bedeuten.

4. Verfahren nach Anspruch 1 oder 2, **dadurch gekennzeichnet,** daß

A) X ein Sauerstoffatom oder ein Schwefelatom oder eine Methylengruppe, $R_1$ und $R_2$ jeweils eine Methylgruppe oder gemeinsam eine Tetramethylenkette $-(CH_2)_4-$, lediglich eine der Gruppen $R_3$ oder $R_4$ bzw. $R_8$ oder $R_8$ ein Wasserstoffatom, $R_7$ und $R_8$ gemeinsam eine Bindung und $R_9$ und $R_{10}$ jeweils ein Wasserstoffatom bedeuten, oder

B) X, $R_1$, $R_2$, $R_7$, $R_8$, $R_9$ und $R_{10}$ die oben unter A) angegebenen Bedeutungen besitzen und einer der

Substituenten $R_3$ und $R_4$ ein Wasserstoffatom und der andere ein Fluoratom oder ein Chloratom und jeder der beiden Substituenten $R_5$ und $R_5$ ein Wasserstoffatom bedeutet, oder

C) X, $R_1$, $R_2$, $R_7$, $R_5$, $R_9$ und $R_{10}$ die oben unter A) angegebenen Bedeutungen besitzen und einer der Substituenten $R_3$ und $R_4$ ein Wasserstoffatom und der andere ein Fluoratom bedeuten und jeder der beiden Substituenten $R_5$ und $R_5$ ein Wasserstoffatom darstellt, oder

D) X, $R_1$, $R_2$, $R_7$, $R_5$, $R_9$ und $R_{10}$ die unter A) angegebenen Bedeutungen besitzen und die beiden Substituenten $R_3$ und $R_4$ Wasserstoffatome darstellen und lediglich eine der Gruppen $R_5$ und $R_6$ ein Wasserstoffatom bedeutet, oder

E) X, $R_3$, $R_4$, $R_5$, $R_5$, $R_7$, $R_8$, $R_9$ und $R_{10}$ die oben unter C) angegebenen Bedeutungen besitzen und $R_1$ und $R_2$ gemeinsam eine Tetramethytenkette $-(CH_2)_4-$ bilden, oder

F) X, $R_1$, $R_2$, $R_7$, $R_5$, $R_9$ und $R_{10}$ die oben unter E) angegebenen Bedeutungen besitzen und jeder der Substituenten $R_3$, $R_4$, $R_5$ und $R_5$ ein Wasserstoffatom bedeutet.

5. Verfahren nach Anspruch 1 oder 2, **dadurch gekennzeichnet**, daß die hergestellten Produkte die folgenden sind: (+,-)-6E-Erythro-7-(4-(4-fluor-phenyl)-3- spiro(1-2H-benzopyran-2,1'-cyclopentyl))-3,5-dihydroxy-6-heptensäureethylester, (+,-)-6E-Erythro-7-(4-(4-fluor-phenyl)-3-spiro(1-2H-benzopyran-2-1'-cyclopentyl))-3,5-dihydroxy-6-heptensäure-natriumsalz, (+,-)-6E-Erythro-7-(1,2-dihydro-2,2-dimethyl-4-phenyl-napth-3-yl)-3,5-dihydroxy-6-heptensäuremethylester, (+,-)-6E-Erythro-7-(4-(4-chlor-phenyl)-3-spiro(1-2H-benzopyran- 2,1'-cyclopentyl))-3,5-dihydroxy-6-heptensäureethylester, (+,-)-6E-Erythro-7- (4-(4-chlor-phenyl)-3-spiro(1-2H-benzopyran-2,1'-cyclopentyl))-3,5-dihydroxy- 6-heptensäure-natriumsalz, (+,-)-6E-Erythro-3,5-dihydroxy-7-(4-phenyl-2,2- dimethyl-2H-benzothiapyran-3-yl)-6-heptensäuremethylester, (+,-)-6E-Erythro-3,5-dihydroxy-7-(4-phenyl-2,2-dimethyl-2H-benzothiapyran-3-yl)-6-heptensäure-natri umsalz, (+,-)-6E-Erythro-3,5-dihydroxy-7-(4-phenyl-3-spiro(1-2H-benzothiapyran-2,1'-cyclopentyl))-6-heptensäure-natriumsalz, (+,-)-Erythro-7-(4-(4-fluor-phenyl)-3-spiro(1-2H-benzopyran-2,1'-cyclopentyl))-3,5-dihydroxy-heptansäureethylester.

6. Verfahren zur Herstellung von Zwischenverbindungen für die Herstellung der Verbindungen der Formel 1, wie sie in Anspruch 1 oder 2 definiert sind, der allgemeinen Formeln

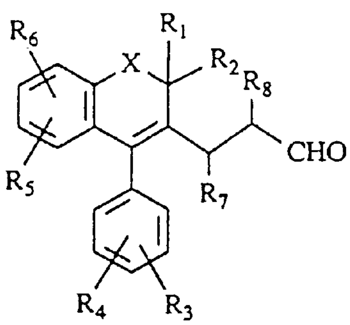

2

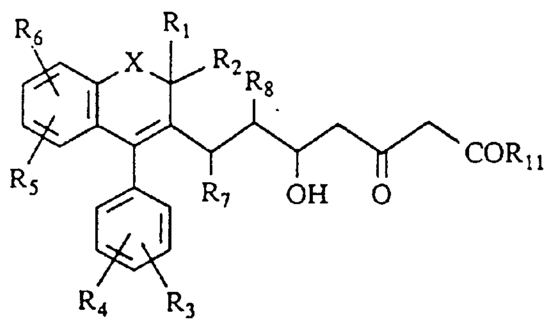

4

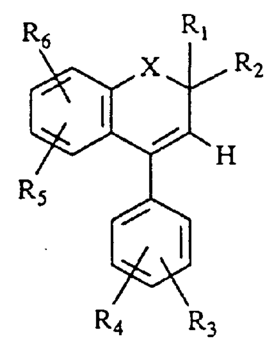

5

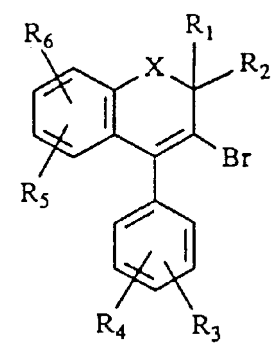

6

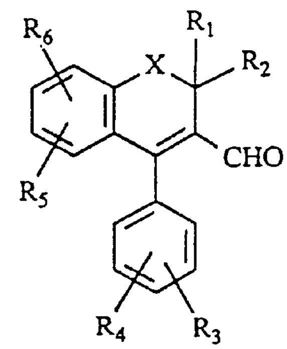

7

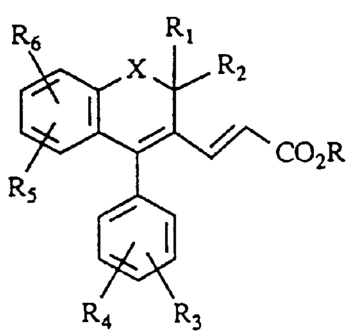

8

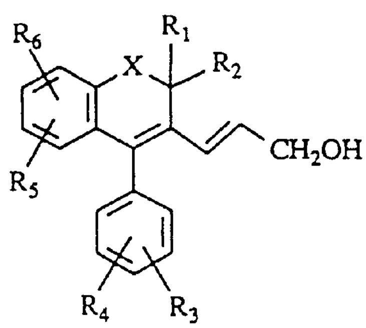

9

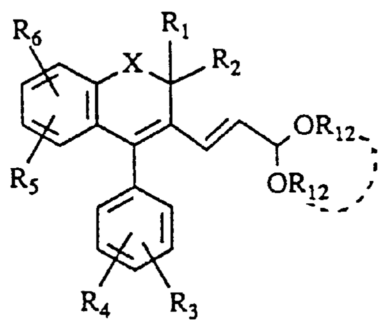

10

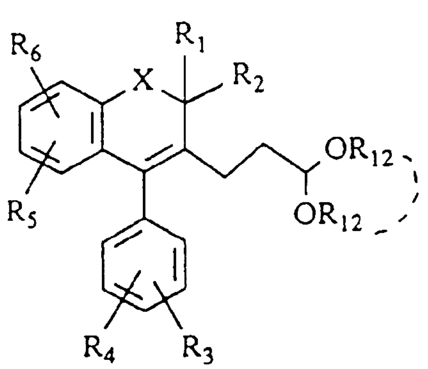

11

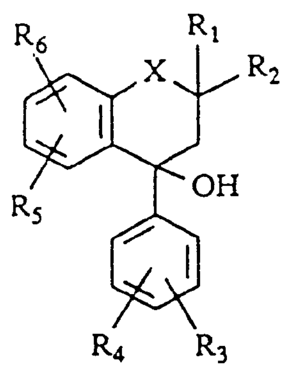

14

worin $R_1$, $R_2$, $R_3$, $R_4$, $R_5$, $R_6$, $R_7$, $R_8$, $R_9$, $R_{10}$ und $R_{11}$ die in Anspruch 1 angegebenen Bedeutungen besitzen und $R_{12}$ eine Alkylgruppe mit 1 bis 4 Kohlenstoffatomen bedeutet oder $R_{12}$...$R_{12}$ eine zweiwertige Methylengruppe $(CH_2)_q$-, worin q 2 oder 3 darstellt, bedeuten und mit den Sauerstoffatomen, an die sie gebunden sind, einen Ring mit 5 oder 6 Kettengliedern bilden, mit Ausnahme der Produkte der Formel 5, worin $R_1$ und $R_2$ H und X S oder $CH_2$ bedeuten, **dadurch gekennzeichnet**, daß es mindestens umfaßt:

α) die Aldolkondensation des entsprechenden Acetoacetats $LiCH_2COCH(Na)\ COR_{11}$ in einem geeigneten

Lösungsmittel in Gegenwart eines geeigneten Komplexierungsmittels mit der entsprechenden Verbindung der Formel 2, oder

β) zur Herstellung der Zwischenverbindungen der Formel 1, worin $R_7$ und $R_8$ eine Doppelbindung bedeuten, die Umsetzung der entsprechenden Verbindung der Formel 5

A) mit 3-N,N'-Dimethylamino-acrolein in einem inerten Lösungsmittel bei einer Temperatur zwischen 20°C und der Ruckflußtemperatur zur Bildung der entsprechenden Verbindung der Formel 2 in der trans-Form; oder

B) mit N-Bromsuccinimid in einem geeigneten Lösungsmittel zur Bildung der entsprechenden 3-bromierten Verbindung der Formel 6,

a) welche mit Butyllithium in einem geeigneten Lösungsmittel und dann mit 3-Ethoxyacrolein oder 3-N,N-Dimethylamino-acrolein umgesetzt wird zur Bildung der entsprechenden Verbindung der Formel 2 in der trans-Form; oder

b) welche mit einem Acrylsäurealkylester in Gegenwart eines basischen Mittels und eines Palladiumderivats in einem geeigneten Lösungsmittel umgesetzt wird zur Bildung der entsprechenden Verbindung 8, oder

C) mit N,N-Dimethylformamid zur Bildung des entsprechenden Aldehyds der Formel 7, der mit Ethoxy- oder Methoxy-carboxymethylen-triphenylphosphoran oder Triphenyl-phosphonoessigsäuremethylester oder -ethylester umgesetzt wird zur Bildung des entsprechenden 3-substituierten Propensäureesters der Formel 8 in der trans-Form, wobei die entsprechende Verbindung 8 in Form des entsprechenden 3-substituierten Propenols der Formel 9 in der trans-Form reduziert wird, welches einer milden Oxidation in einem geeigneten Lösungsmittel unterworfen wird zur Bildung der entsprechenden Verbindung der Formel 2 in der trans-Form, oder

γ) zur Herstellung der Verbindungen der Formel 2, worin $R_7$ und $R_8$ Wasserstoffbedeuten, die Behandlung der entsprechenden Verbindung der Formel 11 im Rahmen einer säurekatalysierten Desacetalisierung in einem geeigneten Lösungsmittel, wobei die entsprechende Verbindung 11 durch katalytische Hydrierung der entsprechenden Verbindung der Formel 10, worin $R_7$ und $R_8$ eine Doppelbindung darstellen, erhalten wird, welche durch Acetalisierng des entsprechenden Aldehyds der Formel 2 hergestellt worden ist, oder durch Behandeln der entsprechenden Verbindung der Formel 6 mit Allylalkohol in Gegenwart eines geeigneten basischen Mittels zur Bildung des entsprechenden Aldehyds der Formel 2.

7. Verfahren zur Herstellung von pharmazeutischen Zubereitungen, **dadurch gekennzeichnet**, daß man mindestens eine Verbindung der Formel 1, wie sie in Anspruch 1 definiert ist, mit einem pharmazeutisch annehmbaren Trägermaterial vermischt.

**Patentansprüche für folgenden Vertragsstaat : GR**

1. Benzocycloalkenyl-dihydroxy-alkansäure-Derivate der folgenden Formel 1

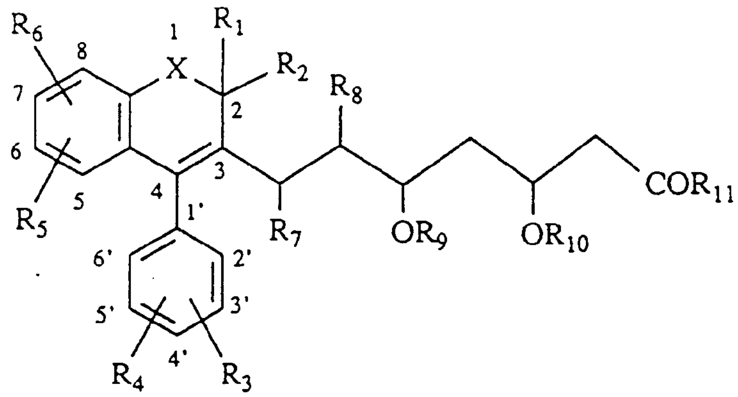

1

in der X eine Methylenkette $-CH_2-$, ein Sauerstoffatom oder ein Schwefelatom; $R_1$ und $R_2$, die gleichartig oder verschieden sein können, Wasserstoffatome oder Alkylgruppen mit 1 bis 3 Kohlenstoffatomen; oder $R_1$ und $R_2$ gemeinsam eine Alkylenkette $-(CH_2)_n-$, deren Anzahl der Kettenglieder n 4 oder 5 sein kann, und die gegebenenfalls durch eine oder zwei Alkylgruppen mit 1 bis 3 Kohlenstoffatomen substituiert sein

kann; $R_3$ und $R_4$, die gleichartig oder verschieden sein können, Wasserstoffatome, Fluoratome, Chloratome oder Bromatome, $CF_3$-Gruppen, N,N-Dialkylaminogruppen mit 1 bis 3 Kohlenstoffatomen, Alkylgruppen mit 1 bis 4 Kohlenstoffatomen, Alkoxygruppen mit 1 bis 5 Kohlenstoffatomen, Phenylgruppen, die gegebenenfalls durch höchstens zwei Substituenten substituiert sein können, die gleichartig oder verschieden sein können, und $C_{1-3}$-Alkylgruppen, oder Fluor- oder Chloratome darstellen, mit der Maßgabe, daß, wenn einer der Substituenten $R_3$ oder $R_4$ eine $CF_3$-Gruppe. eine N,N-Dialkylaminogruppe, eine Phenylgruppe oder eine substituierte Phenylgruppe darstellt, er in der Stellung 3', 4' oder 5' steht und der andere Substituent ein Wasserstoffatom bedeutet; $R_5$ und $R_6$, die gleichartig oder verschieden sein können, Wasserstoffatome, Fluoratome, Chloratome, Bromatome, $CF_3$-Gruppen, $C_{1-3}$-Alkylgruppen, $C_{1-3}$-Alkoxygruppen oder Phenylgruppen, die gegebenenfalls durch höchstens zwei $C_{1-3}$-Alkylgruppen, $C_{1-3}$-Alkoxygruppen, Fluoratome oder Chloratome substituiert sein können, mit der Maßgabe, daß, wenn einer der Substituenten $R_5$ oder $R_6$ eine $CF_3$-Gruppe, eine Phenylgruppe oder eine substituierte Phenylgruppe darstellt, er in der Stellung 6 oder 7 steht und der andere Substituent ein Wasserstoffatom bedeutet; die Substituenten $R_3$ und $R_4$ bzw. $R_5$ und $R_6$, wenn sie an zwei benachbarten Stellungen stehen, auch gemeinsam zweiwertige Gruppen der Formeln -CH=CH-CH=CH-, $-(CH_2)_m-$ oder-$O(CH_2)_pO$-, worin m 3 oder 4 und p 1 oder 2 darstellen, mit der Maßgabe bedeuten, daß, wenn $R_3$ und $R_4$ bzw. $R_5$ und $R_6$ eine zweiwertige Gruppe der Formel -$O(CH_2)_pO$- darstellt, diese an die Stellungen 3' und 4' oder 4' und 5' bzw. 6 und 7 gebunden ist; die Substituenten $R_7$ und $R_6$ jeweils ein Wasserstoffatom oder gemeinsam mit der vorhandenen C-C-Bindung eine Doppelbindung der trans-Form (E); die Substituenten $R_9$ und $R_{10}$ jeweils ein Wasserstoffatom oder gemeinsam eine Dialkylmethylengruppe mit 1 bis 3 Kohlenstoffatomen; und $R_{11}$ mit der Gruppe CO, an die sie gebunden ist, eine freie Säuregruppe, eine Estergruppe, eine Amidgruppe, eine in Salzform vorliegende Säuregruppe oder zusammen mit $R_9$ einen δ-Lactonring bedeuten, sowie deren optisch aktive Isomeren.

2. Verbindung nach Anspruch 1, **dadurch gekennzeichnet**, daß $R_{11}$ eine Hydroxylgruppe, eine Alkoxygruppe mit 1 bis 4 Kohlenstoffatomen, eine Benzyloxygruppe, eine Alkylaminogruppe oder eine N,N-Dialkylaminogruppe mit 1 bis 3 Kohlenstoffatomen, eine Iminogruppe mit 4 bis 6 Kohlenstoffatomen, eine Cycloaminogruppe mit 3 bis 6 Kohlenstoffatomen, eine Aminogruppe oder eine Benzylaminogruppe oder -$O^- M^+$ bedeutet, worin $M^+$ ein pharmazeutisch annehmbares Kation darstellt, oder $R_{11}$ zusammen mit $R_9$ eine Einfachbindung bildet.

3. Verbindungen nach Anspruch 1 oder 2, in deren Formel die Substituenten $R_1$ und $R_2$ jeweils eine Methylgruppe oder gemeinsam eine Tetramethylenkette, $R_3$ und $R_4$ bzw. $R_5$ und $R_6$, die gleichartig oder verschieden sein können, Wasserstoffatome, Fluoratome, Chloratome, Methylgruppen, Ethylgruppen, Methoxygruppen, Methylthiogruppen, Phenylgruppen, 4-Fluor-phenylgruppen, 4-Methyl-phenylgruppen, 4-Methoxy-phenylgruppen oder gemeinsam die zweiwertigen Gruppen: -CH=CH-CH=CH-, Tetramethylen: $-(CH_2)_4-$ oder Methylendioxy: -$O(CH_2)O$- bedeuten.

4. Verbindungen nach Anspruch 1 oder 2, **dadurch gekennzeichnet**, daß
A) X ein Sauerstoffatom oder ein Schwefelatom oder eine Methylengruppe, $R_1$ und $R_2$ jeweils eine Methylgruppe oder gemeinsam eine Tetramethylenkette $-(CH_2)_4-$, lediglich eine der Gruppen $R_3$ oder $R_4$ bzw. $R_6$ oder $R_6$ ein Wasserstoffatom, $R_7$ und $R_6$ gemeinsam eine Bindung und $R_9$ und $R_{10}$ jeweils ein Wasserstoffatom bedeuten, oder
B) X, $R_1$, $R_2$, $R_7$, $R_6$, $R_9$ und $R_{10}$ die oben unter A) angegebenen Bedeutungen besitzen und einer der Substituenten $R_3$ und $R_4$ ein Wasserstoffatom und der andere ein Fluoratom oder ein Chloratom und jeder der beiden Substituenten $R_6$ und $R_6$ ein Wasserstoffatom bedeutet, oder
C) X, $R_1$, $R_2$, $R_7$, $R_6$, $R_9$ und $R_{10}$ die oben unter A) angegebenen Bedeutungen besitzen und einer der Substituenten $R_3$ und $R_4$ ein Wasserstoffatom und der andere ein Fluoratom bedeuten und jeder der beiden Substituenten $R_6$ und $R_6$ ein Wasserstoffatom darstellt, oder
D) X, $R_1$, $R_2$, $R_7$, $R_6$, $R_9$ und $R_{10}$ die unter A) angegebenen Bedeutungen besitzen und die beiden Substituenten $R_3$ und $R_4$ Wasserstoffatome darstellen und lediglich eine der Gruppen $R_6$ und $R_6$ ein Wasserstoffatom bedeutet, oder
E) X, $R_3$, $R_4$, $R_5$, $R_6$, $R_7$, $R_8$, $R_9$ und $R_{10}$ die oben unter C) angegebenen Bedeutungen besitzen und $R_1$ und $R_2$ gemeinsam eine Tetramethylenkette $-(CH_2)_4-$ bilden, oder
F) X, $R_1$, $R_2$, $R_7$, $R_8$, $R_9$ und $R_{10}$ die oben unter E) angegebenen Bedeutungen besitzen undjeder der Substituenten $R_3$, $R_4$, $R_6$ und $R_6$ ein Wasserstoffatom bedeutet.

5. Verbindungen nach Anspruch 1 oder 2, nämlich: (+,-)-6E-Erythro-7-(4-(4- fluor-phenyl)-3-spiro(1-2H-

benzopyran-2,1'-cyclopentyl))-3,5-dihydroxy-6- heptensäureethylester, (+,-)-6E-Erythro-7-(4-(4-fluor-phenyl)-3-spiro(1-2H- benzopyran-2,1'-cyclopentyl))-3,5-dihydroxy-6-heptensäure-natriumsalz, (+,-)-6E-Erythro-7-(1,2-dihydro-2,2-dimethyl-4-phenyl-napth-3-yl)-3,5-dihydroxy- 6-heptensäuremethylester, (+,-)-6E-Erythro-7-(4-(4-chlor-phenyl)-3-spiro(1-2H-benzopyran-2,1'-cyclopentyl))-3,5-dihydroxy-6-he ptensäureethylester, (+,-)-6E-Erythro-7-(4-(4-chlor-phenyl)-3-spiro(1-2H-benzopyran-2,1'-cyclopentyl))-3,5-dihydroxy-6-heptensäure-natriumsalz, (+,-)-6E-Erythro-3,5-dihydroxy-7-(4-phenyl-2,2-dimethyl-2H-benzothiapyran-3-yl)-6-heptensäuremethylester, (+,-)-6E-Erythro-3,5-dihydroxy-7-(4-phenyl-2,2-dimethyl-2H-benzothiapyran-3-yl)-6-heptensäure-natriumsalz, (+,-)-6E-Erythro-3,5-dihydroxy-7-(4-phenyl-3-spiro(1-2H-benzothiapyran-2,1'-cyclopentyl))-6-heptensäure-natriumsalz, (+,-)-Erythro-7-(4-(4-fluor-phenyl)-3-spiro(1-2H-benzopyran-2,1'- cyclopentyl))-3,5-dihydroxy-heptansäureethylester.

6. Verfahren zur Herstellung der Verbindungen der Formel 1, wie sie in Anspruch 1 oder 2 definiert sind, **dadurch gekennzeichnet**, daß sie mindestens

a) die Reduktion eines gegebenenfalls optisch aktiven Ketoesters der Formel 4

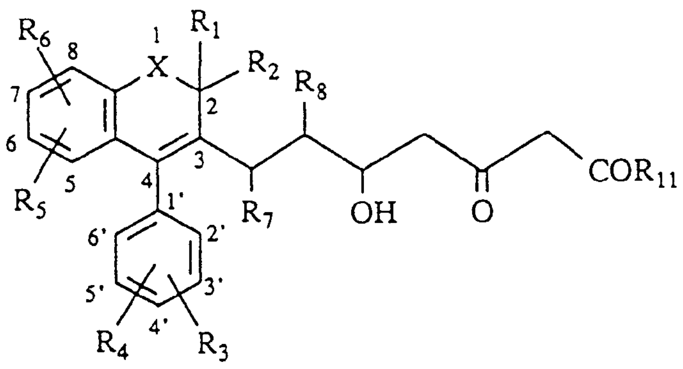

4

umfassen und
gegebenenfalls

b) die Umesterung der Verbindungen der Formel 1 oder die Alkoholyse der Verbindungen der Formel 1 in Form des δ-Lactons oder die Alkylierung der Verbindungen der Formel 1 in Form der Salze und/oder

c) die Hydrolyse der Verbindungen der Formel 1 in Form des Esters oder des δ-Lactons oder

d) wenn $R_7$ und $R_8$ bzw. $R_9$ und $R_{11}$ eine Einfachbindung bilden, die Behandlung der entsprechenden Verbindungen der Formel 1 in Form der Salze mit einem tertiären Chloramin oder

e) wenn $R_7$, $R_8$ und $R_{10}$ ein Wasserstoffatom und $R_9$ und $R_{11}$ gemeinsam eine Bindung bedeuten, die katalytische Reduktion der δ-Lactonverbindungen der Formel 1, in der $R_7$ und $R_8$ eine Bindung bilden, oder die Lactonisierung der entsprechenden Säureverbindungen der Formel 1, worin $R_7$, $R_8$ und $R_{10}$ ein Wasserstoffatom bedeuten, oder

f) die Aminolyse der Verbindungen der Formel 1 in Form des Esters oder des δ-Lactons oder

g) wenn $R_9$ und $R_{10}$ gemeinsam einen Dialkylalkylenrest bilden, die Cyclisierung der entsprechenden Verbindungen der Formel 1, in der $R_9$ und $R_{10}$ jeweils ein Wasserstoffatom bedeuten, mit einem Alkoxyalken, und

h) gegebenenfalls die Aufspaltung des erhaltenen Racemats.

7. Zwischenverbindungen zur Herstellung der Verbindungen der Formel 1, wie sie in Anspruch 1 oder 2 definiert sind, der folgenden allgemeinen Formeln

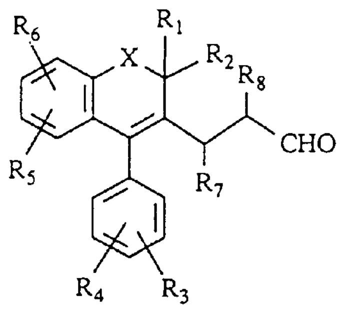

2

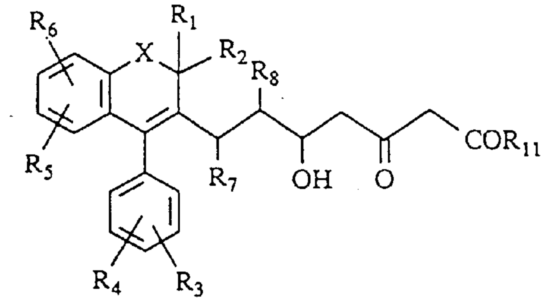

4

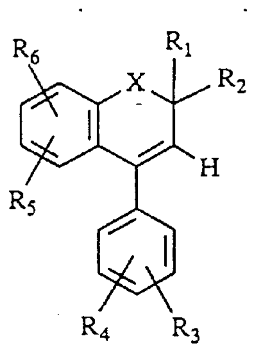

5

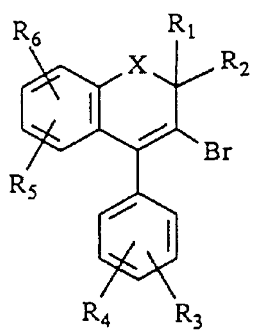

6

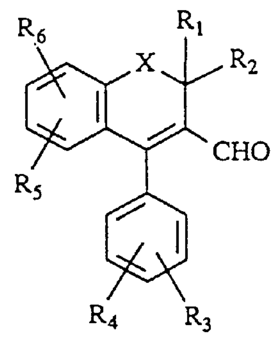

7

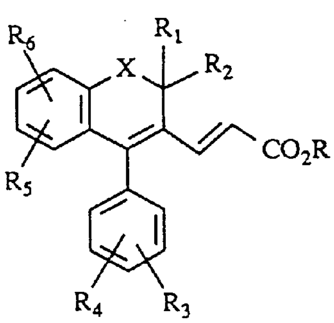

8

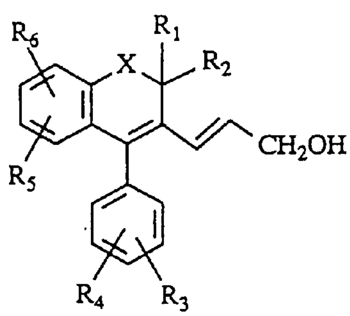

9

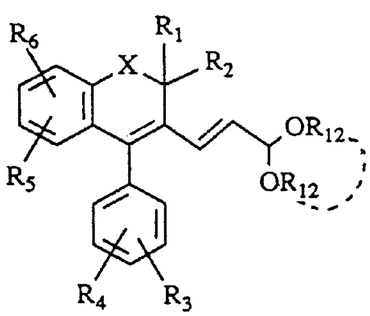

10

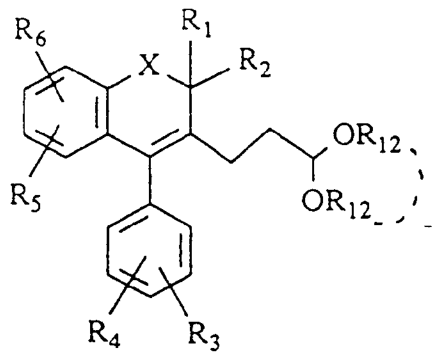

11

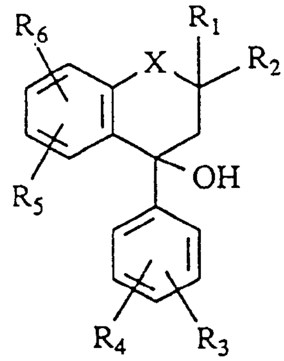

14

worin $R_1$, $R_2$, $R_3$, $R_4$, $R_5$, $R_6$, $R_7$, $R_8$, $R_9$, $R_{10}$ und $R_{11}$ die in Anspruch 1 angegebenen Bedeutungen besitzen und $R_{12}$ eine Alkylgruppe mit 1 bis 4 Kohlenstoffatomen bedeutet oder $R_{12}...R_{12}$ eine zweiwertige Methylengruppe $-(CH_2)_q-$, worin q 2 oder 3 darstellt, bedeuten und mit den Sauerstoffatomen, an die sie gebunden sind, einen Ring mit 5 oder 6 Kettengliedern bilden, mit Ausnahme der Produkte der Formel 5, worin $R_1$ und $R_2$ H und X S oder $CH_2$ bedeuten.

8. Verfahren zur Herstellung der Zwischenverbindungen der Formel 4, wie sie in Anspruch 7 definiert sind, **dadurch gekennzeichnet**, daß es die Aldolkondensation des entsprechenden Acetoacetats $LiCH_2COCH(Na)COR_{11}$ in einem geeigneten Lösungsmittel in Gegenwart eines geeigneten Komplexierungsmittels mit der entsprechenden Verbindung der Formel 2 umfaßt.

9. Verfahren zur Herstellung der Zwischenverbindungen der Formel 2, wie sie in Anspruch 7 definiert sind, worin $R_7$ und $R_8$ eine Doppelbindung bedeuten, **dadurch gekennzeichnet,** daß es die Reaktion der entsprechenden Verbindung der Formel 5

A) mit 3-N,N'-Dimethylamino-acrolein in einem inerten Lösungsmittel bei einer Temperatur zwischen 20°C und der Rückflußtemperatur zur Bildung der entsprechenden Verbindung der Formel 2 in der trans-Form; oder

B) mit N-Bromsuccinimid in einem geeigneten Lösungsmittel zur Bildung der entsprechenden 3-bromierten Verbindung der Formel 6,

a) welche mit Butyllithium in einem geeigneten Lösungsmittel und dann mit 3-Ethoxyacrolein oder 3-N,N-Dimethylamino-acrolein umgesetzt wird zur Bildung der entsprechenden Verbindung der Formel 2 in der trans-Form; oder

b) welche mit einem Acrylsäurealkylester in Gegenwart eines basischen Mittels und eines Palladiumderivats in einem geeigneten Lösungsmittel umgesetzt wird zur Bildung der entsprechenden Verbindung 8, oder

C) mit N,N-Dimethylformamid zur Bildung des entsprechenden Aldehyds der Formel 7, der mit Ethoxy- oder Methoxy-carboxymethylen-triphenylphosphoran oder Triphenyl-phosphonoessigsäuremethylester oder -ethylester umgesetzt wird zur Bildung des entsprechenden 3-substituierten Propensäureesters der Formel 8 in der trans-Form, wobei die entsprechende Verbindung 8 in Form des entsprechenden 3-substituierten Propenols der Formel 9 in der trans-Form reduziert wird, welches einer milden Oxidation in einem geeigneten Lösungsmittel unterworfen wird zur Bildung der entsprechenden Verbindung der Formel 2 in der trans-Form umfaßt.

10. Verfahren zur Herstellung der Verbindungen der Formel 2, wie sie in Anspruch 7 definiert sind, worin $R_7$ und $R_6$ Wasserstoff bedeuten, **dadurch gekennzeichnet,** daß man die entsprechende Verbindung der Formel 11 einer säurekatalysierten Desacetalisierung in einem geeigneten Lösungsmittel unterwirft, wobei die entsprechende Verbindung 11 durch katalytische Hydrierung der entsprechenden Verbindung der Formel 10, worin $R_7$ und $R_6$ eine Doppelbindung darstellen, erhalten wird, welche man durch Acetalisierung des entsprechenden Aldehyds der Formel 2 erhalten hat, oder indem man die entsprechende Verbindung der Formel 6 mit Allylalkohol in Gegenwart eines geeigneten basischen Mittels behandelt, um den entsprechenden Aldehyd der Formel 2 zu erhalten.

11. Verfahren zur Herstellung von pharmazeutischen Zubereitungen, **dadurch gekennzeichnet,** daß man

mindestens eine Verbindung der Formel 1, wie sie in Anspruch 1 definiert worden ist, mit einem pharmazeutisch annehmbaren Träger vermischt.

## Claims

**Claims for the following Contracting States : AT, BE, CH, DE, DK, FR, GB, IT, LI, LU, NL, SE**

1. Derivatives of benzocycloalkenyldihydroxyalkanoic acids of the following formula 1,

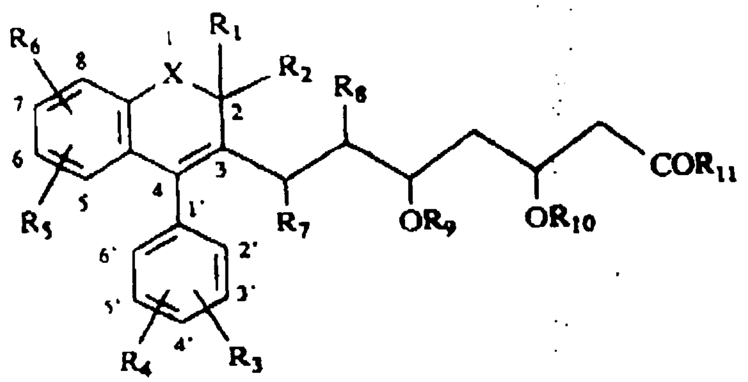

1

in which: X denotes a $-CH_2-$ methylene group or an oxygen or sulphur atom; $R_1$ and $R_2$, which are identical or different, denote hydrogen atoms or alkyl radicals containing 1 to 3 carbon atoms; $R_1$ and $R_2$ may also together form a $-(CH_2)_n-$ alkylene chain in which the number of groups n may be equal to 4 or 5 and, if appropriate, substituted symmetrically by one or two alkyl radicals containing 1 to 3 carbon atoms; $R_3$ and $R_4$, which may be identical or different, denote hydrogen, fluorine, chlorine or bromine atoms, $CF_3$ radicals, N,N-dialkylamino containing 1 to 3 carbon atoms, alkyl containing 1 to 4 carbon atoms, alkoxy containing 1 to 5 carbon atoms, phenyl optionally substituted by at most two substituents which may be identical or different and may denote $C_{1-3}$-alkyl radicals or fluorine or chlorine atoms, it being understood that when one of the substituents $R_3$ and $R_4$ denotes a $CF_3$, N,N-dialkylamino, phenyl or substituted phenyl radical, it is present on the 3', 4' or 5' positions and the other substituent denotes a hydrogen atom; $R_5$ and $R_6$, which may be identical or different, denote hydrogen, fluorine, chlorine or bromine atoms or the radicals: $CF_3$, $C_{1-3}$-alkyl, $C_{1-3}$-alkoxy or phenyl, substituted if appropriate by at most two $C_{1-3}$-alkyl or $C_{1-3}$-alkoxy radicals, or fluorine or chlorine atoms, on condition that when one of the substituents $R_4$ and $R_6$ denotes the radicals $CF_3$, phenyl or substituted phenyl, it is present on the positions 6 or 7 and the other denotes a hydrogen atom; the substituents $R_3$ and $R_4$ or $R_5$ and $R_6$ may also together form, on condition of being on two adjacant positions, the diradicals of formulae: -CH=CH-CH=CH-, $-(CH_2)_m-$ or $-O(CH_2)_pO-$, in which m may be equal to 3 or 4 and p to 1 or 2, it being understood that when $R_3$ and $R_4$ or $R_5$ and $R_6$ denote the diradical $-O(CH_2)_pO-$ the latter is linked to the positions 3' and 4' or 4' and 5' or 6 and 7; each of the substituents $R_7$ and $R_8$ denotes a hydrogen atom or, with the existing C-C bond, they together form a double bond of trans (E) geometry; each of the substituents $R_9$ and $R_{10}$ denotes a hydrogen atom or they together form a dialkylmethylene radical containing 1 to 3 carbon atoms, $R_{11}$ denoting, with the CO group to which it is bonded, a free acid, ester, amide or acid salt functional group or forming a δ-lactone ring with $R_9$, and their optically active isomers.

2. Compound according to Claim 1, characterized in that $R_{11}$ denotes one of the following radicals: hydroxy, alkoxy containing 1 to 4 carbon atoms, benzyloxy, alkylamino or N,N-dialkylamino containing 1 to 3 carbon atoms, imino containing 4 to 6 carbon atams, cycloamino containing 3 to 6 carbon atoms, amino or benzylamino or $-O^-M^+$ where $M^+$ denotes a pharmaceutically acceptable cation, or $R_{11}$ forms a single bond with $R_9$.

3. Compounds according to Claim 1 or 2, in the formula of which each of the substituents $R_1$ and $R_2$ denotes a methyl group or they together form a tetramethylene chain, $R_3$ and $R_4$ or $R_6$ and $R_6$, which may be identical or different, denote hydrogen, fluorine or chlorine atoms, methyl or ethyl, methoxy, methylthio, phenyl, 4-fluorophenyl, 4-methylphenyl or 4-methoxyphenyl radicals or together form one of the following diradicals: -CH=CH-CH=CH-, tetramethylene: $-(CH_2)_4-$ or methylenedioxy: $-O(CH_2)O-$.

4. Compounds according to Claim 1 or 2, characterized in that

A) X denotes oxygen or sulphur atoms or a methylene group, each of $R_1$ and $R_2$ denotes a methyl radical or they together form a $-(CH_2)_4-$ tetramethylene chain, only one of the radicals $R_3$ and $R_4$ or $R_5$ and $R_5$ denotes a hydrogen atom, $R_7$ and $R_8$ together form a bond and each of $R_9$ and $R_{10}$ denotes a hydrogen atom, or

B) X, $R_1$, $R_2$, $R_7$, $R_8$, $R_9$ and $R_{10}$ have the meanings given immediately above in A), one of the substituents $R_3$ and $R_4$ denotes a hydrogen atom and the other a fluorine or chorine atom, and each of the two substituents $R_5$ and $R_5$ denotes a hydrogen atom, or

C) X, $R_1$, $R_2$, $R_7$, $R_5$, $R_9$ and $R_{10}$ have the meanings which have just been defined above in A), one of the substituents $R_3$ and $R_4$ is a hydrogen atom and the other denotes a fluorine atom, and each of the two substituents $R_5$ and $R_5$ denotes a hydrogen atom, or

D) X, $R_1$, $R_2$, $R_7$, $R_8$, $R_9$ and $R_{10}$ have the particular meanings defined in A), the two substituents $R_3$ and $R_4$ are hydrogen atoms and only one of the radicals $R_5$ and $R_6$ denotes a hydrogen atom, or

E) X, $R_3$, $R_4$, $R_5$, $R_6$, $R_7$, $R_8$, $R_9$ and $R_{10}$ have the meanings above in C) and $R_1$ and $R_2$ together form a $-(CH_2)_4-$ tetramethylene chain, or

F) X, $R_1$, $R_2$, $R_7$, $R_5$, $R_9$ and $R_{10}$ have the meanings shown immediately above in E) and each of the substituents $R_3$, $R_4$, $R_5$ and $R_6$ denotes a hydrogen atom.

5. Compounds according to Claim 1 or 2, called: (+,-)ethyl 6E-erythro-7-(4-(4-fluorophenyl-3-spiro(2,1'-cyclopentyl-2H-1-benzopyran))-3,5-dihydroxyhept-6-enoate, (+,-)sodium 6E-erythro-7-(4-(4-fluorophenyl)-3-spiro-(2,1'-cyclopentyl-2H-1-benzopyran))-3,5-dihydroxyhept-6-enoate, (+,-)methyl 6E-erythro-7-(1,2-dihydro-2,2-dimethyl-4-phenyl-3-naphthyl)-3,5-dihydroxyhept-6-enoate, (+,-)ethyl 6E-erythro-7-(4-(4-chlorophenyl)-3-spiro(2,1'-cyclopentyl-2H-1-benzopyran))-3,5-dihydroxyhept-6-enoate, (+,-)-sodium 6E-erythro-7-(4-(4-chlorophenyl)-3-spiro(2,1'-cyclopentyl-2H-1-benzopyran)-3,5-dihydroxyhept-6-enoate, (+,-) methyl 6E-erythro-3,5-dihydroxy-7-(4-phenyl-2,2-dimethyl-3-2H-benzothiapyranyl)hept-6-enoate, (+,-)sodium 6E-erythro-3,5-dihydroxy-7-(4-phenyl-2,2-dimethyl-3-2H-benzothiapyranyl)hept-6-enoate, (+,-)sodium 6E-erythro-3,5-dihydroxy-7-(4-phenyl-3-spiro(2,1'-cyclopentyl-2H-1-benzothiapyran))hept-6-enoate, (+,-)ethyl erythro-7-(4-(4-fluorophenyl)-3-spiro(2,1'-cyclopentyl-2H-1-benzopyran))-3,5-dihydroxyheptanoate.

6. Processes for the preparation of the compounds of the formula 1 as defined in Claim 1 or 2, characterized in that they comprise at least

a) reducing a keto-ester of the formula 4, which may be optically active,

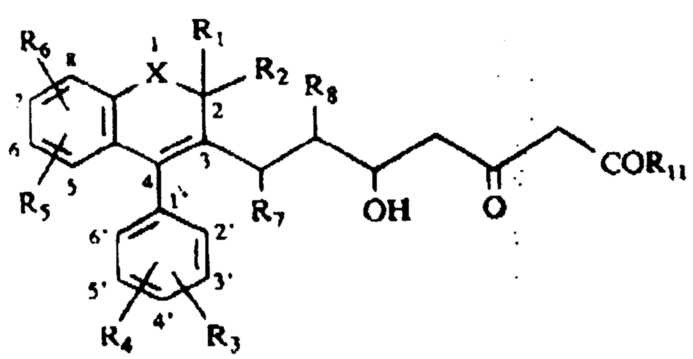

4

and, if appropriate,

b) transesterifying compounds of the formula 1 or alcoholizing compounds of the formula 1 in δ-lactone form or else alkylating compounds of the formula 1 in the form of salts and, or

c) hydrolysing compounds of the formula 1 in ester or δ-lactone form, or

d) when $R_7$ and $R_5$ or $R_9$, and $R_{11}$ together form single bond: treating the corresponding compounds

of the formula 1 in the form of salts using a tertiary chloroamine, or

e) when $R_7$, $R_8$ and $R_{10}$ denote a hydrogen atom and $R_9$ and $R_{11}$ together form a bond: catalytically reducing the δ-lactone compounds of the formula 1 in which $R_7$ and $R_8$ form a bond or lactonizing of the corresponding acid compounds of the formula 1 in which $R_7$, $R_8$ and $R_{10}$ denote a hydrogen atom, or

f) aminolysing the compounds of the formula 1 in ester or δ-lactone form, or

g) when $R_9$ and $R_{10}$ together form a dialkylalkylene radical, cyclizing the corresponding compounds of the formula 1 in which each of $R_9$ and $R_{10}$ denotes a hydrogen atom, using an alkoxyalkene, and

h) optionally splitting the racemate obtained.

7. Intermediate compounds in the preparation of the compounds of the formula 1 as defined in Claim 1 or 2, which are denoted by the general formulae,

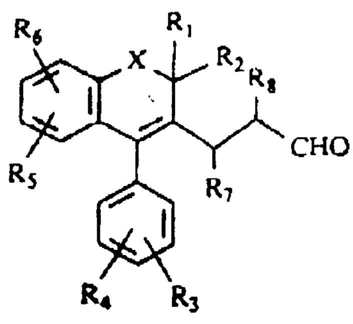

2

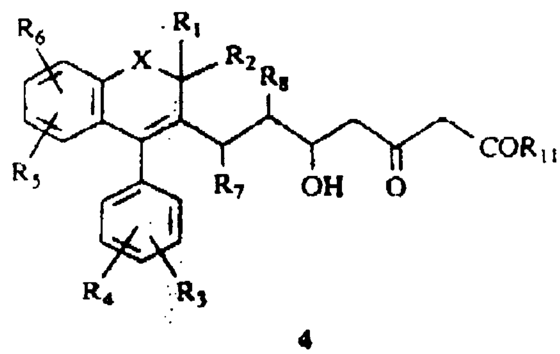

4

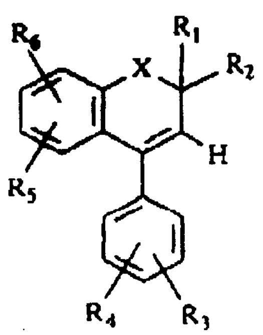

5

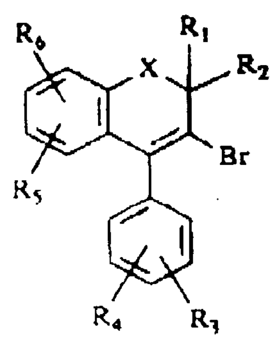

6

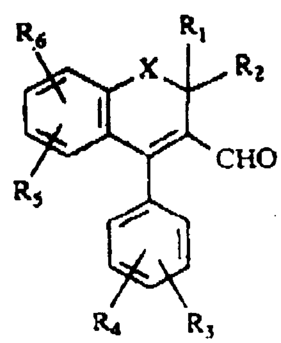

7

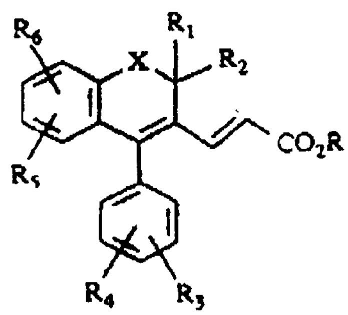

8

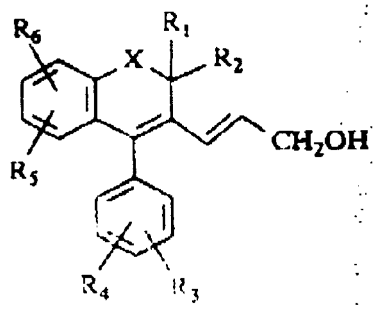

9

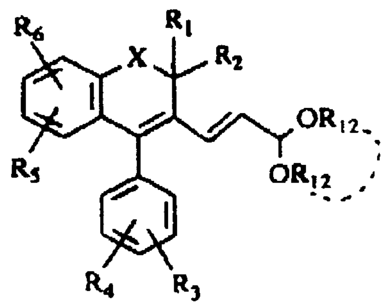

10

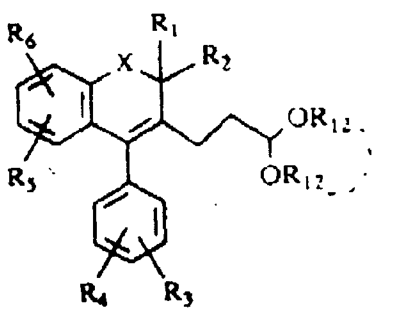

11

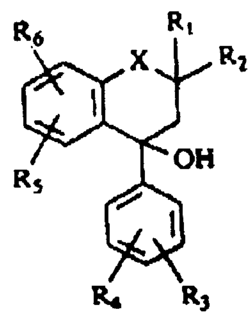

14

in which $R_1$, $R_2$, $R_3$, $R_4$, $R_5$, $R_6$, $R_7$, $R_6$, $R_9$, $R_{10}$ and $R_{11}$ are defined in Claim 1 and $R_{12}$ denotes an alkyl radical containing 1 to 4 carbon atoms or $R_{12}$ ... $R_{12}$ denotes a $-(CH_2)_q$-methylene diradical where q denotes 2 or 3 and forms a 5 or 6-membered ring with the oxygen atoms to which it is attached, with the exception of the products of formula 5 where $R_1$ and $R_2$ denote H and X denotes S or $CH_2$.

8. Process for the preparation of the intermediate compounds of the formula 4, as defined in Claim 7, characterized in that it comprises aldol condensation of the corresponding acetoacetate $LiCH_2COCH(Na)COR_{11}$ in an appropriate solvent, in the presence of an appropriate complexing agent, with the corresponding compound of the formula 2.

9. Process for the preparation of the intermediate compounds of the formula 2, which are defined in Claim 7, where $R_7$ and $R_6$ denote a double bond, characterized in that it comprises reacting the corresponding compound of the formula 5

A) with N,N'-dimethyl-3-aminoacrolein in an inert solvent at a temperature between 20°C and the reflux temperature to form the corresponding compound of the formula 2 in trans form; or

B) with N-bromosuccinimide in an appropriate solvent to produce the corresponding 3-brominated compound of the formula 6,

a) which reacts with butyllithium in an appropriate solvent and then 3-ethoxyacrolein or N,N-dimethyl-3-aminoacrolein to form the corresponding compound of the formula 2 in trans form; or

b) which reacts with an alkyl acrylate in the presence of a basic agent and of a palladium derivative

in an appropriate solvent to form the corresponding compound 8, or

C) N,N-dimethylformamide to produce the corresponding aldehyde of the formula 7, which reacts with ethoxy- or methoxycarboxymethylenetriphenylphosphorane or methyl or ethyl triphenylphosphonoacetate to give the corresponding 3-substituted propenoic ester of the formula 8 in trans form, the corresponding compound 8 being reduced in the form of the corresponding 3-substituted propenol of the formula 9 in trans form, which undergoes a controlled oxidation in an appropriate solvent to produce the corresponding compound of the formula 2 in trans form.

**10.** Process for the preparation or the compounds of the formula 2 which are defined in Claim 7, where $R_7$ and $R_8$ denote hydrogen, characterized in that the corresponding compound of the formula 11 is subjected to a deacetalization with acidic catalysis in an appropriate solvent, the corresponding compound 11 being prepared by catalytic hydrogenation of the corresponding compound of the formula 10 where $R_7$ and $R_8$ denote a double bond, obtained by acetalization of the corresponding aldehyde of the formula 2, or in that the corresponding compound of the formula 6 is treated with allyl alcohol in the presence of an appropriate basic agent to obtain the corresponding aldehyde of the formula 2.

**11.** Medicament having as its active ingredient a compound of the formula 1 according to one of Claims 1 to 5.

**12.** Medicament according to Claim 11, in dose unit form in which each dose unit contains from 1 to 500 mg of active ingredient mixed with a pharmaceutically acceptable excipient.

**13.** Use of the compounds of the formula 1 according to one of Claims 1 to 5 for the preparation of medicanents which can be used as hypocholesterolaemiants, antiatherosclerous agents and antithrombotic agents in the treatment of cardiovascular disorders and thrombotic symptoms of diabetes, atherosclerosis or hyperlipoproteinaemia.

**14.** Use of the compounds of the formula 1 according to one of claims 1 to 5 for the preparation of medicaments which can be used as antifungal agents.

**15.** Pharmaceutical compositions, characterized in that they comprise at least one compound of the formula 1 as defined in Claim 1, and a pharmaceutically acceptable excipient.

**Claims for the following Contracting State : ES**

**1.** Process for the preparation of derivatives of benzocycloalkenyldihydroxyalkanoic acids of the following formula 1,

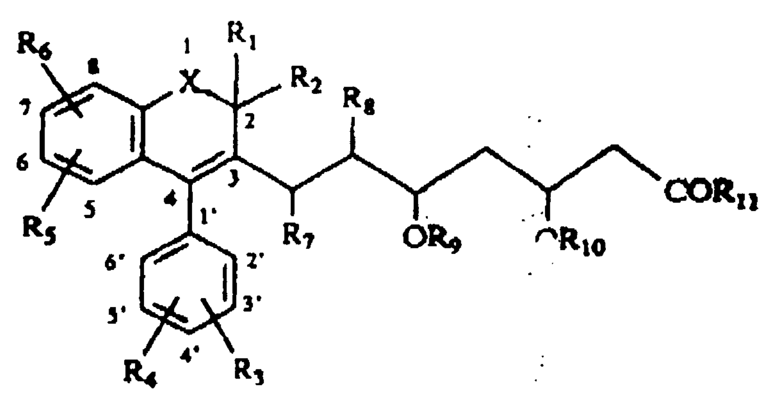

1

and their optically active isomers,

in which: X denotes a $-CH_2-$ methylene group or an oxygen or sulphur atom; $R_1$ and $R_2$, which are identical or different, denote hydrogen atoms or alkyl radicals containing 1 to 3 carbon atoms; $R_1$ and $R_2$ may also together form a $-(CH_2)_n-$ alkylene chain in which the number of groups n may be equal to 4 or 5 and, if appropriate, substituted symmetrically by one or two alkyl radicals containing 1 to 3 carbon atoms; $R_3$ and $R_4$, which may be identical or different, denote hydrogen, fluorine, chlorine or bromine atoms, $CF_3$ radicals,

N,N-dialkylamino containing 1 to 3 carbon atoms, alkyl containing 1 to 4 carbon atoms, alkoxy containing 1 to 5 carbon atoms, phenyl optionally substituted by at most two substituents which may be identical or different and may denote $C_{1-3}$-alkyl radicals or fluorine or chlorine atoms, it being understood that when one of the substituents $R_3$ and $R_4$ denotes a $CF_3$, N,N-dialkylamino, phenyl or substituted phenyl radical, it is present on the 3', 4' or 5' positions and the other substituent denotes a hydrogen atom; $R_5$ and $R_6$, which may be identical or different, denote hydrogen, fluorine, chlorine or bromine atoms or the radicals: $CF_3$, $C_{1-3}$-alkyl, $C_{1-3}$-alkoxy or phenyl, substituted if appropriate by at most two $C_{1-3}$-alkyl or $C_{1-3}$-alkoxy radicals, or fluorine or chlorine atoms, on condition that when one of the substituents $R_5$ and $R_6$ denotes the radicals $CF_3$, phenyl or substituted phenyl, it is present on the positions 6 or 7 and the other denotes a hydrogen atom; the substituents $R_3$ and $R_4$ or $R_5$ and $R_6$ may also together form, on condition of being on two adjacant positions, the diradicals of formulae: -CH=CH-CH=CH-, $-(CH_2)_m-$ or $-O(CH_2)_pO-$, in which m may be equal to 3 or 4 and p to 1 or 2, it being understood that when $R_3$ and $R_4$ or $R_5$ and $R_6$ denote the diradical $-O(CH_2)_pO-$ the latter is linked to the positions 3' and 4' or 4' and 5' or 6 and 7; each of the substituents $R_7$ and $R_6$ denotes a hydrogen atom or, with the existing C-C bond, they together form a double bond of trans (E) geometry; each of the substituents $R_9$ and $R_{10}$ denotes a hydrogen atom or they together form a dialkylmethylene radical containing 1 to 3 carbon atoms, $R_{11}$ denoting, with the CO group to which it is bonded, a free acid, ester, amide or acid salt functional group or forming a δ-lactone ring with $R_9$, characterized in that it comprises at least

a) reducing a keto-ester of the formula 4, which may be optically active,

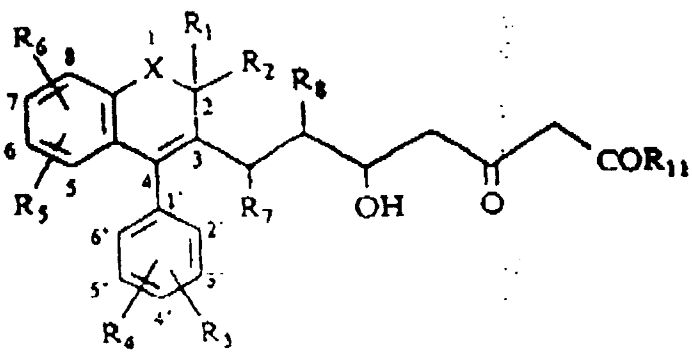

4

and, if appropriate,

b) transesterifying compounds of the formula 1 or alcoholizing compounds of the formula 1 in δ-lactone form or else alkylating compounds of the formula 1 in the form of salts and, or

c) hydrolysing compounds of the formula 1 in ester or δ-lactone form, or

d) when $R_7$ and $R_6$ or $R_9$ and $R_{11}$ together, form a single bond: treating the corresponding compounds of the formula 1 in the form of salts using a tertiary chloroamine, or

e) when $R_7$, $R_6$ and $R_{10}$ denote a hydrogen atom and $R_9$ and $R_{11}$ together form a bond: catalytically reducing the δ-lactone compounds of the formula 1 in which $R_7$ and $R_8$ form a bond or lactonizing of the corresponding acid compounds of the formula 1 in which $R_7$, $R_6$ and $R_{10}$ denote a hydrogen atom, or

f) aminolysing the compounds of the formula 1 in ester or δ-lactone form, or

g) when $R_9$ and $R_{10}$ together form a dialkylalkylene radical, cyclizing the corresponding compounds of the formula 1 in which each of $R_9$ and $R_{10}$ denotes a hydrogen atom, using an alkoxyalkene, and

h) optionally splitting the racemate obtained.

2. Process according to Claim 1, characterized in that $R_{11}$ denotes one of the following radicals: hydroxy, alkoxy containing 1 to 4 carbon atoms, benzyloxy, alkylamino or N,N-dialkylamino containing 1 to 3 carbon atoms, imino containing 4 to 6 carbon atoms, cycloamino containing 3 to 6 carbon atoms, amino or benzylamino or $-O^-M^+$ where $M^+$ denotes a pharmaceutically acceptable cation, or $R_{11}$ forms a single bond with $R_9$.

3. Process according to Claim 1 or 2, characterized in that each of the substituents $R_1$ and $R_2$ denotes a

methyl group or they together form a tetramethylene chain, $R_3$, and $R_4$ or $R_5$ and $R_5$, which may be identical or different, denote hydrogen, fluorine or chlorine atoms, methyl or ethyl, methoxy, methylthio, phenyl, 4-fluorophenyl, 4-methylphenyl or 4-methoxyphenyl radicals or together form one of the following diradicals: -CH=CH-CH-CH-, tetramethylene: $-(CH_2)_4-$ or methylenedioxy: $-O(CH_2)O-$.

**4.** Process according to claim 1 or 2, characterized in that

A) X denotes oxygen or sulphur atoms or a methylene group, each of $R_1$ and $R_2$ denotes a methyl radical or they together form a $-(CH_2)_4-$ tetramethylene chain, only one of the radicals $R_3$ and $R_4$ or $R_5$ and $R_5$ denotes a hydrogen atom, $R_7$, and $R_8$ together form a bond and each of $R_9$ and $R_{10}$ denotes a hydrogen atom, or

B) X, $R_1$, $R_2$, $R_7$, $R_8$, $R_9$, and $R_{10}$ have the meanings given immediately above in A), one of the substituents $R_3$ and $R_4$ denotes a hydrogen atom and the other a fluorine or chorine atom, and each of the two substituents $R_5$ and $R_5$ denotes a hydrogen atom, or

C) X, $R_1$, $R_2$, $R_7$, $R_5$, $R_9$ and $R_{10}$ have the meanings which have just been defined above in A), one of the substituents $R_3$ and $R_4$ is a hydrogen atom and the other denotes a fluorine atom, and each of the two substituents $R_5$ and $R_5$ denotes a hydrogen atom, or

D) X, $R_1$, $R_2$, $R_7$, $R_8$, $R_9$ and $R_{10}$ have the particular meanings defined in A), the two substituents $R_3$ and $R_4$ are hydrogen atoms and only one of the radicals $R_5$ and $R_6$ denotes a hydrogen atom, or

E) X, $R_3$, $R_4$, $R_5$, $R_5$, $R_7$, $R_5$, $R_9$ and $R_{10}$, have the meanings above in C) and $R_1$ and $R_2$ together form a $-(CH_2)_4-$ tetramethylene chain, or

F) X, $R_1$, $R_2$, $R_7$, $R_5$, $R_9$ and $R_{10}$ have the meanings shown immediately above in E) and each of the substituents $R_3$, $R_4$, $R_5$ and $R_6$ denotes a hydrogen atom.

**5.** Process according to Claim 1 or 2, characterized in that the products prepared are: (+,-)ethyl 6E-erythro-7-(4-(4-fluorophenyl-3-spiro(2,1'-cyclopentyl-2H-1-benzopyran))-3,5-dihydroxyhept-6-enoate, (+,-)sodium 6E-erythro-7-(4-(4-fluorophenyl)-3-spiro-(2,1'-cyclopentyl-2H-1-benzopyran))-3,5-dihydroxyhept-6-enoate, (+,-)methyl 6E-erythro-7-(1,2-dihydro-2,2-dimethyl-4-phenyl-3-naphthyl)-3,5-dihydroxyhept-6-enoate, (+,-)ethyl 6E-erythro-7-(4-(4-chlorophenyl)-3-spiro-2,1'-cyclopentyl-2H-1-benzopyran))-3,5-dihydroxyhept-6-enoate,(+,-)-sodium 6E-erythro-7-(4-(4-chlorophenyl))-3-spiro(2,1'-cyclopentyl-2H-1-benzopyran)-3,5-dihydroxyhept-6-enoate, (+,-) methyl 6E-erythro-3,5-dihydroxy-7-(4-phenyl-2,2-dimethyl-3-2H-benzothiapyranyl)hept-6-enoate,
(+,-)sodium 6E-erythro-3,5-dihydroxy-7-(4-phenyl-2,2-dimethyl-3-2H-benzothiapyranyl)hept-6-enoate, (+,-)sodium 6E-erythro-3,5-dihydroxy-7-(4-phenyl-3-spiro(2,1'-cyclopentyl-2H-1-benzothiapyran))hept-6-enoate, or (+,-) ethyl erythro-7-(4-(4-fluorophenyl)-3-spiro(2,1'-cyclopentyl-2H-1-benzopyran))-3,5-dihydroxyheptanoate.

**6.** Process for the preparation of intermediate compounds in the preparation of the compounds of the formula 1 as defined in Claim 1 or 2, which are denoted by the general formulae,

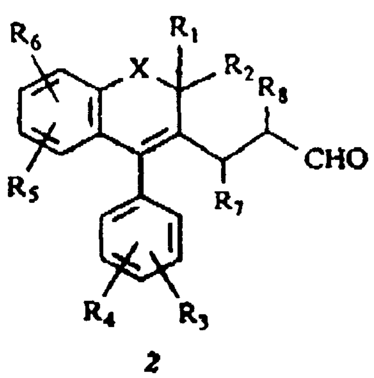
2

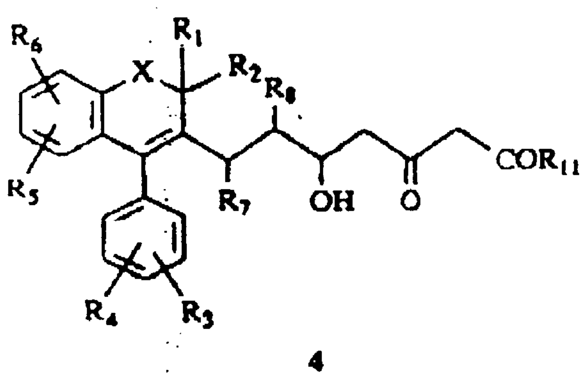
4

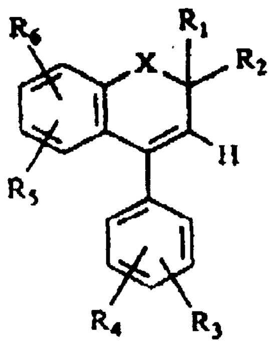

5

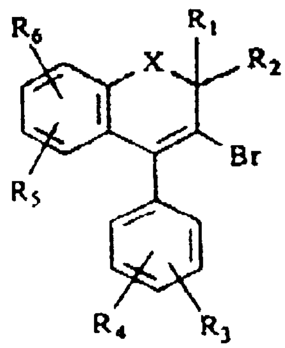

6

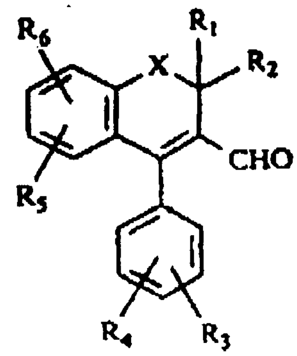

7

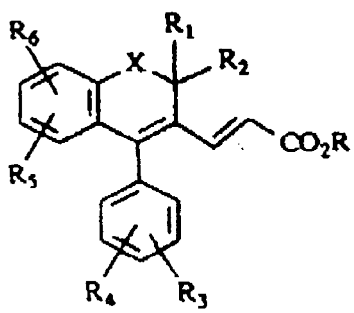

8

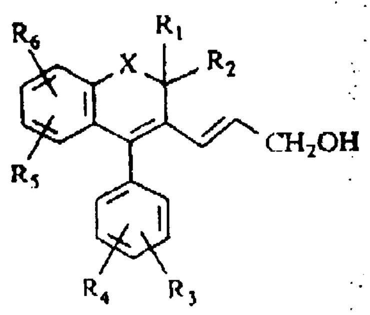

9

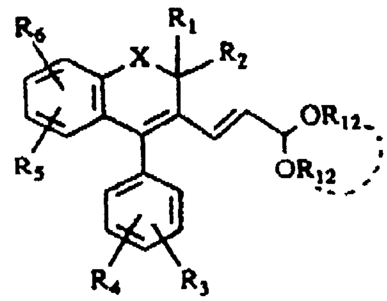

10

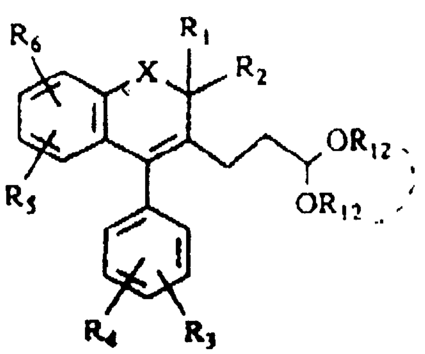

11

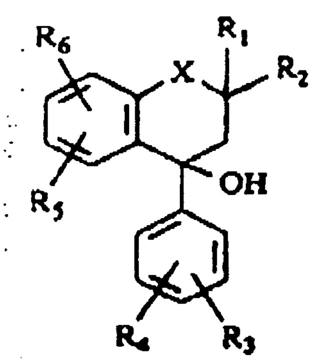

14

in which $R_1$, $R_2$, $R_3$, $R_4$, $R_5$, $R_6$, $R_7$, $R_6$, $R_9$, $R_{10}$ and $R_{11}$ are defined in Claim 1 and $R_{12}$ denotes an alkyl radical containing 1 to 4 carbon atoms or $R_{12}$ ... $R_{12}$ denotes a $-(CH_2)_q$-methylene diradical where q denotes 2 or 3 and forms a 5 or 6-membered ring with the oxygen atoms to which it is attached, with the exception of the products of formula 5 where $R_1$ and $R_2$ denote H and X denotes S or $CH_2$, characterized in that it comprises at least

α) aldol condensation of the corresponding acetoacetate $LiCH_2COCH(Na)COR_{11}$ in an appropriate solvent, in the presence of an appropriate complexing agent, with the corresponding compound of the formula 2, or

β) for the preparation of the intermediate compounds of the formula 1, where $R_7$ and $R_8$ denote a double bond, reacting the corresponding compound of the formula 5

A) with N,N'-dimethyl-3-aminoacrolein in an inert solvent at a temperature between 20°C and the reflux temperature to form the corresponding compound of the formula 2 in trans form; or

B) with N-bromosuccinimide in an appropriate solvent to produce the corresponding 3-brominated compound of the formula 6,

a) which reacts with butyllithium in an appropriate solvent and then 3-ethoxyacrolein or N,N-dimethyl-3-aminoacrolein to form the corresponding compound of the formula 2 in trans form; or

b) which reacts with an alkyl acrylate in the presence of a basic agent and of a palladium derivative in an appropriate solvent to form the corresponding compound 8, or

C) N,N-dimethylformamide to produce the corresponding aldehyde of the formula 7, which reacts with ethoxy- or methoxycarboxymethylenetriphenylphosphorane or methyl or ethyl triphenylphosphonoacetate to give the corresponding 3-substituted propenoic ester of the formula 8 in trans form, the corresponding compound 8 being reduced in the form of the corresponding 3-substituted propenol of the formula 9 in trans form, which undergoes a controlled oxidation in an appropriate solvent to produce the corresponding compound of the formula 2 in trans form, or

γ) for the preparation of the compounds of the formula 2, where $R_7$ and $R_8$ denote hydrogen, treatment of the corresponding compound of the formula 11 by deacetalization with acidic catalysis in an appropriate solvent, the corresponding compound 11 being prepared by catalytic hydrogenation of the corresponding compound of the formula 10 where $R_7$ and $R_8$ denote a bouble bond, obtained by acetalization of the corresponding aldehyde of the formula 2, or treatment of the corresponding compound of the formula 6 is treated with allyl alcohol in the presence of an appropriate basic agent to obtain the corresponding aldehyde of the formula 2.

7. Process for the preparation of pharmaceutical compositions, characterized in that at least one compound of the formula 1 as defined in Claim 1 is mixed with a pharmaceutically acceptable excipient.

**Claims for the following Contracting State : GR**

1. Derivatives of benzocycloalkenyldihydroxyalkanoic acids of the following formula 1,

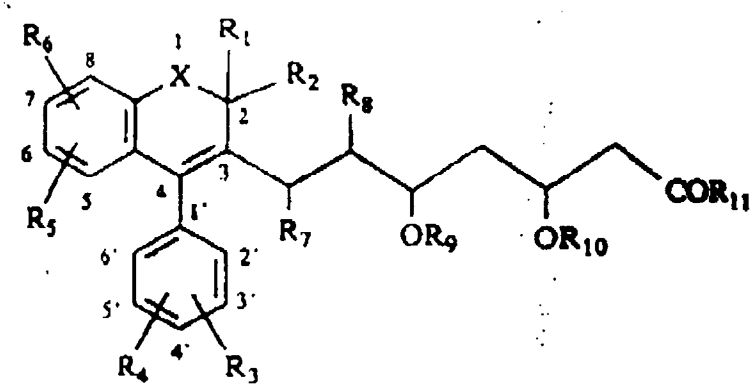

1

in which: X denotes a $-CH_2-$ methylene group or an oxygen or sulphur atom; $R_1$ and $R_2$, which are identical or different, denote hydrogen atoms or alkyl radicals containing 1 to 3 carbon atoms; $R_1$ and $R_2$ may also together form a $-(CH_2)_n-$ alkylene chain in which the number of groups n may be equal to 4 or 5 and, if appropriate, substituted symmetrically by one or two alkyl radicals containing 1 to 3 carbon atoms; $R_3$ and $R_4$, which may be identical or different, denote hydrogen, fluorine, chlorine or bromine atoms, $CF_3$ radicals, N,N-dialkylamino containing 1 to 3 carbon atoms, alkyl containing 1 to 4 carbon atoms, alkoxy containing 1 to 5 carbon atoms, phenyl optionally substituted by at most two substituents which may be identical or different and may denote $C_{1-3}$-alkyl radicals or fluorine or chlorine atoms, it being understood that when one of the substituents $R_3$ and $R_4$ denotes a $CF_3$, N,N-dialkylamino, phenyl or substituted phenyl radical, it is present on the 3', 4' or 5' positions and the other substituent denotes a hydrogen atom; $R_5$ and $R_6$, which may be identical or different, denote hydrogen, fluorine, chlorine or bromine atoms or the radicals: $CF_3$, $C_{1-3}$-alkyl, $C_{1-3}$-alkoxy or phenyl, substituted if appropriate by at most two $C_{1-3}$-alkyl or $C_{1-3}$-alkoxy

radicals, or fluorine or chlorine atoms, on condition that when one of the substituents $R_5$ and $R_6$ denotes the radicals $CF_3$, phenyl or substituted phenyl, it is present on the positions 6 or 7 and the other denotes a hydrogen atom: the substituents $R_3$ and $R_4$ or $R_5$ and $R_6$ may also together form, on condition of being on two adjacent positions, the diradicals of formulae: -CH=CH-CH=CH-, $-(CH_2)_m-$ or $-O(CH_2)_pO-$, in which m may be equal to 3 or 4 and p to 1 or 2, it being understood that when $R_3$ and $R_4$ or $R_5$ and $R_6$ denote the diradical $-O(CH_2)_pO-$ the latter is linked to the positions 3' and 4' or 4' and 5' of 6 and 7; each of the substituents $R_7$ and $R_8$ denotes a hydrogen atom or, with the existing C-C bond, they together form a double bond of trans (E) geometry; each of the substituents $R_9$ and $R_{10}$ denotes a hydrogen atom or they together form a dialkylmethylene radical containing 1 to 3 carbon atoms, $R_{11}$ denoting, with the CO group to which it is bonded, a free acid, ester, amide or acid salt functional group or forming a δ-lactone ring with $R_9$, and their optically active isomers.

2. Compound according to Claim 1, characterized in that $R_{11}$ denotes one of the following radicals: hydroxy, alkoxy containing 1 to 4 carbon atoms, benzyloxy, alkylamino or N,N-dialkylamino containing 1 to 3 carbon atoms, imino containing 4 to 6 carbon atoms, cycloamino containing 3 to 6 carbon atoms, amino or benzylamino or $-O^-M^+$ where $M^+$ denotes a pharmaceutically acceptable cation, or $R_{11}$ forms a single bond with $R_9$.

3. Compounds according to Claim 1 or 2, in the formula of which each of the substituents $R_1$ and $R_2$ denotes a methyl group or they together form a tetramethylene chain, $R_3$ and $R_4$ or $R_8$ and $R_8$, which may be identical or different, denote hydrogen, fluorine or chlorine atoms, methyl or ethyl, methoxy, methylthio, phenyl, 4-fluorophenyl, 4-methylphenyl or 4-methoxyphenyl radicals or together form one of the following diradicals: -CH=CH-CH=CH-, tetramethylene: $-(CH_2)_4-$ or methylenedioxy: $-O(CH_2)O-$.

4. Compounds according to Claim 1 or 2, characterized in that

A) X denotes oxygen or sulphur atoms or a methylene group, each of $R_1$ and $R_2$ denotes a methyl radical or they together form a $-(CH_2)_4-$ tetramethylene chain, only one of the radicals $R_3$ and $R_4$ or $R_8$ and $R_8$ denotes a hydrogen atom, $R_7$, and $R_8$ together form a bond and each of $R_9$ and $R_{10}$ denotes a hydrogen atom, or

B) X, $R_1$, $R_2$, $R_7$, $R_8$, $R_9$ and $R_{10}$ have the meanings given immediately above in A), one of the substituents $R_3$ and $R_4$ denotes a hydrogen atom and the other a fluorine or chorine atom, and each of the two substituents $R_8$ and $R_8$ denotes a hydrogen atom, or

C) X, $R_1$, $R_2$, $R_7$, $R_8$, $R_9$ and $R_{10}$ have the meanings which have just been defined above in A), one of the substituents $R_3$ and $R_4$ is a hydrogen atom and the other denotes a fluorine atom, and each of the two substituents $R_8$ and $R_8$ denotes a hydrogen atom, or

D) X, $R_1$, $R_2$, $R_7$, $R_8$, $R_9$ and $R_{10}$ have the particular meanings defined in A), the two substituents $R_3$ and $R_4$ are hydrogen atoms and only one of the radicals $R_5$ and $R_6$ denotes a hydrogen atom, or

E) X, $R_3$, $R_4$, $R_5$, $R_6$, $R_7$, $R_8$, $R_9$ and $R_{10}$ have the meanings above in C) and $R_1$ and $R_2$ together form a-$(CH_2)_4$- tetramethylene chain, or

F) X, $R_1$, $R_2$, $R_7$, $R_8$, $R_9$ and $R_{10}$ have the meanings shown immediately above in E) and each of the substituents $R_3$, $R_4$, $R_5$ and $R_6$ denotes a hydrogen atom.

5. Compounds according to Claim 1 or 2, called: (+,-)ethyl 6E-erythro-7-(4-(4-fluorophenyl-3-spiro(2,1'-cyclopentyl-2H-1-benzopyran))-3,5-dihydroxyhept-6-enoate, (+,-)sodium 6E-erythro-7-(4-(4-fluorophenyl)-3-spiro-(2,1'-cyclopentyl-2H-1-benzopyran))-3,5-dihydroxyhept-6-enoate, (+,-)methyl 6E-erythro-7-(1,2-dihydro-2,2-dimethyl-4-phenyl-3-naphthyl)-3,5-dihydroxyhept-6-enoate, (+,-)ethyl 6E-erythro-7-(4-(4-chlorophenyl)-3-spiro(2,1'-cyclopentyl-2H-1-benzopyran))-3,5-dihydroxyhept-6-enoate, (+,-)-sodium 6E-erythro-7-(4-(4-chlorophenyl)-3-spiro(2,1'-cyclopentyl-2H-1-benzopyran)-3,5-dihydroxyhept-6-enoate, (+,-) methyl 6E-erythro-3,5-dihydroxy-7-(4-phenyl-2,2-dimethyl-3-2H-benzothiapyranyl)hept-6-enoate, (+,-)sodium 6E-erythro-3,5-dihydroxy-7-(4-phenyl-2,2-dimethyl-3-2H-benzothiapyranyl)hept-6-enoate,(+,-) sodium 6E-erythro-3,5-dihydroxy-7-(4-phenyl-3-spiro(2,1'-cyclopentyl-2H-1-benzothiapyran))hept-6-enoate, (+,-)ethyl erythro-7-(4-(4-fluorophenyl)-3-spiro(2,1'-cyclopentyl-2H-1-benzopyran))-3,5-dihydroxyheptanoate.

6. Processes for the preparation of the compounds of the formula 1 as defined in claim 1 or 2, characterized in that they comprise at least

a) reducing a keto-ester of the formula 4, which may be optically active,

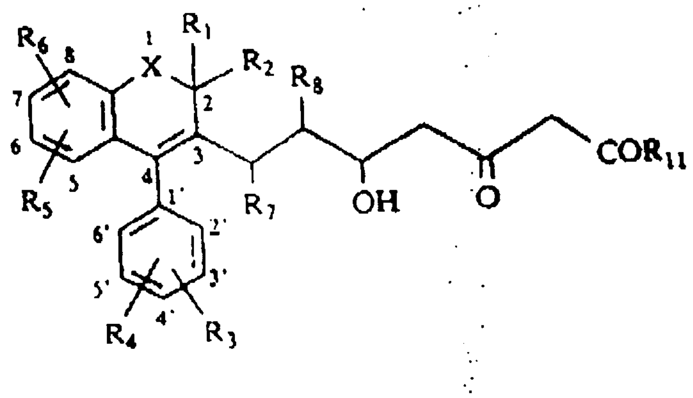

4

and, if appropriate,

b) transesterifying compounds of the formula 1 or alcoholizing compounds of the formula 1 in δ-lactone form or else alkylating compounds or the formula 1 in the form of salts and, or

c) hydrolysing compounds of the formula 1 in ester or δ-Lactone form, or

d) when $R_7$ and $R_8$ or $R_9$ and $R_{11}$ together form a single bond: treating the corresponding compounds of the formula 1 in the form of salts using a tertiary chloroamine, or

e) when $R_7$, $R_8$ and $R_{10}$ denote a hydrogen atom and $R_9$ and $R_{11}$ together form a bond: catalytically reducing the δ-lactone compounds of the formula 1 in which $R_7$ and $R_8$ form a bond or lactonizing of the corresponding acid compounds of the formula 1 in which $R_7$, $R_8$ and $R_{10}$ denote a hydrogen atom, or

f) aminolysing the compounds of the formula 1 in ester or δ-lactone form, or

g) when $R_9$ and $R_{10}$ together form a dialkylalkylene radical, cyclizing the corresponding compounds of the formula 1 in which each of $R_9$ and $R_{10}$ denotes a hydrogen atom, using an alkoxyalkene, and

h) optionally splitting the racemate obtained.

7. Intermediate compounds in the preparation of the compounds of the formula 1 as defined in Claim 1 or 2, which are denoted by the general formulae,

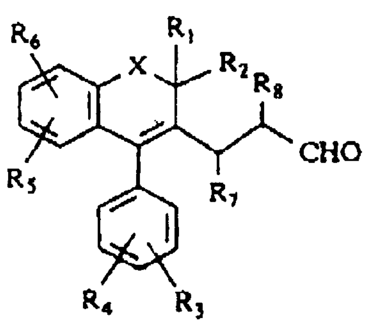

2

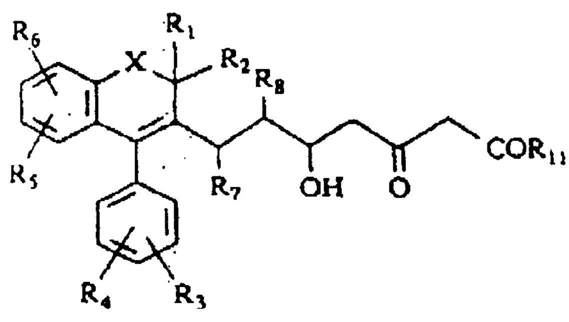

4

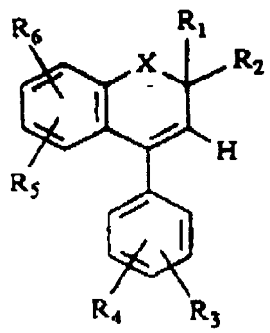

5

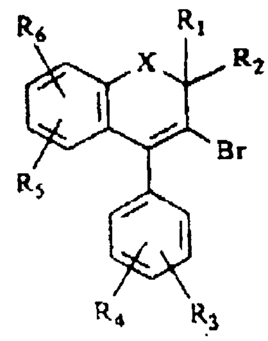

6

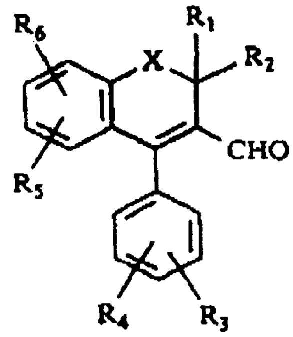

7

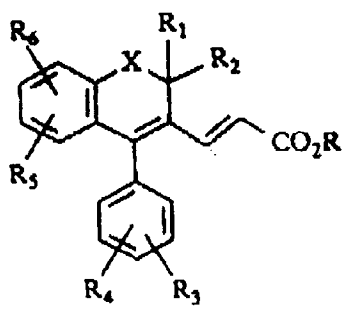

8

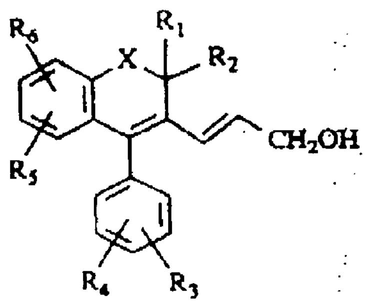

9

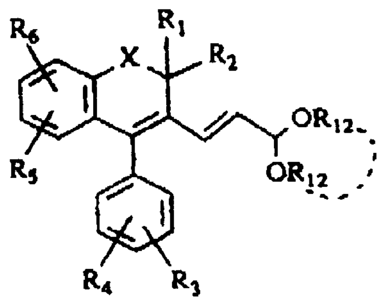

10

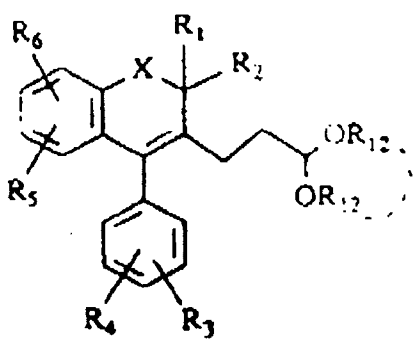

11

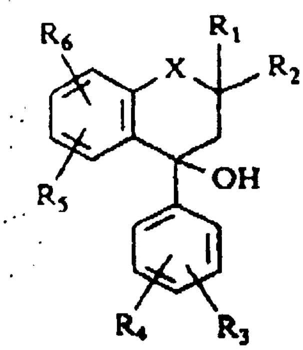

14

in which $R_1$, $R_2$, $R_3$, $R_4$, $R_5$, $R_6$, $R_7$, $R_6$, $R_9$, $R_{10}$ and $R_{11}$ are defined in Claim 1 and $R_{12}$ denotes an alkyl radical containing 1 to 4 carbon atoms or $R_{12}$ ... $R_{12}$ denotes a $-(CH_2)_q$-methylene diradical where q denotes 2 or 3 and forms a 5 or 6-members ring with the oxygen atoms to which it is attached, with the exception of the products of formula 5 where $R_1$ and $R_2$ denote H and X denotes S or $CH_2$.

**8.** Process for the preparation of the intermediate compounds of the formula 4, as defined in claim 7, characterized in that it comprises aldol condensation of the corresponding acetoacetate $LiCH_2COCH(Na)COR_{11}$ in an appropriate solvent, in the presence of an appropriate complexing agent, with the corresponding compound of the formula 2.

**9.** Process for the preparation of the intermediate compounds of the formula 2, which are defined in Claim

7, where $R_7$ and $R_8$ denote a double bond, characterized in that it comprises reacting the corresponding compound of the formula 5

A) with N,N'-dimethyl-3-aminoacrolein in an inert solvent at a temperature between 20°C and the reflux temperature to form the corresponding coupound of the formula 2 in trans form; or

B) with N-bromosuccinimide in an appropriate solvent to produce the corresponding 3-brominated compound of the formula 6,

a) which reacts with butyllithium in an appropriate solvent and then 3-ethoxyacrolein or N,N-dimethyl- 3-aminoacrolein to form the corresponding compound of the formula 2 in trans form; or

b) which reacts with an alkyl acrylate in the presence of a basic agent and of a palladium derivative in an appropriate solvent to form the corresponding compound 8, or

C) N,N-dimethylformamide to produce the corresponding aldehyde of the formula 7, which reacts with ethoxy- or methoxycarboxymethylenetriphenylphosphorane or methyl or ethyl triphenylphosphonoacetate to give the corresponding 3-substituted propenoic ester of the formula 8 in trans form, the corresponding compound 8 being reduced in the form of the corresponding 3-substituted propenol of the formula 9 in trans form, which undergoes a controlled oxidation in an appropriate solvent to produce the corresponding compound of the formula 2 in trans form.

**10.** Process for the preparation of the compounds of the formula 2 which are defined in Claim 7, where $R_7$ and $R_8$ denote hydrogen, characterized in that the corresponding compound of the formula 11 is subjected to a deacetalization with acidic catalysis in an appropriate solvent, the corresponding compound 11 being prepared by catalytic hydrogenation of the corresponding compound of the formula 10 where $R_7$ and $R_8$ denote a double bond, obtained by acetalization of the corresponding aldehyde of the formula 2, or in that the corresponding compound of the formula 6 is treated with allyl alcohol in the presence of an appropriate basic agent to obtain the corresponding aldehyde of the formula 2.

**11.** Process for the preparation of pharmaceutical compositions, characterized in that at least one compound of the formula 1 as defined in Claim 1 is mixed with a pharmaceutically acceptable excipient.

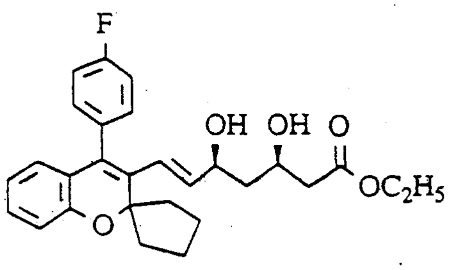

Composé n°1

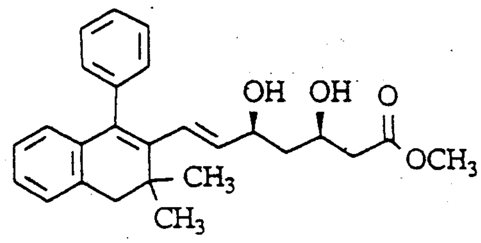

Composé n°2

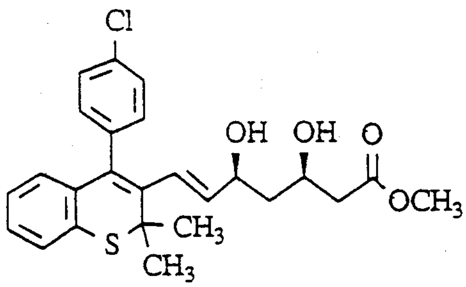

Composé n°3

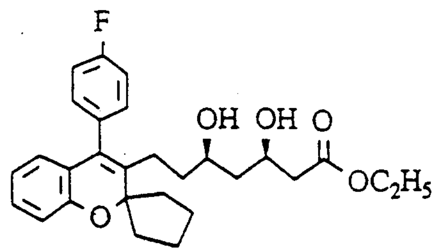

Composé n°4

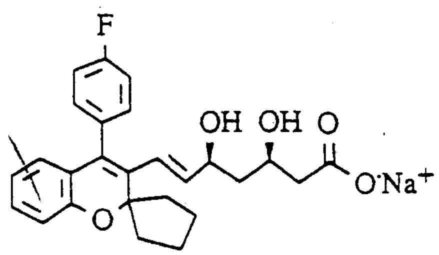

Composé n°5

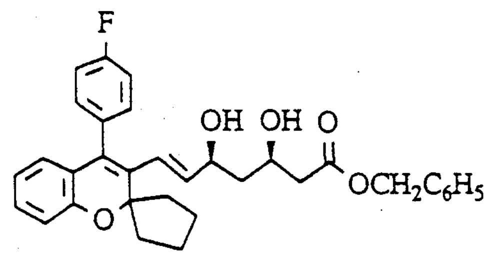

Composé n°6

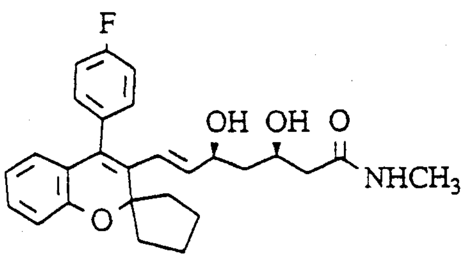

Composé n°7

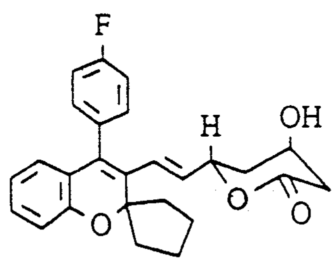

Composé n°8

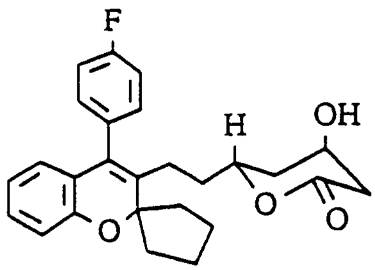

Composé n°9

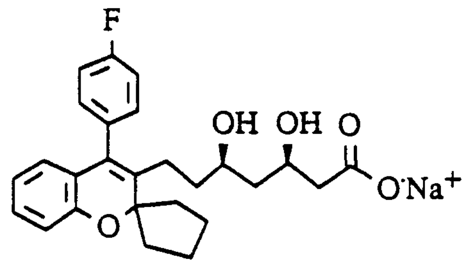

Composé n°10

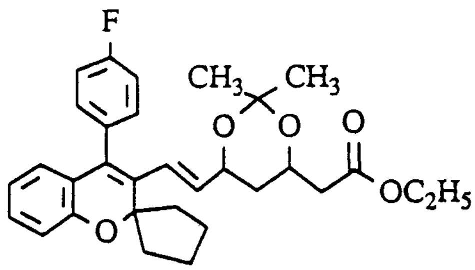

Composé n°11

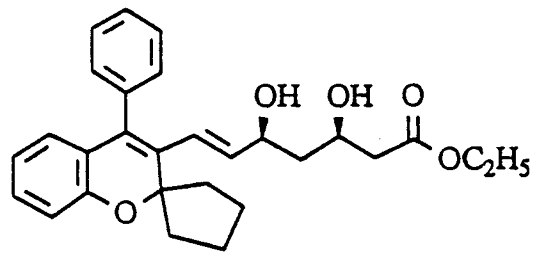

Composé n°12

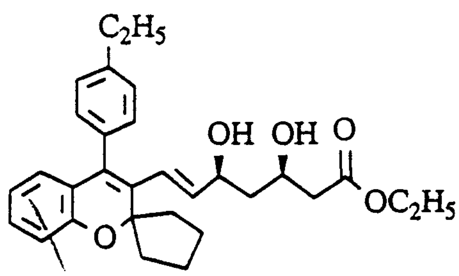

Composé n°13

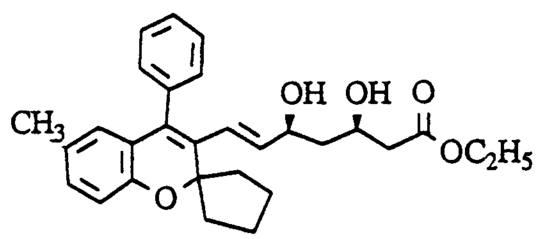

Composé n°14

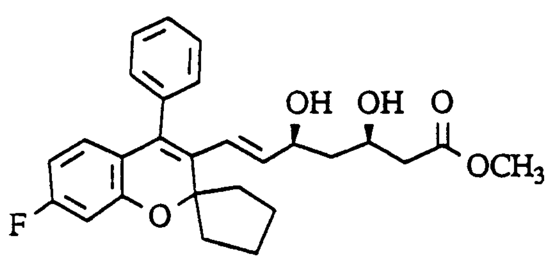

Composé n°15

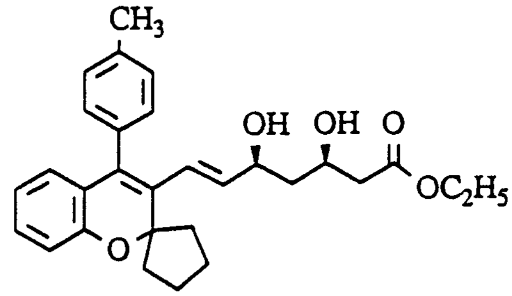

Composé n°16

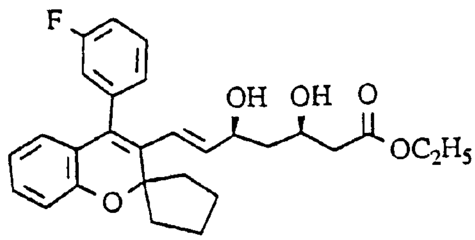

Composé n°17

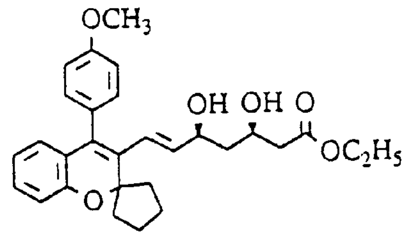

Composé n°18

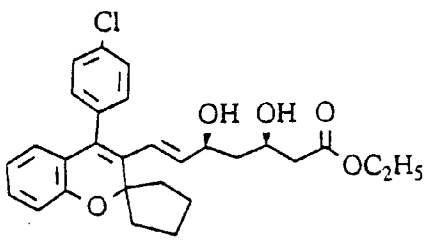

Composé n°19

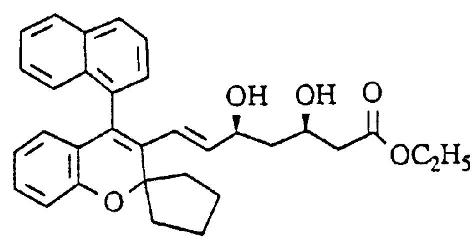

Composé n°20

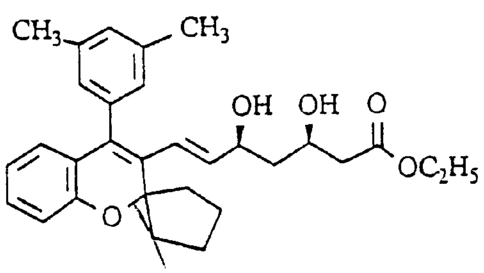

Compose n°21

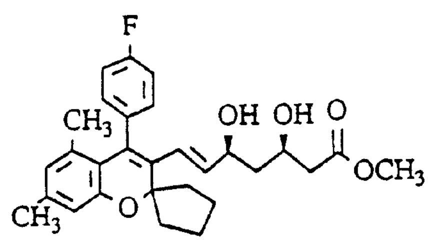

Composé n°22

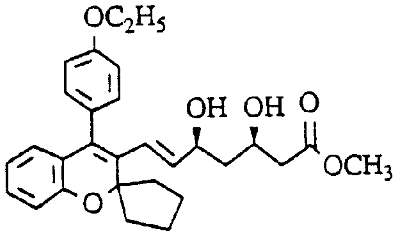

Composé n°23

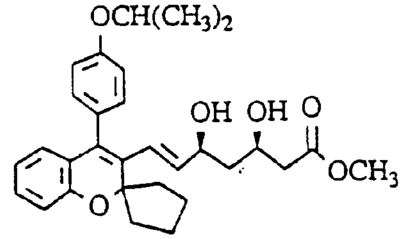

Composé n°24

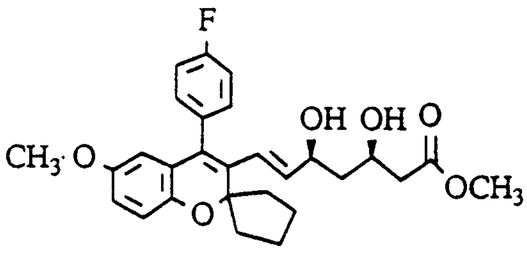

Composé n°25

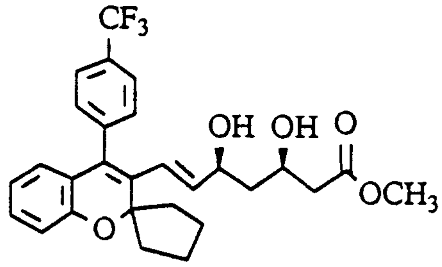

Composé n°26

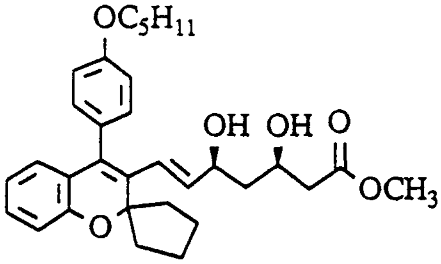

Composé n°27

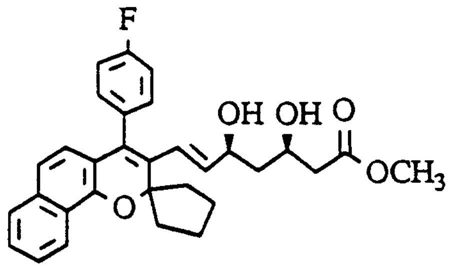

Composé n°28

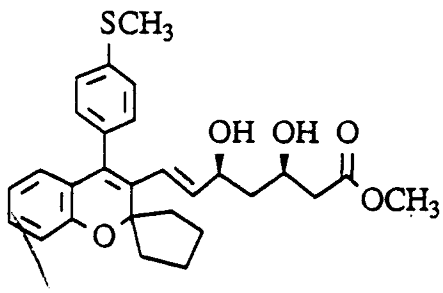

Composé n°29

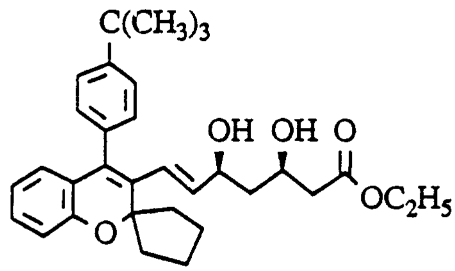

Composé n°30

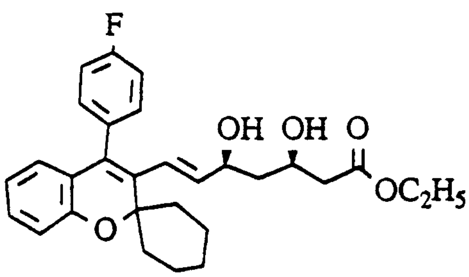

Composé n°31

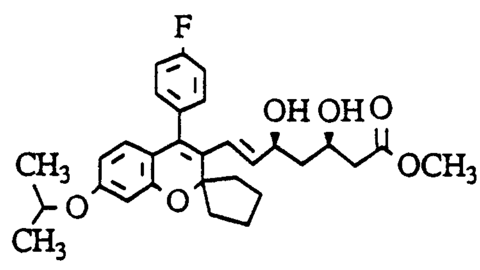

Composé n°32

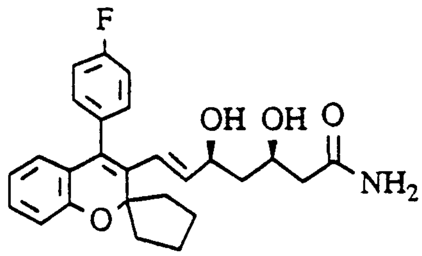

Composé n°33

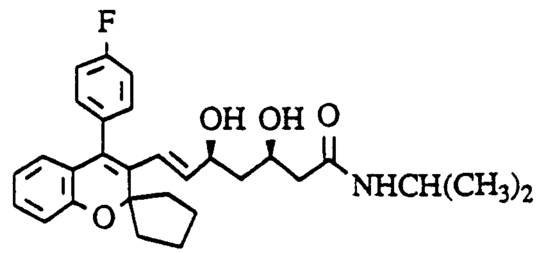

Composé n°34

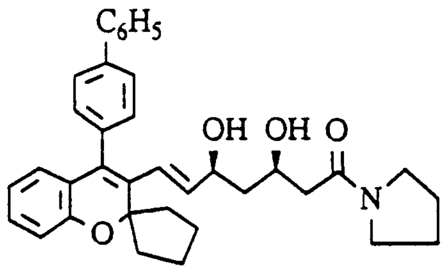

Composé n°35

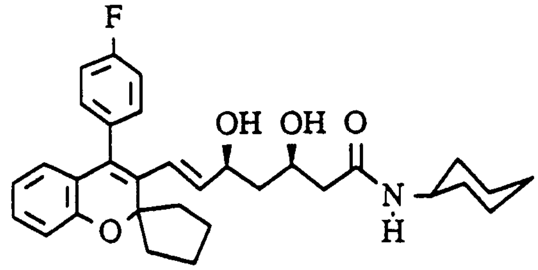

Composé n°36

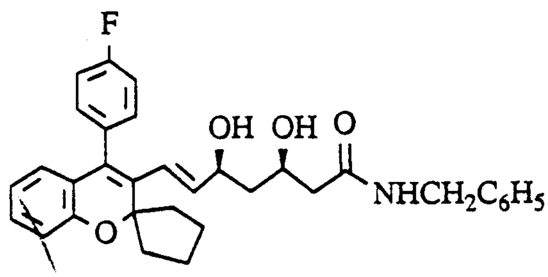

Composé n°37

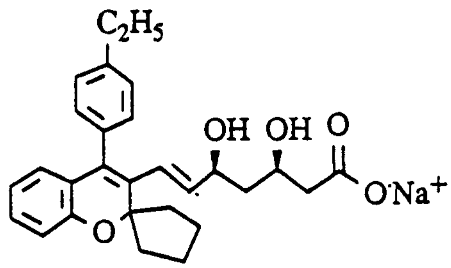

Composé n°38

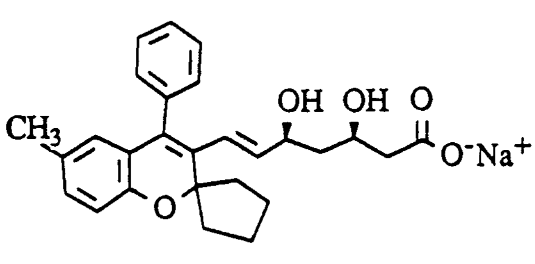

Composé n°39

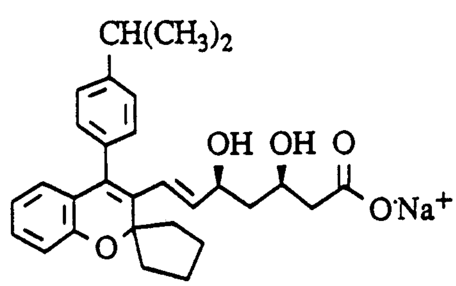

Composé n°40

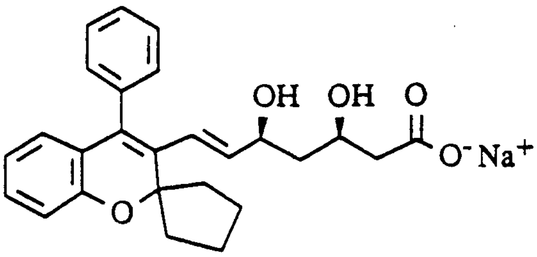

Composé n°41

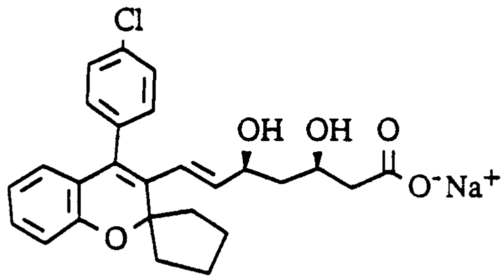

Composé n°42

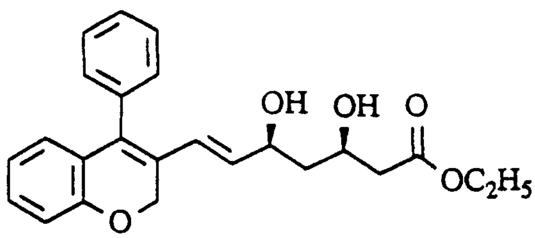

Composé n°43

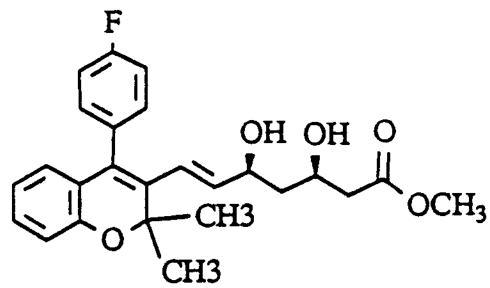

Composé n°44

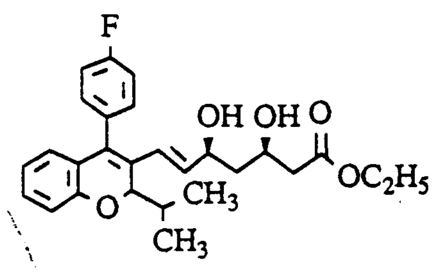

Composé n°45

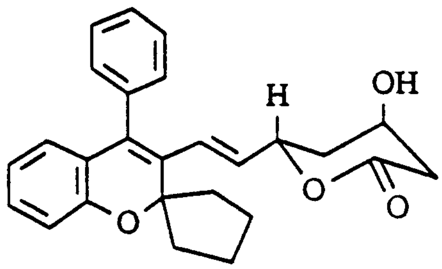

Composé n°46

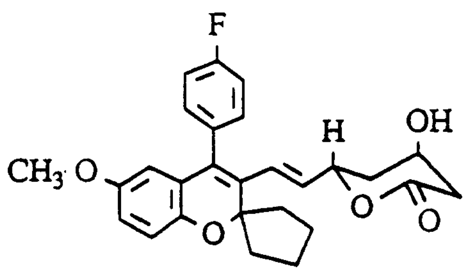

Composé n°47

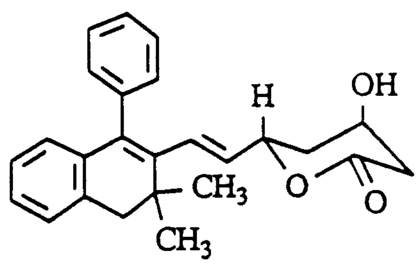

Composé n°48

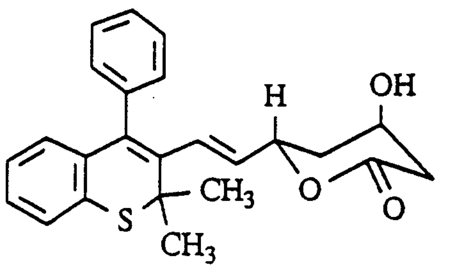

Composé n°49

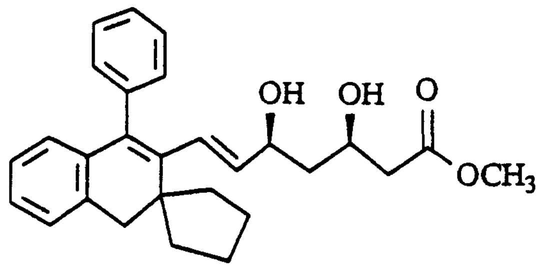

Composé n°50

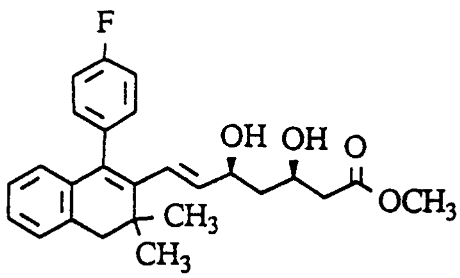

Composé n°51

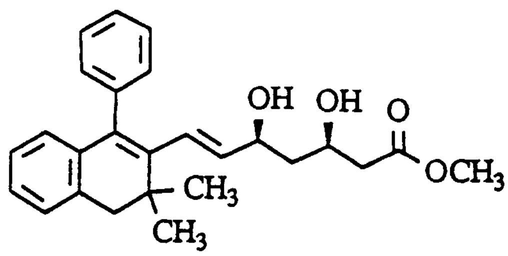

Composé n°52

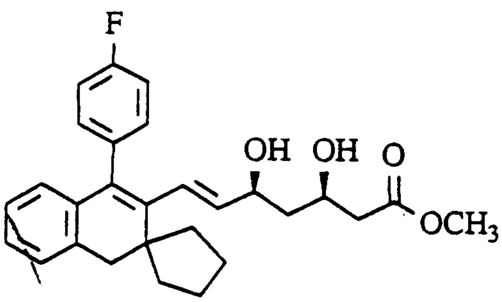

Composé n°53

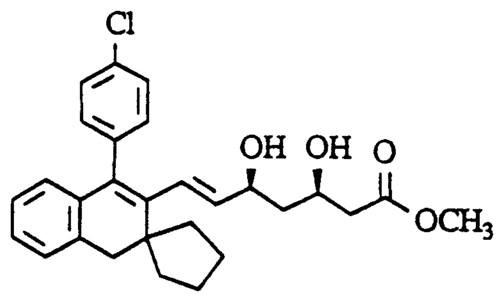

Composé n°54

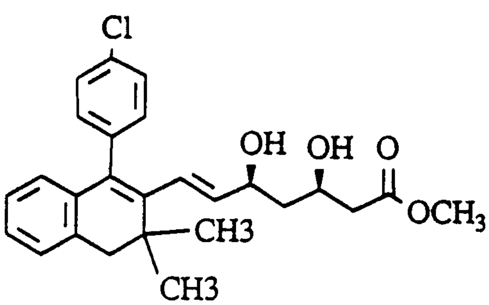

Composé n°55

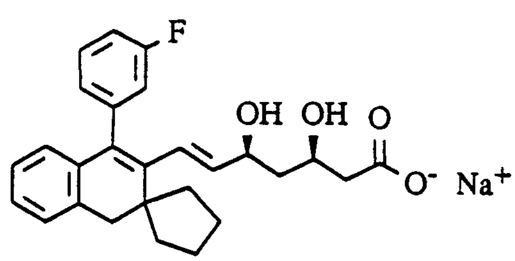

Composé n°56

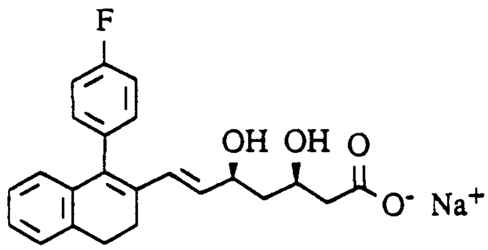

Composé n°57

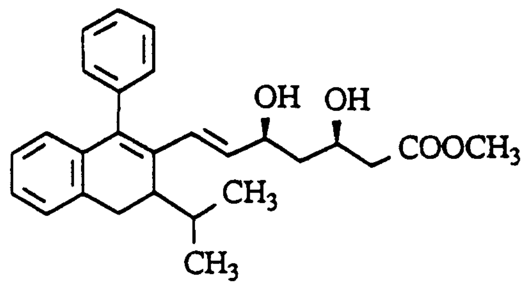

Composé n°58

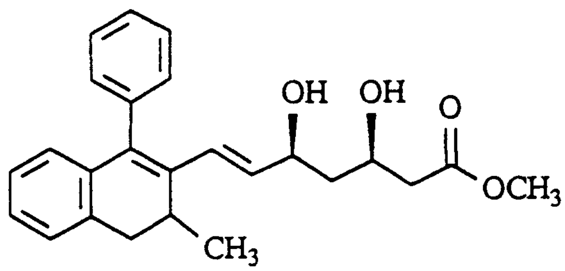

Composé n°59

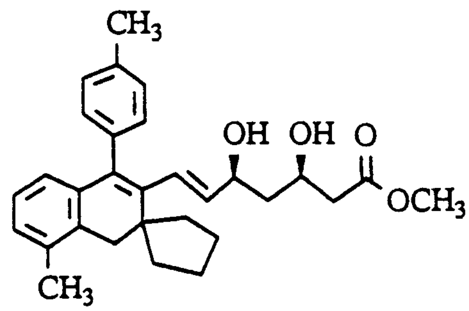

Composé n°60

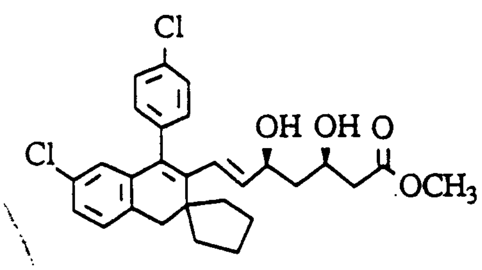

Composé n°61

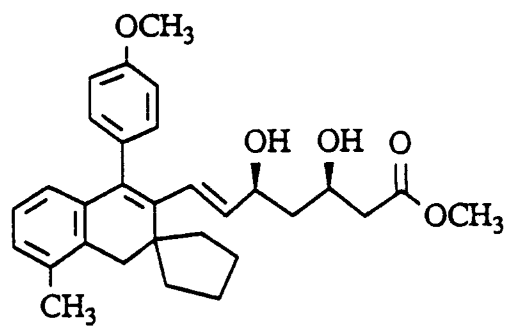

Composé n°62

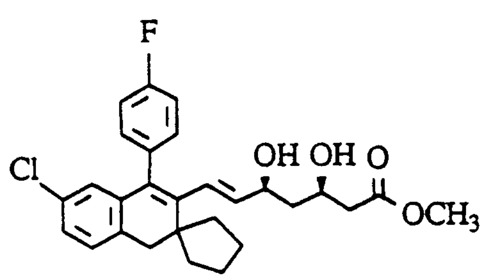

Composé n°63

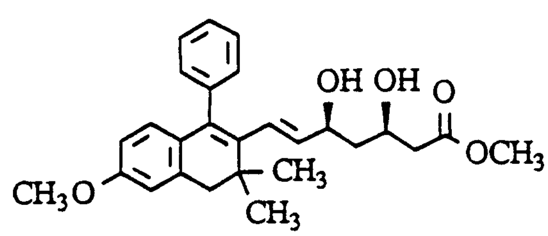

Composé n°64

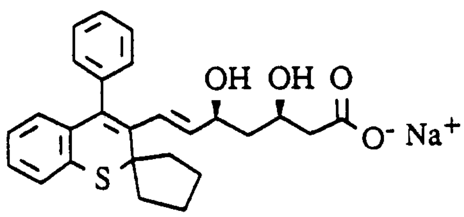

Composé n°65

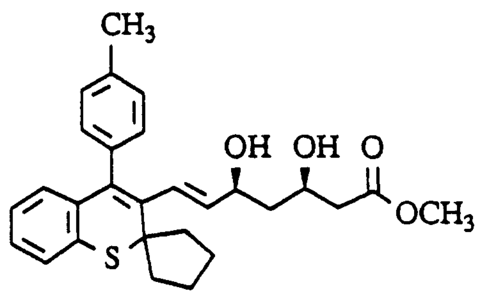

Composé n°66

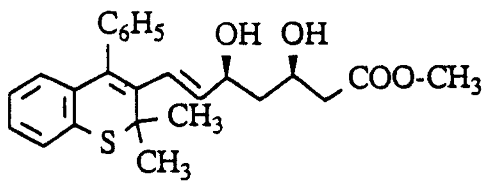

Composé n°67

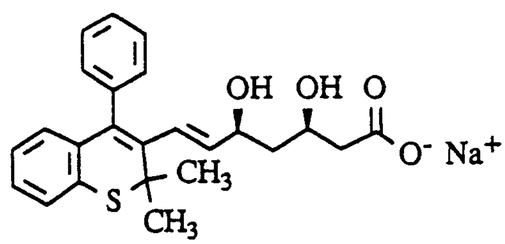

Composé n°68